# EUROPEAN PATENT APPLICATION

(11) **EP 2 993 167 A1**
(43) Date of publication of application: **09.03.2016**
(21) Application number: 14791512.8
(22) Date of filing: 28.04.2014
(51) Int. Cl.: C07C 323/18, A61K 31/198, A61K 31/4402, A61K 31/4409, A61K 31/4525, A61P 3/10, A61P 43/00, C07D 207/16, C07D 211/34, C07D 211/58, C07D 213/56, C07D 231/12, C07D 239/26, C07D 249/08, C07D 263/32, C07D 271/06

(54) **PHARMACEUTICAL COMPOSITION FOR THE TREATMENT OF DIABETES**

(30) Priority: 01.05.2013 JP 2013096535
(71) Applicant: Ajinomoto Co., Inc., Tokyo, 104-8315 (JP)
(72) Inventor: MATSUMOTO, Kayo, Kawasaki-shi Kanagawa 210-8681 (JP); MIYANAGA, Wataru, Kawasaki-shi Kanagawa 210-8681 (JP); DOHI, Mizuki, Kawasaki-shi Kanagawa 210-8681 (JP); NAKAGAWA, Tadakiyo, Kawasaki-shi Kanagawa 210-8681 (JP); KOBAYASHI, Kaori, Kawasaki-shi Kanagawa 210-8681 (JP); TAKESHITA, Sen, Kawasaki-shi Kanagawa 210-8681 (JP)
(74) Representative: Nicholls, Kathryn Margaret
(86) International application number: PCT/JP2014/061893
(87) International publication number: WO 2014/178381

(57) **Abstract**

An object is to provide a novel compound which has a glycogen synthase activation ability, but activates a receptor PPAR to a low degree and is highly safe. Provided is a compound represented by the following general formula (I) or a pharmaceutically acceptable salt thereof: 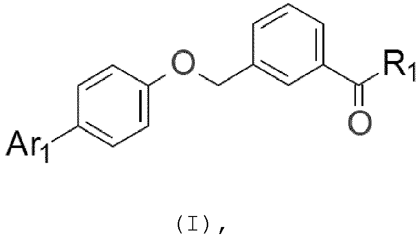 wherein Ar₁ represents any one of the following rings (II) and (III) : 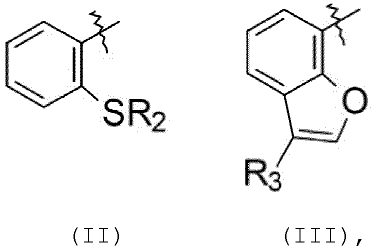 wherein R₂ represents an alkyl group, and R₃ represents a hydrogen atom or an alkyl group, and
R₁ represents any one of the following substituents (IV) and (V):

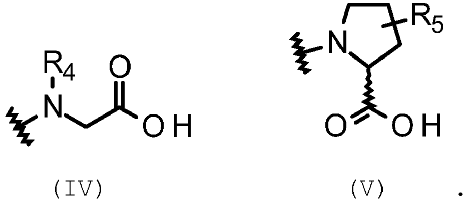

## Description

### Technical Field

The present invention relates to a novel compound having a glycogen synthase activation function, and to a pharmaceutical composition for treating diabetes mellitus comprising the compound.

### Background Art

Diabetes mellitus is an important disease for people of today. The incidence of diabetes mellitus has recently been on an upward trend. Many drugs for treating diabetes mellitus have been developed based on the mechanisms of the development of diabetes mellitus, and have been actually used. For example, one of or a combination of two or more of an insulin sensitivity enhancer, an α-glycosidase inhibitor, an insulin secretion promoter, an insulin preparation, and the like have been used.

Under such circumstances, a technology for treating diabetes mellitus has been under development based on a novel mechanism, namely, activation of glycogen synthase, different from those of the above-described conventional drugs for treating diabetes mellitus. Specifically, biaryloxymethylarenecarboxylic acids have been proposed as compounds capable of activating glycogen synthase (Patent Literatures 1 to 9).

Meanwhile, a novel pharmaceutically active compound is required not only to have an intended effect for treating a disease, but also to be safe, for example, to have no adverse effects. Examination of peroxisome proliferator-activated receptors (PPAR) has been proposed as means for drug safety evaluation (Non Patent Literature 1). This is based on the fact that administration of a certain drug to experimental animals such as rats causes liver enlargement and remarkable induction of enzymes in the liver, and long-term administration of the drug causes hepatic cancer. Here, a characteristic change is remarkable proliferation of peroxisomes, which are organelles in the liver. It has been revealed that the receptors PPARs, especially a subfamily PPARα, which are activated by a peroxisome proliferator (PP) is involved in the mechanism of drug-liver peroxisome proliferation-liver carcinogenesis. This phenomenon has attracted attention in association with drug safety evaluation (especially, carcinogenicity evaluation) at the drug development stage.

### Citation List

### Patent Literatures

Patent Literature 1: WO2005/000781
Patent Literature 2: WO2006/058648
Patent Literature 3: WO2011/057956
Patent Literature 4: WO2011/057959
Patent Literature 5: WO2011/057993
Patent Literature 6: WO2011/058122
Patent Literature 7: WO2011/058154
Patent Literature 8: WO2011/067174
Patent Literature 9: WO2011/067266

### Non Patent Literature

Non Patent Literature 1: Journal of Clinical and Experimental Medicine (Igaku no Ayumi), Vol. 220, No. 1, (2007) pp. 75-80

### Summary of Invention

### Technical Problems

An object of the present invention is to provide a novel compound which has a glycogen synthase activation ability, but activates a receptor PPAR to a low degree and is highly safe.

Another object of the present invention is to provide a pharmaceutical composition comprising the above-described compound.

Still another object of the present invention is to provide a pharmaceutical composition for treating diabetes mellitus comprising the above-described compound.

Still another object of the present invention is to provide a glycogen synthase activator comprising the above-described compound.

### Solution to Problems

The present invention has been made based on the following finding. Specifically, the biaryloxymethylarenecarboxylic acid compounds described in Patent Literatures 1 to 9 have been studied intensively, and it has been found that compounds which have a specific aryl group as the terminal aryl group of the biaryl ring positioned at one terminal and which have a specific amino group as the amino group positioned at the other terminal have extremely high glycogen synthase activation ability, and that the use of such a compound makes it possible to efficiently achieve the above-described objects.

Specifically, the present invention provides the following [1] to [10].
[1] A compound represented by the following general formula (I) or a pharmaceutically acceptable salt thereof: 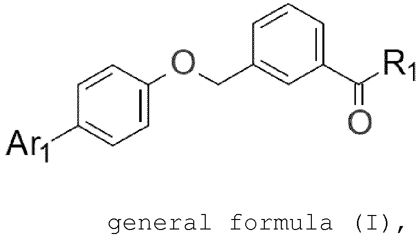 wherein Ar₁ represents any one of the following rings (II) and (III) : 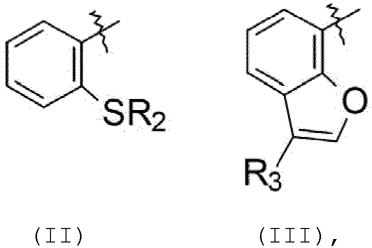 wherein each of the rings may have one or more substituents, and the substituents are selected from the group consisting of acetamido groups, aminocarbonyl groups, halogen atoms, alkyl groups, hydroxyalkyl groups, alkoxyalkyl groups, cyano groups, cyanoalkyl groups, amino groups, aminoalkyl groups, monoalkylaminoalkyl groups, dialkylaminoalkyl groups, alkoxy groups, and halogenoalkoxy groups;
   R₂ represents an alkyl group, and R₃ represents a hydrogen atom or an alkyl group, and
   R₁ represents any one of the following substituents (IV) and (V): 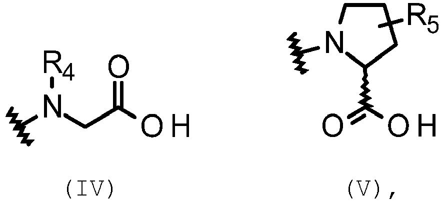
   wherein R₄ represents a substituent selected from cyanoalkyl groups, aminocarbonylalkyl groups, dialkylaminocarbonylalkyl groups, monoalkylaminocarbonylalkyl groups, alkylsulfonylalkyl groups, aminoalkyl groups, aminosulfonylalkyl groups, monoalkylaminosulfonylalkyl groups, dialkylaminosulfonylalkyl groups, monoalkylaminoalkyl groups, dialkylaminoalkyl groups, aminocycloalkyl groups, monoalkylaminocycloalkyl groups, dialkylaminocycloalkyl groups, alkoxyalkyl groups, monoalkoxyaminoalkyl groups, alkoxyalkyleneoxyalkyl groups, alkylcarbonylaminoalkyl groups, alkylsulfonylaminoalkyl groups, hydroxyalkyl groups, carboxyalkyl groups, and groups represented by -L₁-B, where L₁ represents a bond or an alkylene group, and B represents an optionally substituted 5-membered or 6-membered heterocyclic group having at least one heteroatom selected from nitrogen atoms, oxygen atoms, and sulfur atoms, and
   R₅ represents -L₂-R₆, where L₂ represents a bond or an alkylene group, and R₆ represents a hydroxy group, an alkoxy group, an amide group, an amino group, a monoalkylamino group, a dialkylamino group, a cyano group, an aminocarbonyl group, a monoalkylaminocarbonyl group, or a dialkylaminocarbonyl group.
[2] The compound according to [1] or a pharmaceutically acceptable salt thereof, wherein B in -L₁-B of R₄ in the general formula (I) is any one of the following groups: 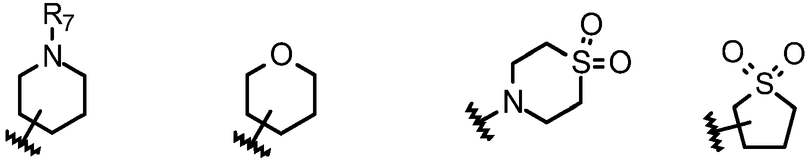 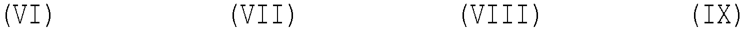 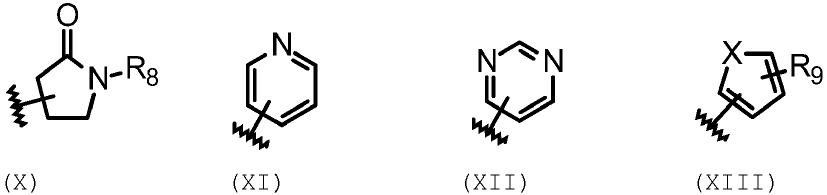 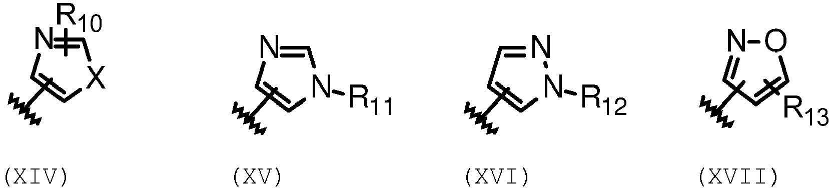 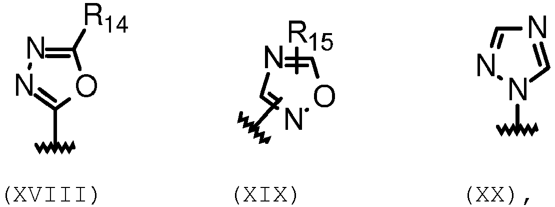 wherein R₇ represents a hydrogen atom, an alkyl group, or an alkylcarbonyl group, R₈ to R₁₅ each represent a hydrogen atom or an alkyl group, and X represents an oxygen atom or a sulfur atom.
[3] The compound according to [1] or a pharmaceutically acceptable salt thereof, wherein R₁ represents any one of the following substituents (IV), (V-I), and (V-II): 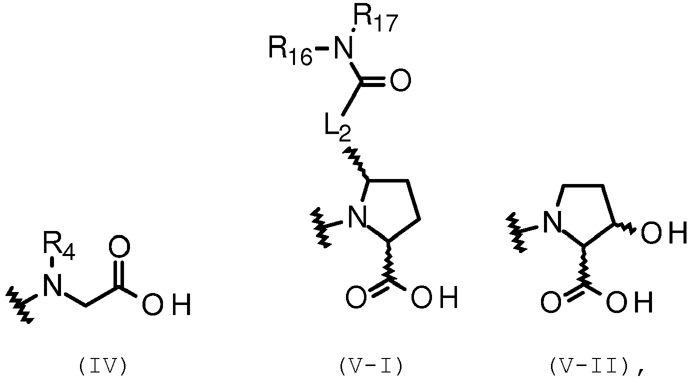 wherein R₄ represents a substituent selected from cyanoalkyl groups, aminocarbonylalkyl groups, dialkylaminocarbonylalkyl groups, monoalkylaminocarbonylalkyl groups, alkylsulfonylalkyl groups, aminoalkyl groups, monoalkylaminoalkyl groups, dialkylaminoalkyl groups, alkoxyalkyl groups, alkylsulfonylaminoalkyl groups, hydroxyalkyl groups, carboxyalkyl groups, and the following (VI-I), (VII-I), and (XI-I): 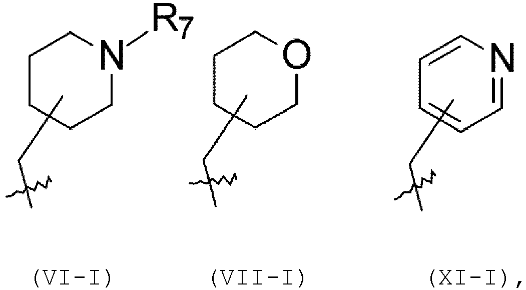
   wherein R₇ represents a hydrogen atom or an alkyl group,
   R₁₆ and R₁₇, which may be the same or different, each represent a hydrogen atom or an alkyl group, or R₁₆ and R₁₇ taken together may form a ring, and
   L₂ represents a bond or an alkylene group.
[4] The compound according to any one of [1] to [3] or a pharmaceutically acceptable salt thereof, wherein Ar₁ in the general formula (I) has 2 or 3 substituents which are halogen atoms.
[5] The compound according to any one of [1] to [4] or a pharmaceutically acceptable salt thereof, wherein
   in the general formula (I), R₂ in the ring (II) represents a methyl group, or R₃ in the ring (III) represents a hydrogen atom or a methyl group.
[6] The compound according to any one of [3] to [5] or a pharmaceutically acceptable salt thereof, wherein L₂ in the substituent (V-I) in the general formula (I) represents a bond or an alkylene group having 1 to 5 carbon atoms.
[7] The compound according to any one of [3] to [6] or a pharmaceutically acceptable salt thereof, wherein
   in the general formula (I), each of R₁₆ and R₁₇ in the substituent (V-I) represents a hydrogen atom, and R₇ in the substituent (VI-I) represents a hydrogen atom.
[8] A pharmaceutical composition comprising the compound according to any one of [1] to [7] or a pharmaceutically acceptable salt thereof.
[9] A pharmaceutical composition for treating diabetes mellitus, comprising the compound according to any one of [1] to [7] or a pharmaceutically acceptable salt thereof.
[10] A glycogen synthase activator comprising the compound according to any one of [1] to [7] or a pharmaceutically acceptable salt thereof.

### Description of Embodiments

Hereinafter, definitions of the compound of the formula (I) are described.

In this description, an "alkyl group" is a monovalent group derived from a linear or branched aliphatic hydrocarbon having 1 to 12, preferably 1 to 6 carbon atoms by removing one hydrogen atom at any position. Specifically, the alkyl group may be a methyl, ethyl, isopropyl, butyl, n-butyl, isobutyl, sec-butyl, t-butyl, pentyl, isopentyl, 2,3-dimethylpropyl, or hexyl group or the like, and is more preferably C₁₋₄ alkyl.

An "alkylene group" is a divalent group derived from a linear or branched aliphatic hydrocarbon having 1 to 6 carbon atoms by removing two hydrogen atoms at any positions. The alkylene group may be a methylene, ethylene, propylene, or butylene group or the like. The alkylene group is preferably an alkylene group having 1 to 3 carbon atoms, and further preferably a methylene group or an ethylene group.

A "heterocyclic group" means a group obtainable by removing one hydrogen atom from a 5-membered or 6-membered saturated or unsaturated ring (heterocycle) containing 1 to 3 heteroatoms selected from oxygen atoms, sulfur atoms, and nitrogen atoms.

The saturated heterocyclic group may specifically be a group obtainable by removing one hydrogen atom from piperidine, piperazine, pyrrolidine, tetrahydrofuran, tetrahydropyran, or the like.

The unsaturated heterocyclic group may specifically be a group obtainable by removing one hydrogen atom from thiophene, furan, oxazole, isoxazole, thiazole, isothiazole, imidazole, pyrazole, oxadiazole, oxathiazole, triazole, pyridine, pyrimidine, pyrazine, pyridazine, or the like.

A "halogen atom" means a fluorine, chlorine, bromine, or iodine atom or the like.

An "alkoxy group" means an alkyl-O- group having 1 to 6 carbon atoms. Specifically, the alkoxy group may be a methoxy, ethoxy, 1-propoxy, 2-propoxy, n-butoxy, i-butoxy, sec-butoxy, t-butoxy, 1-pentyloxy, 2-pentyloxy, 3-pentyloxy, 2-methyl-1-butyloxy, 3-methyl-1-butyloxy, 2-methyl-2-butyloxy, 3-methyl-2-butyloxy, 2,2-dimethyl-1-propyloxy, 1-hexyloxy, 2-hexyloxy, or 3-hexyloxy group or the like. The alkoxy group is preferably an alkoxy having 1 to 3 carbon atoms.

A "halogenoalkoxy group" means a group which is the same as the above-described alkoxy group, except that the group is substituted with one or more halogen atoms.

A "monoalkylamino group" means alkyl-NH-, which is an amino group in which one hydrogen atom on the nitrogen atom is substituted by the above-described alkyl. Specifically, the monoalkylamino group may be a methylamino or ethylamino group or the like, and is preferably a monoalkylamino group having 1 to 3 carbon atoms.

A "monoalkylaminoalkyl group" means alkyl-NH-alkyl-, which is an alkyl group substituted with the above-described monoalkylamino group. The alkyl group is as described above. The monoalkylaminoalkyl group is preferably a monoalkylaminoalkyl group in which each alkyl group has 1 to 3 carbon atoms.

A "dialkylamino group" means (alkyl)₂N-, which is an amino group in which the two hydrogen atoms on the nitrogen atom are each substituted by the above-described alkyl. The alkyl groups may be the same or different. Specifically, the dialkylamino group may be a dimethylamino or diethylamino group or the like. The dialkylamino group is preferably dialkylamino in which each alkyl group has 1 to 4 carbon atoms. In addition, the two alkyl groups may share a carbon atom to form a ring having 3 to 6 carbon atoms, and such a dialkylamino group is specifically a pyrrolidinyl or piperidinyl group or the like.

A "dialkylaminoalkyl group" means (alkyl)₂N-alkyl-, which is an alkyl group substituted with the above-described dialkylamino groups. The alkyl groups are as described above. The dialkylaminoalkyl group is preferably a dialkylaminoalkyl group in which each alkyl group has 1 to 4 carbon atoms.

An "alkylsulfonyl group" means alkyl-SO₂-, which is a sulfonyl group substituted with the above-described alkyl. The alkyl is as described above. The alkylsulfonyl group is preferably alkylsulfonyl having 1 to 3 carbon atoms.

An "alkylsulfonylalkyl group" means alkyl-SO₂-alkyl, which is an alkyl group substituted with the above-described alkylsulfonyl. The alkyl is as described above. The alkylsulfonylalkyl group is preferably alkylsulfonylalkyl in which each alkyl group has 1 to 3 carbon atoms.

An "alkylcarbonylamino group" means alkyl-C (O) NH-, which is a carbonylamino group substituted with the above-described alkyl. The alkyl is as described above. The alkylcarbonylamino group is preferably an alkylcarbonylamino group in which the alkyl group has 1 to 3 carbon atoms.

An "alkylcarbonylaminoalkyl group" means alkyl-C(O)NH-alkyl, which is an alkyl group substituted with the above-described alkylcarbonylamino. The alkyl is as described above. The alkylcarbonylaminoalkyl group is preferably an alkylcarbonylaminoalkyl group in which each alkyl group has 1 to 3 carbon atoms.

An "alkylsulfonylamino group" means alkyl-SO₂-NH-, which is a sulfonylamino group substituted with the above-described alkyl. The alkyl is as described above. The alkylsulfonylamino group is preferably an alkylsulfonylamino group having 1 to 3 carbon atoms.

An "alkylsulfonylaminoalkyl group" means alkyl-SO₂-NH-alkyl, which is an alkyl group substituted with the above-described alkylsulfonylamino group. The alkyl is as described above. The alkylsulfonylaminoalkyl group is preferably an alkylsulfonylaminoalkyl group in which each alkyl group has 1 to 3 carbon atoms.

A "monoalkylaminocarbonyl group" means alkyl-NH-C(O)-, which is a carbonyl group to which an amino group in which one hydrogen atom on the nitrogen atom is substituted with the above-described alkyl is attached. The alkyl groups may be the same or different. Specifically, the monoalkylaminocarbonyl group may be a methylaminocarbonyl or ethylaminocarbonyl group or the like. The monoalkylaminocarbonyl group is preferably a monoalkylaminocarbonyl group in which the alkyl group has 1 to 3 carbon atoms.

A "monoalkylaminocarbonylalkyl group" means alkyl-NH-C(O)-alkyl-, which is an alkyl group substituted with the above-described monoalkylaminocarbonyl group. The alkyl groups are as described above. Specifically, the monoalkylaminocarbonylalkyl group may be a methylaminocarbonylalkyl or ethylaminocarbonylalkyl group or the like. The monoalkylaminocarbonylalkyl group is preferably a monoalkylaminocarbonylalkyl group in which each alkyl group has 1 to 3 carbon atoms.

A "dialkylaminocarbonyl group" means (alkyl)₂N-C(O)-, which is a carbonyl group to which an amino group in which the two hydrogen atoms on the nitrogen atom are each substituted by the above-described alkyl is attached. The alkyl groups may be the same or different. Specifically, the dialkylaminocarbonyl group may be a carbonyl group to which a dimethylamino or diethylamino group or the like is attached. In addition, the two alkyl groups may share a carbon atom to form a ring having 3 to 6 carbon atoms. Specifically, such a dialkylaminocarbonyl group may be a pyrrolidinylcarbonyl or piperidinylcarbonyl group or the like.

An "dialkylaminocarbonylalkyl group" means (alkyl)₂N-C (O) -alkyl-, which is an alkyl group substituted with the above-described dialkylaminocarbonyl group. The alkyl groups are as described above. The alkyl groups may be the same or different. Specifically, the dialkylaminocarbonylalkyl group may be a carbonyl group to which a dimethylamino or diethylamino group or the like is attached. In addition, the alkyl groups may share a carbon atom to form a ring having 3 to 6 carbon atoms. Specifically, the dialkylaminocarbonylalkyl group may be a group such as a pyrrolidinylcarbonylalkyl group or a piperidinylcarbonylalkyl group.

An "aminocarbonylalkyl group" means NH₂-C(O)-alkyl-, which is an alkyl group substituted with an aminocarbonyl group. The alkyl group is as described above. The aminocarbonylalkyl group is preferably an aminocarbonylalkyl group in which the alkyl group has 1 to 3 carbon atoms.

An "aminoalkyl group" means NH₂-alkyl-, which is an alkyl group substituted with an amino group. The alkyl group is as described above. The aminoalkyl group is preferably an aminoalkyl group in which the alkyl group has 1 to 4 carbon atoms.

A "cycloalkyl group" is a cyclic alkyl group having 3 to 10 carbon atoms. Specifically, the cycloalkyl group may be cyclopropyl, cyclobutyl, cyclopentyl, cyclohexyl, cycloheptyl, cyclooctyl, or the like. The cycloalkyl group is preferably a cyclic alkyl group having 3 to 6 carbon atoms, such as a cyclopropyl, cyclopentyl, or cyclohexyl group. The cycloalkyl group is more preferably cyclopropyl or cyclohexyl.

An "aminocycloalkyl group" means an H₂N-cycloalkyl group, which is the same group as the above-described cycloalkyl group, except that an amino group is attached. Specifically, the aminocycloalkyl group may be an aminocyclopropyl, 2-aminocyclobutyl, 3-aminocyclobutyl, 2-aminocyclopentyl, 3-aminocyclopentyl, 2-aminocyclohexyl, 3-aminocyclohexyl, or 4-aminocyclohexyl group, or the like. The aminocycloalkyl group is preferably an aminocycloalkyl group having 3 to 6 carbon atoms.

A "monoalkylaminocycloalkyl group" means an alkyl-NH-cycloalkyl group, which is the same group as the above-described cycloalkyl group, except that the above-described monoalkylamino group is attached. The monoalkylaminocycloalkyl group is preferably a monoalkylaminocycloalkyl group in which the alkyl group has 3 to 6 carbon atoms.

A "dialkylaminocycloalkyl group" means an (alkyl)₂-N-cycloalkyl group, which is the same group as the above-described cycloalkyl group, except that the above-described dialkylamino group is attached. The dialkylaminocycloalkyl group is preferably a monoalkylaminocycloalkyl group having 3 to 6 carbon atoms.

An "aminosulfonylalkyl group" means NH₂-SO₂-alkyl-, which is an alkyl group substituted with an aminosulfonyl group. The alkyl group is as described above. The aminosulfonylalkyl group is preferably an aminosulfonylalkyl group in which the alkyl group has 1 to 4 carbon atoms.

A "monoalkylaminosulfonylalkyl group" means alkyl-NH-SO₂-alkyl-, which is an alkyl group substituted with a monoalkylaminosulfonyl group. The alkyl group is as described above. The monoalkylaminosulfonylalkyl group is preferably a monoalkylaminosulfonylalkyl group in which each alkyl group has 1 to 4 carbon atoms.

A "dialkylaminosulfonylalkyl group" means (alkyl)₂-N-SO₂-alkyl-, which is an alkyl group substituted with a dialkylaminosulfonyl group. The alkyl groups are as described above. The dialkylaminosulfonylalkyl group is preferably a dialkylaminosulfonylalkyl group in which each alkyl group has 1 to 4 carbon atoms.

An "alkoxyalkyl group" means alkyl-O-alkyl-, which is an alkyl group substituted with an alkoxy group. The alkoxy group and the alkyl group are as described above. The alkoxyalkyl group is preferably an alkoxy alkyl group in which each of the alkoxy group and the alkyl group has 1 to 4 carbon atoms.

A "monoalkoxyaminoalkyl group" means alkyl-O-NH-alkyl-, which is an alkyl group substituted with a monoalkoxyamino group. The alkyl group is as described above. The monoalkoxyaminoalkyl group is preferably a monoalkoxyaminoalkyl group in which each alkyl group has 1 to 4 carbon atoms.

An "alkoxyalkyleneoxyalkyl group" means alkyl-O-alkylene-O-alkyl-, which is an alkyl group substituted with an alkoxyalkyleneoxy group. The alkyl groups and the alkylene group are as described above. The alkoxyalkyleneoxyalkyl group is preferably an alkoxyalkyleneoxyalkyl group in which each of the alkyl group and the alkylene group has 1 to 4 carbon atoms.

A "hydroxyalkyl group" means HO-alkyl-, which is an alkyl group substituted with a hydroxy group. The alkyl group is as described above. The hydroxyalkyl group is preferably a hydroxyalkyl group having 1 to 5 carbon atoms.

A "carboxyalkyl group" means HO(O)C-alkyl-, which is an alkyl group substituted with a carboxyl group. The alkyl group is as described above. The carboxyalkyl group is preferably a carboxyalkyl group having 2 to 5 carbon atoms.

A "cyanoalkyl group" means NC-alkyl-, which is an alkyl group substituted with a cyano group. The alkyl group is as described above. The cyanoalkyl group is preferably a cyanoalkyl group having 2 to 5 carbon atoms.

In addition, the group of the general formula (II) represented by Ar₁ and the group of the general formula (III) represented by Ar₁ each may have 1 to 4 substituents, which may be the same or different. In particular, each substituent is preferably a halogen atom, a hydroxyalkyl group, an alkyl group, a cyano group, or an amino group. The group more preferably has 1 to 3 of these substituents, and particularly preferably has 2 or 3 of these substituents. Especially, halogen atoms such as fluorine or chlorine atoms and methyl groups are preferable.

The bond between L₂ and the pyrrolidine ring in the formula (V-I) may be in any configuration. Meanwhile, the substituent represented by the formula (V-II) preferably has the configuration shown below:

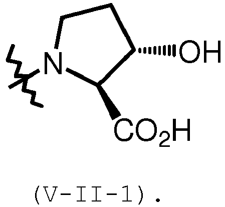

In the general formula (IV), R₄ is preferably a cyanoalkyl group, an aminocarbonylalkyl group, a dialkylaminocarbonylalkyl group, a monoalkylaminocarbonylalkyl group, an alkylsulfonylalkyl group, an aminoalkyl group, a monoalkylaminoalkyl group, a dialkylaminoalkyl group, an alkoxyalkyl group, an alkylsulfonylaminoalkyl group, a hydroxyalkyl group, or a carboxyalkyl group. Alternatively, R₄ is preferably an aminosulfonylalkyl group, a monoalkylaminosulfonylalkyl group, a dialkylaminosulfonylalkyl group, an aminocycloalkyl group, a monoalkylaminocycloalkyl group, a dialkylaminocycloalkyl group, a monoalkoxyaminoalkyl group, an alkoxyalkyleneoxyalkyl group, or an alkylcarbonylaminoalkyl group. Still alternatively, R₄ is preferably a group represented by -L₁-B, where L₁ represents a bond or an alkylene group, and B represents an optionally substituted 5-membered or 6-membered heterocyclic group having at least one heteroatom selected from nitrogen atoms, oxygen atoms, and sulfur atoms.

Here, the alkylene group represented by L₁ in the group represented by -L₁-B may be a linear or branched alkylene group. The alkylene group is preferably an alkylene group having 1 to 3 carbon atoms, and is particularly preferably an alkylene group having 1 or 2 carbon atoms. L₁ is also preferably a bond. Meanwhile, the heterocyclic group B is preferably a 5-membered or 6-membered heterocyclic group having one nitrogen, oxygen, or sulfur atom, a 5-membered or 6-membered heterocyclic group having one nitrogen atom and one oxygen or sulfur atom, a 5-membered or 6-membered heterocyclic group having two nitrogen atoms and one oxygen or sulfur atom, or a 5-membered or 6-membered heterocyclic group having two or three nitrogen atoms.

In particular, the heterocyclic group B is preferably one represented by any of the above-described formulae (VI) to (XX). In these formulae, the alkyl group represented by each of R₇ to R₁₅ is preferably a linear or branched alkyl group having 1 to 3 carbon atoms, and is particularly preferably an alkyl group having 1 or 2 carbon atoms. The alkyl group in the alkylcarbonyl group is also the same. R₇ to R₁₅ are each preferably a hydrogen atom.

R₄ in the formula (IV) is also preferably one represented by any of the following formulae (VI-I), (VII-I), and (XI-I):

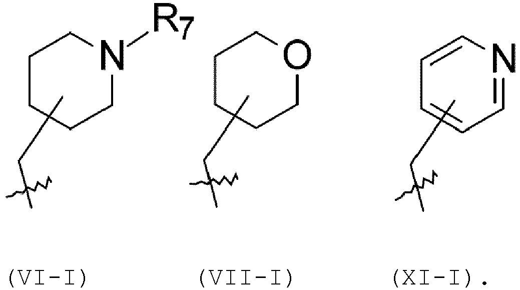

In the general formula (V-I), one or two of R₁₆ and R₁₇ are preferably hydrogen atoms. Meanwhile, when R₁₆ and R₁₇ taken together form a ring, the ring is preferably a 4- to 7-membered cyclic amine which contains at least one nitrogen atom and which may contain oxygen and sulfur atoms. A pyrrolidine ring or a piperidine ring is particularly preferable. R₇ is preferably a hydrogen atom.

R₄ in the substituent of the formula (IV) represented by R₁ is preferably a cyanoalkyl group, a monoalkylaminoalkyl group, an alkoxyalkyl group, an alkoxyalkyleneoxyalkyl group, or a group represented by -L₁-B, where the ring B is represented by (VI), (XI), (XII), (XIII), (XIV), (XV), (XIX), or (XX).

The alkylene group represented by L₂ in -L₂-R₆ of the substituent of the formula (V) represented by R₁ may be a linear or branched alkylene group. The alkylene group is preferably an alkylene group having 1 to 3 carbon atoms, and is particularly preferably an alkylene group having one or two carbon atoms. L₂ is also preferably a bond.

Meanwhile, R₆ is preferably an alkoxy group, a cyano group, a monoalkylamino group, or a dialkylamino group. Especially, -L₂-R₆ is particularly preferably an alkoxy group, an alkoxyalkyl group, a dialkylamino group, or a cyanoamino group.

Compounds of the present invention represented by the general formula (I) and pharmaceutically acceptable salts thereof can be synthesized, for example, according to the following reaction formulae A to D.

In the following reaction formulae, Ar₁ and R₄ are the same groups as defined in the above-described formula (I). In addition, each of M₁, M₂, and M₃ in the reaction formulae (A) to (D) represents a functional group (for example, a halogen atom) which enables coupling with the compound represented by the corresponding one of the formulae (A-2), (A-3), (B-1), and (B-3). Rₐ and R_{b} each independently represent a hydroxy group or an alkoxy group, and may be taken together to form a ring. Y represents an alkyl group or a benzyl group.

### Method for Synthesizing Biarylcarboxylic Acid Derivative (A-6)

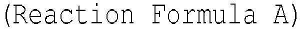

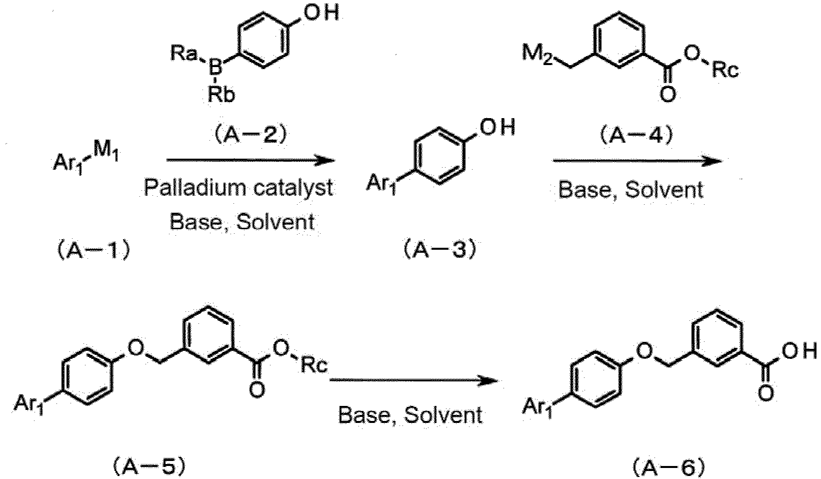

A biaryl derivative (A-3) can be obtained from an aryl halide reagent (A-1) having a corresponding Ar₁ and a phenol derivative (A-2), which is a boronic acid reagent (for example, Rₐ and R_{b} are hydroxy groups) and which undergoes a coupling reaction with the aryl halide reagent (A-1) in a solvent such as N, N-dimethylformamide by using a metal catalyst or the like, in the presence of a palladium catalyst such as [1,1'-bis(diphenylphosphino)ferrocene]dichloropalladium(II) and a base such as sodium carbonate, if necessary, by subjecting the reaction system to cooling, heating, or the like. An ester derivative (A-5) can be obtained from the obtained biaryl derivative (A-3) and a corresponding benzoic acid ester derivative (A-4), for example, in a solvent such as N,N-dimethylformamide in the presence of a base such as potassium carbonate, if necessary, by subjecting the reaction system to cooling, heating, or the like. A biarylcarboxylic acid derivative (A-6) can be obtained from the obtained ester derivative (A-5), for example, in a solvent such as tetrahydrofuran or methanol in the presence of a base such as sodium hydroxide, if necessary, by subjecting the reaction system to cooling, heating, or the like.

### Method for Synthesizing Glycine Ester Derivative (B-3)

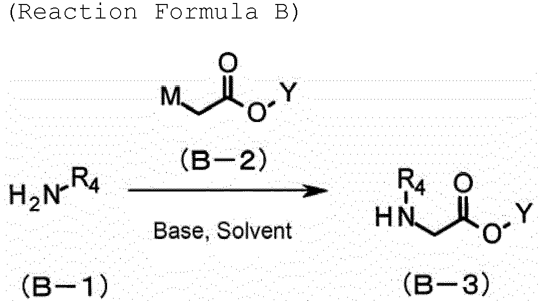

A glycine derivative (B-3) can be obtained from an amine derivative having a corresponding R₄ or a salt thereof (B-1) and a corresponding ester derivative (B-2) such as bromobenzylacetic acid, for example, in a solvent such as acetonitrile in the presence of a base such as potassium carbonate, if necessary, by subjecting the reaction system to cooling, heating, or the like.

### Method for Synthesizing Amide Ester Derivative (C-2)

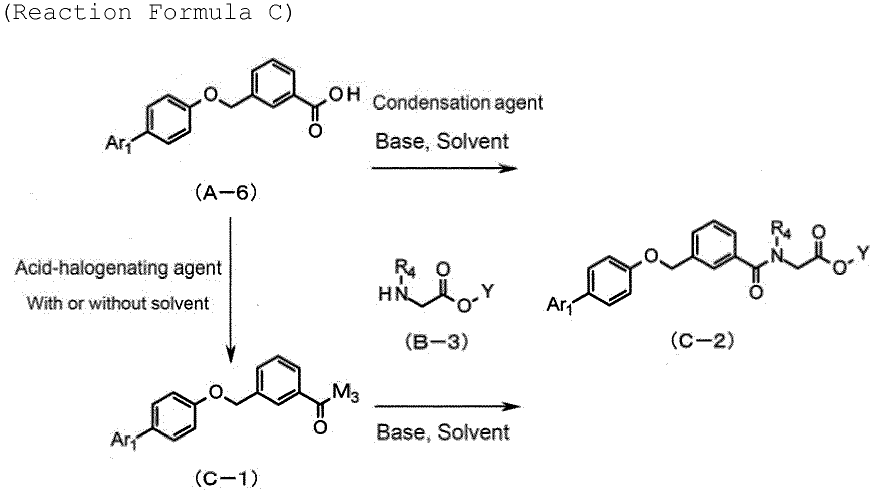

The corresponding carboxylic acid derivative (A-6) can be converted to an amide ester derivative (C-2) by, for example, one of the two production methods. One is a production method using a condensation agent and the like. The amide ester derivative (C-2) can be obtained from the corresponding carboxylic acid derivative (A-6) and the corresponding glycine ester derivative (B-3), for example, in a solvent such as dichloromethane in the presence of a condensation agent such as 1-ethyl-3-(3-dimethylaminopropyl)-carbodiimide hydrochloride and a base such as diisopropylethylamine, if necessary, by subjecting the reaction system to cooling, heating, or the like. In the other production method, the amide ester derivative (C-2) can be produced through an acid halide (C-1). The acid halide derivative (C-1) can be obtained from the corresponding carboxylic acid derivative (A-6), for example, without a solvent or in a solvent such as N,N-dimethylformamide in the presence of an acid-halogenating agent such as thionyl chloride, if necessary, by subjecting the reaction system to cooling, heating, or the like. The amide ester derivative (C-2) can be obtained from the obtained acid halide derivative (C-1) and the corresponding glycine ester derivative (B-3), for example, in a solvent such as dichloromethane in the presence of a base such as diisopropylethylamine, if necessary, by subjecting the reaction system to cooling, heating, or the like.

### Method for Synthesizing Amide Carboxylic Acid Derivative (D-1)

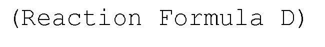

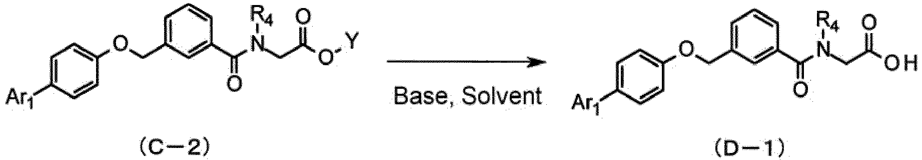

An amide carboxylic acid derivative (D-1) can be obtained from the corresponding amide ester derivative (C-2), for example, in a solution of tetrahydrofuran, methanol, or the like in the presence of a base such as an aqueous solution of sodium hydroxide or lithium hydroxide, if necessary, by subjecting the reaction system to cooling, heating, or the like.

In embodiments in which R₁ in the formula (I) is represented by the formula (V), such a compound can be synthesized by employing the same method and by replacing the compound of the formula (B-3) in the reaction formula C with a proline ester derivative having a corresponding R₅.

Alternatively, compounds of the present invention represented by the general formula (I) and pharmaceutically acceptable salts thereof can be synthesized according to the following synthesis scheme.

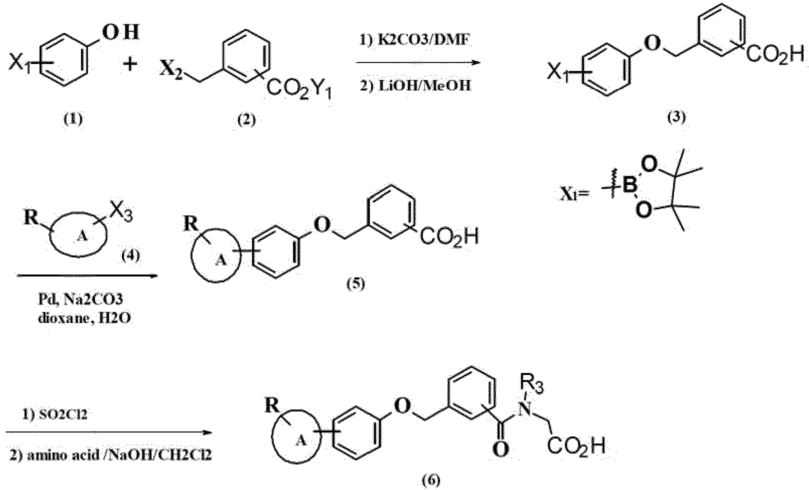

An ester derivative can be obtained, for example, by treating a phenol derivative (1) in which X₁ is a boronic acid derivative with a benzoic acid ester derivative (2) in which X₂ is a halogen atom in a solvent such as DMF in the presence of a base such as potassium carbonate. This ester derivative is converted to a carboxylic acid (3) by hydrolysis, for example, in a solvent such as methanol in the presence of a base such as lithium hydroxide. A compound (5) can be obtained, for example, by a coupling reaction between the carboxylic acid (3) and any of various derivatives (4) in which X3 is a halogen atom in a solvent such as dioxane or water in the presence of a base such as sodium carbonate by using Pd or the like as a catalyst. After that, the compound (5) is converted to an acid chloride by using, for example, thionyl chloride or the like. Then, an amide (6) can be obtained, for example, by treating the acid chloride with any one of various amino acids in a solvent such as dichloromethane in the presence of a base such as sodium hydroxide.

In the present invention, when the compound represented by general formula (I) can form salts, the salts may be any, as long as the salts are pharmaceutically acceptable. For example, when an acidic group such as a carboxyl group is present in the formula, the salts formed with the acidic group include ammonium salts; salts with alkali metals such as sodium and potassium; salts with alkaline earth metals such as calcium and magnesium; aluminum salts; zinc salts; salts with organic amines such as triethylamine, ethanolamine, morpholine, piperidine, and dicyclohexylamine; and salts with basic amino acids such as arginine and lysine. Especially, it is preferable to use sodium.

When a basic group is present in the formula, the salts formed with the basic group include salts with inorganic acids such as hydrochloric acid, sulfuric acid, phosphoric acid, nitric acid, and hydrobromic acid; salts with organic carboxylic acids such as acetic acid, trifluoroacetic acid, citric acid, benzoic acid, maleic acid, fumaric acid, tartaric acid, succinic acid, tannic acid, butyric acid, hibenzic acid, pamoic acid, enanthic acid, decanoic acid, teoclic acid, salicylic acid, lactic acid, oxalic acid, mandelic acid, and malic acid; and salts with organic sulfonic acids such as methanesulfonic acid, benzenesulfonic acid, and p-toluenesulfonic acid. Especially, it is preferable to use hydrochloric acid or trifluoroacetic acid.

Regarding a method for forming the salt, the salt can be obtained by mixing the compound represented by general formula (I) with a necessary acid or base at a suitable amount ratio in a solvent or a dispersant, or by converting another salt by cation exchange or anion exchange.

The compound of the present invention includes solvates, such as hydrates and alcohol adducts, of the compound represented by general formula (I).

The compound of the present invention can also be converted to a prodrug. The prodrug in the present invention refers to a compound which can be converted in vivo to form the compound of the present invention. For example, when the active form has a carboxyl group or the like, the prodrug may be an ester or amide thereof or the like. Meanwhile, when the active form has an amino group, the prodrug may be an amide or carbamate thereof or the like. When the active form has a hydroxyl group, the prodrug may be an ester, carbonate, or carbamate thereof or the like. When a prodrug is formed from the compound of the present invention, it is also possible to bond the compound of the present invention to an amino acid or a saccharide.

The present invention includes all isotopically substituted forms of the compound represented by general formula (I). An isotopically substituted form of the compound of the present invention is one in which at least one atom is replaced by another atom which has the same atomic number (proton number) but a different mass number (the sum of the number of protons and the number of neutrons). Examples of the isotopes contained in the compound of the present invention include hydrogen atoms, carbon atoms, nitrogen atoms, oxygen atoms, phosphorus atoms, sulfur atoms, fluorine atoms, chlorine atoms, and the like, which include 2H, 3H, 13C, 14C, 15N, 170, 180, 31P, 32P, 35S, 18F, 36Cl, and the like. Especially, unstable radioisotopes, such as 3H and 14C, which exhibit radioactivity and release neutrons, are useful for a body tissue distribution test of drugs or compounds, and the like. Stable isotopes can be used safely, because they do not decay, undergo little change in the abundance, and do not have radioactivity. The isotope in the compound of the present invention can be introduced in a usual manner by replacing a reagent used for synthesis with a corresponding reagent containing the isotope.

The pharmaceutical composition of the present invention can be preferably used for treatment of a disease mediated by a decrease in activity of glycogen synthase. Especially, the pharmaceutical composition of the present invention can be preferably used for treatment of diabetes mellitus, especially type 2 diabetes and impaired glucose tolerance.

The administration amount of each of the pharmaceutical composition and the glycogen synthase activator of the present invention varies depending on the subject of the administration, the administration route, the target disease, the symptom, and the like. The administration route is preferably oral administration, and the amount of the active ingredient administered in a single time is preferably 1 mg to 1000 mg/person, and more preferably 1 mg to 100 mg/person. It is desirable to administer the pharmaceutical composition or the glycogen synthase activator of the present invention in this amount one to three times per day.

The pharmaceutical composition and the glycogen synthase activator of the present invention comprise, as an active ingredient, the above-described compound represented by general formula (I) and/or a pharmaceutically acceptable salt thereof. The pharmaceutical composition and the glycogen synthase activator of the present invention may contain various components generally used for orally administered drugs, for example, pharmaceutically or physiologically acceptable solid or liquid carriers and additives, and the like.

Examples of the carriers include glucose, lactose, sucrose, starch, mannitol, dextrin, fatty acid glyceride, polyethylene glycol, hydroxyethyl starch, ethylene glycol, polyoxyethylene sorbitan fatty acid ester, gelatin, albumin, amino acid, water, physiological saline, and the like. In addition, if necessary, conventionally used additives such as stabilizers, wetting agents, emulsifiers, binders, tonicity adjusting agents, and the like can also be added, as appropriate.

The additives are not particularly limited, as long as the additives are ones which are generally used for an intended purpose. Specific examples thereof include flavors, saccharides, sweeteners, dietary fibers, vitamins, amino acids such as sodium glutamate (MSG), nucleic acids such as inosine monophosphate (IMP), inorganic salts such as sodium chloride, water, and the like.

The pharmaceutical composition and the glycogen synthase activator of the present invention can be used in orally administrable forms such as a dry powder, a paste, and a solution without any limitation on physical properties.

Examples of such an orally administrable form include tablets (including sugar-coated tablets, film-coated tablets, sublingual tablets, and orally disintegrating tablets), capsules (including soft capsules and microcapsules), granules, powders, troches, syrups, emulsions, suspensions, films (for example, orally disintegrating films), lyophilized formulations, and the like.

In addition, the pharmaceutical composition and the glycogen synthase activator of the present invention can also be used in the forms of parenteral preparations such as injections (for example, subcutaneous injections, intravenous injections, intramuscular injections, intraperitoneal injections, and infusions), external preparations (for example, transdermal preparations and ointments), suppositories (for example, rectal suppositories and vaginal suppositories), pellets, intranasal agents, transpulmonary agents (inhalants), ophthalmic solutions, and the like.

These preparations can be safely administered orally or parenterally (for example, locally, rectally, or intravenously administered). These preparations may be controlled-release preparations such as immediate-release preparations and sustained-release preparations (for example, sustained-release microcapsules). These preparations can be prepared by pharmaceutically common means.

In addition, the pharmaceutical composition and the glycogen synthase activator of the present invention can be used in combination with other drugs for treating diabetes, drugs for treating diabetic complications, drugs for treating hyperlipidemia, antihypertensives, and anti-obesity agents (hereinafter, generally referred to as concomitant drugs). These concomitant drugs may be low in molecule. Alternatively, these concomitant drugs may be high-molecular weight proteins, polypeptides, antibodies, and nucleic acids (including antisense nucleic acids, siRNAs, and shRNAs), or may be vaccines or the like. One of these concomitant drugs can be used, or two or more thereof can be used in combination.

There is no limitation on the administration timing of the pharmaceutical composition and the glycogen synthase activator of the present invention or the concomitant drug. These may be administered to the subject of administration simultaneously or at any interval.

Note that the drugs for treating diabetes include insulin preparations (for example, animal insulin preparations extracted from the pancreas of cattle or pigs; human insulin preparations synthesized by genetic engineering using Escherichia coli or yeast; insulin zinc; protamine insulin zinc; insulin fragments or derivatives (for example, INS-1), and oral insulin preparations), insulin resistance improvers (for example, pioglitazone or salts thereof (preferably hydrochloride), rosiglitazone or salts thereof (preferably maleate), tesaglitazar, ragaglitazar, muraglitazar, edaglitazone, metaglidasen, naveglitazar, AMG-131, and THR-0921), α-glucosidase inhibitors (for example, voglibose, acarbose, miglitol, and emiglitate), biguanides (for example, metformin, buformin, and salts thereof (for example, hydrochlorides, fumarates, and succinates)), insulin secretion promoters [sulfonylureas (for example, tolbutamide, glibenclamide, gliclazide, chlorpropamide, tolazamide, acetohexamide, glyclopyramide, glimepiride, glipizide, and glybuzole), repaglinide, nateglinide, mitiglinide, and calcium salt hydrates thereof], dipeptidyl peptidase IV inhibitors (for example, alogliptin, vildagliptin, sitagliptin, saxagliptin, T-6666, and TS-021), β3 agonists (for example, AJ-9677), GPR40 agonists, GPR120 agonists, GLP-1 receptor agonists [for example, GLP-1, GLP-1MR agent, NN-2211, AC-2993 (exendin-4), BIM-51077, Aib (8, 35) hGLP-1 (7, 37) NH₂, and CJC-1131], amylin agonists (for example, pramlintide), phosphotyrosine phosphatase inhibitors (for example, sodium vanadate), gluconeogenesis inhibitors (for example, glycogen phosphorylase inhibitors, glucose-6-phosphatase inhibitors, and glucagon antagonists), SGLT (sodium-glucose cotransporter) inhibitors (for example, dapagliflozin, canagliflozin, ipragliflozin, and BI-10773), 11β-hydroxysteroid dehydrogenase inhibitors (for example, BVT-3498), adiponectin and agonists thereof, IKK inhibitors (for example, AS-2868), leptin resistance improvers, somatostatin receptor agonists, glucokinase activators (for example, Ro-28-1675), GIP (Glucose-dependent insulinotropic peptide), and the like.

Further, the compound represented by general formula (I) and/or the pharmaceutically acceptable salt thereof can be used in such forms employed for supplements and the like that it is enclosed in a granule, a tablet, a gelatin capsule, or the like.

### Examples

Hereinafter, the present invention will be described in detail based on Examples; however, the present invention is not limited to these Examples.

### Synthesis of Intermediate 1-A 3-[[4-(4,5-Difluorobenzofuran-7-yl)phenoxy]methyl]benzoic Acid

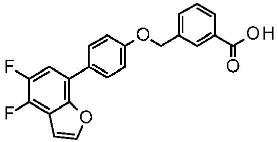

### Step 1

### Synthesis of 7-Bromo-4,5-difluoro-1-benzofuran

To 2-bromo-4,5-difluorophenol (2.51 g, 12.0 mmol) and potassium carbonate (3.32 g, 24.0 mmol) in N,N-dimethylformamide (hereinafter, DMF) (60 mL), bromoacetaldehyde dimethyl acetal (2.82 mL, 24.0 mmol) and a catalytic amount of sodium iodide were added, followed by stirring at 80°C overnight. The solvent was evaporated under reduced pressure. The residue was diluted with ethyl acetate, washed with water and saturated aqueous sodium chloride, and then dried over anhydrous magnesium sulfate. The solvent was evaporated under reduced pressure, and the obtained residue was purified by silica gel chromatography (hexane/ethyl acetate). The obtained compound (3.00 g, 10.1 mmol) was dissolved in chlorobenzene (40 mL), and the solution was added to a suspension (20 mL) of polyphosphoric acid (3.0 g) in chlorobenzene at 120°C. The reaction liquid was stirred at 120°C overnight, and then the solvent was evaporated under reduced pressure. To the residue, ethyl acetate and water were added. This mixture was poured into a 1 N aqueous sodium hydroxide solution under ice cooling, followed by stirring. Then, insoluble matters were separated by filtration, followed by extraction with ethyl acetate. The organic layer was washed with saturated aqueous sodium chloride, and then dried over anhydrous magnesium sulfate. The solvent was evaporated under reduced pressure, and the obtained residue was purified by silica gel chromatography (hexane) to obtain the title compound.
Yield: 572 mg (2.45 mmol), Percentage yield: 20%
¹H NMR (400 MHz, CDCl₃) δ 7.69 (d, J = 2.2 Hz, 1H), 7.31-7.38 (m, 1H), 6.98 (d, J = 2.2 Hz, 1H).

### Step 2

### Synthesis of Intermediate 1-A

DMF (125 mL) was added to 4-(4,4,5,5-tetramethyl-1,3,2-dioxaborolan-2-yl)phenol (8.46 g, 38.4 mmol), methyl 3-(bromomethyl)benzoate (8.80 g, 38.4 mmol), and potassium carbonate (10.6 g, 76.8 mmol), and the mixture was stirred at room temperature overnight. The mixture was diluted with ethyl acetate, washed with water and saturated aqueous sodium chloride, and then dried over anhydrous magnesium sulfate. The solvent was evaporated under reduced pressure. To the obtained residue, methanol (150 mL), water (30 mL), and lithium hydroxide (4.8 g, 114 mmol) were added, followed by stirring at room temperature overnight. After the solvent was evaporated under reduced pressure, the residue was diluted with ethyl acetate, then washed with 1 N hydrochloric acid and saturated aqueous sodium chloride, and dried over anhydrous magnesium sulfate. The solvent was evaporated under reduced pressure. Then, to a portion (3.34 g, 9.4 mmol) of the obtained residue, the compound obtained in Step 1 (2.2 g, 9.4 mmol), 1,4-dioxane (75 mL), water (25 mL), sodium carbonate (1.5 g, 14.2 mmol), and [1,1'-bis(diphenylphosphino)ferrocene]dichloropalladium(II) (hereinafter, PdCl₂(dppf)) (catalytic amount) were added, followed by stirring at 100°C for 2 hours. After insoluble matters were separated by filtration, the solvent was evaporated under reduced pressure. After that, the residue was diluted with ethyl acetate, then washed with 1 N hydrochloric acid and saturated aqueous sodium chloride, and dried over anhydrous magnesium sulfate. The solvent was evaporated under reduced pressure, and the obtained residue was washed with acetonitrile to obtain the title compound.
Yield: 2.95 g (7.76 mmol), Percentage yield: 83%

### Synthesis of Intermediate 1-B 3-[[4-(4,5-Difluorobenzofuran-7-yl)phenoxy]methyl]benzoyl Chloride

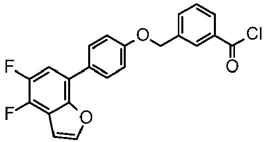

To Intermediate 1-A (2. 95 g, 7.76 mmol), thionyl chloride (15 mL) was added, followed by stirring at 50°C for 2 hours. After cooling, the solvent was evaporated under reduced pressure to obtain the title compound.
Yield: 2.4 g (6.0 mmol), Percentage yield: 77%

### Synthesis of Intermediate 2-A 3-[[4-(4,5-Difluoro-2-sulfanyl-phenyl)phenoxy]methyl]benzoi c Acid

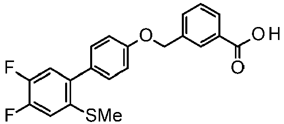

### Step 1

### Synthesis of 4-(4,5-Difluoro-2-methylsulfanyl-phenyl)phenol

To 1-bromo-4,5-difluoro-2-(methylthio)benzene (10.8 g, 45.0 mmol), 1,4-dioxane (75 mL), water (25 mL), 4-hydroxyphenylboronic acid (7.5 g, 54.3 mmol), sodium carbonate (9.6 g, 90 mmol), and PdCl₂(dppf) (catalytic amount) were added, followed by stirring at 100°C for 2 hours. After insoluble matters were separated by filtration, the solvent was evaporated under reduced pressure. After that, the residue was diluted with ethyl acetate, then washed with 1 N hydrochloric acid and saturated aqueous sodium chloride, and dried over anhydrous magnesium sulfate. The solvent was evaporated under reduced pressure, and the obtained residue was purified by silica gel chromatography to obtain the title compound. Yield: 11.3 g (45.0 mmol), Percentage yield: 100%

### Step 2

### Synthesis of 1-Bromo-4,5-difluoro-2-methylsulfanyl-benzene

To a DMF solution (100 mL) of the compound obtained in Step 1 (9.78 g, 43.5 mmol), potassium carbonate (7.5 g, 54.3 mmol) and iodomethane (3.39 mL, 54.3 mmol) were added, followed by stirring at room temperature overnight. The mixture was diluted with ethyl acetate, then washed with water and saturated aqueous sodium chloride, and then dried over anhydrous magnesium sulfate. The solvent was evaporated, and the obtained residue was purified by silica gel chromatography (hexane/ethyl acetate) to obtain the title compound.

### Step 3

### Synthesis of Intermediate 2-A

DMF (125 mL) was added to 4-(4,4,5,5-tetramethyl-1,3,2-dioxaborolan-2-yl)phenol (8.46 g, 38.4 mmol), methyl 3-(bromomethyl)benzoate (8.80 g, 38.4 mmol), and potassium carbonate (10.6 g, 77 mmol), followed by stirring at room temperature overnight. The mixture was diluted with ethyl acetate, washed with water and saturated aqueous sodium chloride, and then dried over anhydrous magnesium sulfate. The solvent was evaporated under reduced pressure. To the obtained residue, methanol (150 mL), water (30 mL), and lithium hydroxide (4.8 g, 114 mmol) were added, followed by stirring at room temperature overnight. After the solvent was evaporated under reduced pressure, the residue was diluted with ethyl acetate, then washed with 1 N hydrochloric acid and saturated aqueous sodium chloride, and dried over anhydrous magnesium sulfate. The solvent was evaporated under reduced pressure. To a portion (3.74 g, 10.6 mmol) of the obtained residue, 1-bromo-4,5-difluoro-2-(methylthio)benzene (2.53 g, 10.6 mmol) obtained in Step 1, 1,4-dioxane (75 mL), water (25 mL), sodium carbonate (2.24 g, 21.2 mmol), and PdCl₂ (dppf) (catalytic amount) were added, followed by stirring at 100°C for 2 hours. After insoluble matters were separated by filtration, the solvent was evaporated under reduced pressure. Then, the residue was diluted with ethyl acetate, washed with 1 N hydrochloric acid and saturated aqueous sodium chloride, and dried over anhydrous magnesium sulfate. The solvent was evaporated under reduced pressure, and the obtained residue was washed with acetonitrile to obtain the title compound.
Yield: 4.02 g (10.4 mmol), Percentage yield: 98%

### Synthesis of Intermediate 2-B 3-[[4-(4,5-Difluoro-2-sulfanyl-phenyl)phenoxy]methyl]benzoy 1 Chloride

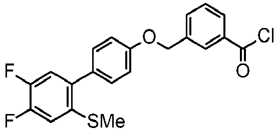

To Intermediate 2-A (4.0 g, 10.4 mmol), thionyl chloride (15 mL) was added, followed by stirring at 50°C for 2 hours. After cooling, the solvent was evaporated under reduced pressure to obtain the title compound.
Yield: 3.3 g (8.5 mmol), Percentage yield: 82%

### Synthesis of Intermediate 3 3-[[4-(4,5-Difluoro-3-methyl-benzofuran-7-yl)phenoxy]methyl ]benzoyl Chloride

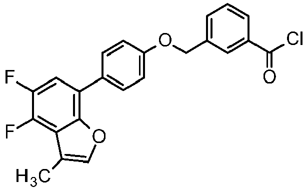

### Step 1

### Synthesis of 7-Bromo-4,5-difluoro-3-methyl-1-benzofuran

The title compound was obtained by conducting the same operation as in Step 1 of Intermediate 1-A by using bromoacetone (0.504 mL, 6.00 mmol) instead of bromoacetaldehyde dimethyl acetal.
Yield: 150 mg (0.607 mmol), Percentage yield: 12%
¹H NMR (400 MHz, CDCl₃) δ 7.43 - 7.40 (m, 1H), 7.27 - 7.32 (m, 1H), 2.35 (d, J = 0.9 Hz, 3H).

### Step 2

### Synthesis of Intermediate 3

DMF (125 mL) was added to 4-(4,4,5,5-tetramethyl-1,3,2-dioxaborolan-2-yl)phenol (8.46 g, 38 mmol), methyl 3-(bromomethyl)benzoate (8.8 g, 38 mmol), and potassium carbonate (10.6 g, 77 mmol), followed by stirring at room temperature overnight. The mixture was diluted with ethyl acetate, washed with water and saturated aqueous sodium chloride, and then dried over anhydrous magnesium sulfate. The solvent was evaporated under reduced pressure. To the obtained residue, methanol (150 mL), water (30 mL), and lithium hydroxide (4.8 g, 114 mmol) were added, followed by stirring at room temperature overnight. After the solvent was evaporated under reduced pressure, the residue was diluted with ethyl acetate, then washed with 1 N hydrochloric acid and saturated aqueous sodium chloride, and dried over anhydrous magnesium sulfate. The solvent was evaporated under reduced pressure. To a portion (6.25 g, 17.7 mmol) of the obtained residue, 7-bromo-4,5-difluoro-3-methylbenzofuran (4.8 g, 19.4 mmol), 1,4-dioxane (75 mL), water (25 mL), sodium carbonate (3.74 g, 35.3 mmol), and PdCl₂(dppf) (catalytic amount) were added, followed by stirring at 100°C for 2 hours. After insoluble matters were separated by filtration, the solvent was evaporated under reduced pressure. After that, the residue was diluted with ethyl acetate, then washed with 1 N hydrochloric acid and saturated aqueous sodium chloride, and dried over anhydrous magnesium sulfate. The solvent was evaporated under reduced pressure, and the obtained residue was washed with acetonitrile. To the obtained residue, thionyl chloride (15 mL) was added, followed by stirring at 50 °C for 2 hours. After cooling, the solvent was evaporated under reduced pressure to obtain the title compound.
Yield: 4.0 g (9.7 mmol), Percentage yield: 55%

Table 1-1 shows the structural formulae of the intermediates obtained in Examples described above.

**Table 1-1**

| Intermediate | moistructure | Name of Compound (IUPAC) |
|---|---|---|
| 1-A | 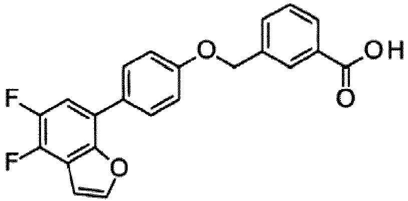 | 3-[[4-(4,5-difluorobenzofuran-7-yl)phenoxy]methyl]benzoic acid |
| 1-B | 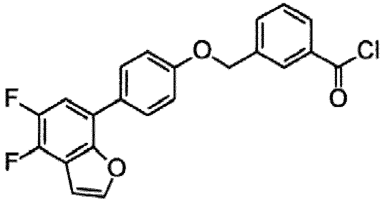 | 3-[[4-(4,5-difluorobenzofuran-7-yl)phenoxy]methyl]benzoyl chloride |
| 2-A | 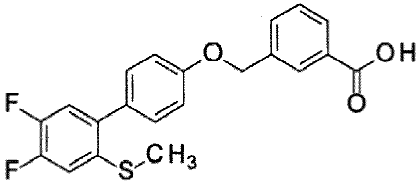 | 3-[[4-(4,5-difluoro-2-methylsulfanyl-phenyl)phenoxy]methyl]benzoic acid |
| 2-B | 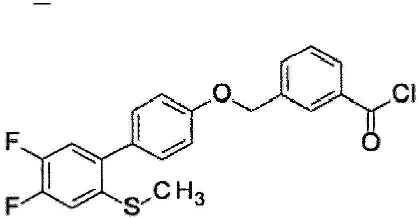 | 3-[[4-(4,5-difluoro-2-methylsulfanyl-phenyl)phenoxy]methyl]benzoyl chloride |
| 3 | 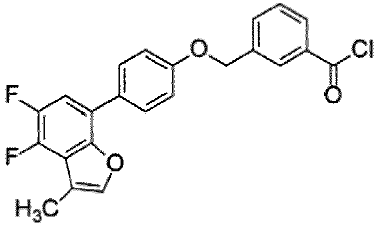 | 3-[[4-(4,5-difluoro-3-methyl-benzofuran-7-yl)phenoxy]methyl]benzoyl chloride |

### Example 1

### 2-[(2-Amino-2-oxo-ethyl)-[3-[[4-(4,5-difluorobenzofuran-7-y l)phenoxy]methyl]benzoyl]amino]acetic Acid

### Step 1

### Synthesis of Benzyl 2-[(2-Amino-2-oxo-ethyl)-[3-[[4-(4,5-difluorobenzofuran-7-y l)phenoxy]methyl]benzoyl]amino]acetate

To 2-aminoacetamide hydrochloride (55.3 mg, 0.500 mmol), methanol (2.5 mL) and a 25% by weight sodium methoxide/methanol solution (0.114 mL) were added, followed by stirring at room temperature for 10 minutes. Then, the solvent was evaporated under reduced pressure. The obtained residue was diluted with acetonitrile (4 mL), and potassium carbonate (69.1 mg, 0.500 mmol) was added thereto, followed by cooling to -10°C to -15°C. Then, benzyl 2-bromoacetate (0.0784 mL, 0.500 mmol) diluted with acetonitrile (1 mL) was added dropwise, followed by stirring for 2.5 hours. After insoluble matters were separated by filtration, the filtrate was concentrated under reduced pressure. The obtained residue was diluted with dichloromethane (4 mL). Under ice cooling, diisopropylethylamine (hereinafter, DIPEA) (0.0871 mL, 0.500 mmol) and Intermediate 1-B (80.0 mg, 0.200 mmol) were added, followed by stirring at room temperature for 2 hours. After the reaction liquid was concentrated under reduced pressure, the residue was diluted with ethyl acetate, and washed with 0.5 N hydrochloric acid, a saturated aqueous sodium hydrogen carbonate solution, and saturated aqueous sodium chloride. The organic layer was dried over anhydrous magnesium sulfate, and the solvent was evaporated under reduced pressure to obtain the title compound without purification.
Yield: 173 mg
MS (ESI) m/z 585 [M+H]⁺

### Step 2

### Synthesis of Compound of Example 1

The compound obtained in Step 1 was dissolved in a solvent mixture of tetrahydrofuran (hereinafter, THF) (3 mL) and methanol (1.5 mL). Under ice cooling, a 1 N aqueous lithium hydroxide solution (0.9 mL) was added, followed by stirring at room temperature for 2 hours. After the reaction liquid was neutralized, the organic solvent was evaporated under reduced pressure. Then, the obtained residue was subjected to reversed phase HPLC using ODS as a packing material, and eluted with a mixture solution of acetonitrile and water containing 0.1% (v/v) of trifluoroacetic acid. The target fraction was freeze dried to obtain the title compound.
Yield: 41.2 mg (0.0832 mmol), Percentage yield: 42%
MS (ESI) m/z 495 [M+H]⁺
¹H NMR (400 MHz, DMSO-d₆) δ 13.19 - 12.77 (m, 1H), 8.19 (d, J = 2.3 Hz, 1H), 7.86 - 7.80 (m, 2H), 7.66 - 7.46 (m, 5H), 7.37 - 7.26 (m, 2H), 7.24 (d, J = 2.3 Hz, 1H), 7.22 - 7.15 (m, 2H), 5.22 (s, 2H), 4.13 - 3.87 (m, 4H).

### Example 2

### (2S,3S)-1-[3-[[4-(4,5-Difluorobenzofuran-7-yl)phenoxy]methy l]benzoyl]-3-hydroxy-pyrrolidine-2-carboxylic Acid

### Step 1

### Synthesis of Methyl (2S,3S)-1-[3-[[4-(4,5-Difluorobenzofuran-7-yl)phenoxy]methy l]benzoyl]-3-hydroxy-pyrrolidine-2-carboxylate

To (2S,3S)-3-hydroxypyrrolidine-2-carboxylic acid (19. 6 mg, 0.15 mmol), hydrochloric acid/methanol prepared from acetyl chloride (0.103 mL) and methanol (3.0 mL) was added under ice cooling, followed by stirring for 4 hours. Then, the solvent was evaporated under reduced pressure. To the obtained residue, dichloromethane (1.5 mL), 1-ethyl-3-(3-dimethylaminopropyl)-carbodiimide (hereinafter, WSC) hydrochloride (56.4 mg, 0.300 mmol), Intermediate 1-A (57.0 mg, 0.150 mmol), 1-hydroxybenzotriazole (hereinafter, HOBt) monohydrate (41.1 mg, 0.300 mmol), and triethylamine (0.0626 mL, 0.450 mmol) were added, followed by stirring at room temperature overnight. The mixture was concentrated under reduced pressure, and the obtained residue was purified by reversed phase HPLC in the same manner as in Step 2 of Example 1 to obtain the title compound.
Yield: 38.5 mg (0.0759 mmol)
MS (ESI) m/z 508 [M+H]⁺

### Step 2

### Synthesis of Compound of Example 2

The compound obtained in Step 1 was dissolved in THF (1.7 mL), and a 1 N aqueous sodium hydroxide solution (1.7 mL) was added under ice cooling, followed by stirring at room temperature for 2 hours. After the reaction liquid was neutralized, the organic solvent was evaporated under reduced pressure, and then the obtained residue was purified by reversed phase HPLC in the same manner as in Step 2 of Example 1 to obtain the title compound.
Yield: 30.0 mg (0.0607 mmol), Percentage yield: 40%
MS (ESI) m/z 494 [M+H]⁺

### Example 3

### (2S,3R)-1-[3-[[4-(4,5-Difluorobenzofuran-7-yl)phenoxy]methy l]benzoyl]-3-hydroxy-pyrrolidine-2-carboxylic Acid

The title compound was obtained by conducting the same operation as in Example 2 by using (2S,3R)-3-hydroxypyrrolidine-2-carboxylic acid instead of (2S,3S)-3-hydroxypyrrolidine-2-carboxylic acid.
Yield: 3.00 mg (0.00601 mmol), Percentage yield: 4.0%
MS (ESI) m/z 494 [M+H]⁺
¹H NMR (400 MHz, DMSO-d₆) δ 12.78 - 11.96 (m, 1H), 8.19 (d, J = 2.2 Hz, 1H), 7.87 - 7.79 (m, 2H), 7.67 - 7.63 (m, 1H), 7.63 - 7.57 (m, 2H), 7.54 - 7.47 (m, 2H), 7.23 (d, J = 2.2 Hz, 1H), 7.22 - 7.15 (m, 2H), 5.29 - 5.18 (m, 2H), 4.56 - 4.38 (m, 2H), 3.72 - 3.58 (m, 1H), 3.48 - 3.43 (m, 1H), 1.99 - 1.73 (m, 2H).

### Example 4

### Synthesis of (2S,5R) or (2S,5S)-5-Carbamoyl-1-[3-[[4-(4,5-difluorobenzofuran-7-yl)p henoxy]methyl]benzoyl]pyrrolidine-2-carboxylic Acid

Ethyl (2S,5R) or (2S,5S)-5-cyanopyrrolidine-2-carboxylate was obtained from ethyl (2S)-5-oxopyrrolidine-2-carboxylate (1.00 g, 6.62 mmol) according to the method described in Tetrahedron Lett. 2002, 43, 1597-1598. The obtained compound was dissolved in a 4 N hydrochloric acid/1,4-dioxane solution, followed by stirring at room temperature overnight. The organic solvent was evaporated under reduced pressure, and then the obtained residue was purified by reversed phase HPLC in the same manner as in Step 2 of Example 1. A portion (44.3 mg, 0.251 mmol) of the obtained compound was dissolved in dichloromethane (2.0 mL). To this solution, DIPEA (0.065 mL, 0.375 mmol) and Intermediate 1-B (50.0 mg, 0.124 mmol) were added in this order, followed by stirring at room temperature for 1.5 hours. The organic solvent was evaporated under reduced pressure, and then the obtained residue was purified by reversed phase HPLC in the same manner as in Step 2 of Example 1. The obtained compound was dissolved in a solvent mixture of THF (1 mL) and methanol (1 mL). At room temperature, a 2 N aqueous sodium hydroxide solution (0.04 mL) was added, followed by stirring at room temperature for 2.5 hours. After the reaction liquid was neutralized, the organic solvent was evaporated under reduced pressure, and then the obtained residue was purified by reversed phase HPLC in the same manner as in Step 2 of Example 1 to obtain the title compound.
Yield: 3.5 mg (0.00672 mmol), Percentage yield: 5.4%
MS (ESI) m/z 521 [M+H]⁺
¹H NMR (400 MHz, DMSO-d₆) δ 12.67 (bs, 1H), 8.19 (d, J = 2.2 Hz, 1H), 7.89 - 7.76 (m, 2H), 7.69 - 7.57 (m, 2H), 7.57 - 7.49 (m, 1H), 7.45 (m, 2H), 7.34 - 7.27 (m, 1H), 7.24 (d, J = 2.2 Hz, 1H), 7.22 - 7.15 (m, 2H), 7.11 - 6.87 (m, 1H), 5.21 (m, 2H), 4.70 - 4.21 (m, 2H), 2.47 - 1.77 (m, 4H).

### Example 5

### (2S,3S)-1-[3-[[4-(4,5-Difluoro-2-methylsulfanyl-phenyl)phen oxy]methyl]benzoyl]-3-hydroxy-pyrrolidine-2-carboxylic Acid

The title compound was obtained by conducting the same operation as in Example 2 by using Intermediate 2-A instead of Intermediate 1-A.
Yield: 8.75 mg (0.0175 mmol), Percentage yield: 12%
MS (ESI) m/z 500 [M+H]⁺
¹H NMR (400 MHz, DMSO-d₆) δ 7.63 - 7.43 (m, 4H), 7.40 - 7.25 (m, 4H), 7.12 - 7.05 (m, 2H), 5.25 - 5.15 (m, 2H), 4.34 - 4.09 (m, 2H), 3.66 - 3.55 (m, 1H), 3.51 - 3.40 (m, 1H), 2.39 (d, J = 1.6 Hz, 3H), 2.04 - 1.73 (m, 3H).

### Example 6

### 2-[[3-[[4-(4,5-Difluoro-2-methylsulfanyl-phenyl)phenoxy]met hyl]benzoyl]-(2-methoxyethyl)amino]acetic Acid

### Step 1

### Synthesis of Ethyl 2-[[3-[[4-(4,5-Difluoro-2-methylsulfanyl-phenyl)phenoxy]met hyl]benzoyl]-(2-methoxyethyl)amino]acetate

To a solution of 2-methoxyethanamine (0.043 mL, 0.50 mmol) in acetonitrile (4 mL), potassium carbonate (69 mg, 0.50 mmol) was added, followed by cooling to -10°C to -15°C. Then, ethyl 2-bromoacetate (0.055 mL, 0.50 mmol) diluted with acetonitrile (1 mL) was added dropwise, followed by stirring for 2 hours. After insoluble matters were separated by filtration, the filtrate was concentrated under reduced pressure, and the obtained residue was diluted with dichloromethane (4 mL). DIPEA (0.087 mL, 0.500 mmol) and Intermediate 2-B (81 mg, 0.20 mmol) were added thereto, followed by stirring at room temperature for 1 hour. To the reaction liquid, water was added, followed by extraction with dichloromethane. The organic layer was washed with a saturated aqueous sodium hydrogen carbonate solution and saturated aqueous sodium chloride, and dried over anhydrous magnesium sulfate. The solvent was evaporated under reduced pressure to obtain the title compound without purification.
MS (ESI) m/z 530 [M+H]⁺

### Step 2

### Synthesis of Compound of Example 6

The compound obtained in Step 1 was dissolved in 1,4-dioxane (3 mL), and a 1 N aqueous lithium hydroxide solution (0.6 mL) was added, followed by stirring at room temperature for 2 hours. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 63.1 mg (0.0126 mmol), Percentage yield: 63%
MS (ESI) m/z 502 [M+H]⁺
¹H NMR (400 MHz, DMSO-d₆) δ 7.60 - 7.23 (m, 8H), 7.08 (d, J = 8.8 Hz, 2H), 5.29 - 5.13 (m, 2H), 4.20 - 3.97 (m, 2H), 3.66 - 3.31 (m, 4H), 3.30 - 3.10 (m, 3H), 2.39 (s, 3H).

### Example 7

### 2-[2-Cyanoethyl-[3-[[4-(4,5-difluoro-2-methylsulfanyl-pheny l)phenoxy]methyl]benzoyl]amino]acetic Acid

To Intermediate 2-B (50 mg, 0.13 mmol), N-(2-cyanoethyl) glycine (65 mg, 0.51 mmol), dichloromethane (2 mL), and a 1 N aqueous sodium hydroxide solution (2 mL) were added thereto, followed by stirring at room temperature overnight. After the reaction liquid was neutralized, the organic solvent was evaporated under reduced pressure, and then the obtained residue was purified by reversed phase HPLC in the same manner as in Step 2 of Example 1 to obtain the title compound.
Yield: 28 mg (0.056 mmol), Percentage yield: 43%
MS (ESI) m/z 497 [M+H]⁺

### Example 8

### 2-[[3-[[4-(4,5-Difluorobenzofuran-7-yl)phenoxy]methyl]benzo yl]-(2-methylsulfonylethyl)amino]acetic Acid

The title compound was obtained by conducting the same operation as in Example 1 by using 2-methylsulfonylethanamine hydrochloride instead of 2-aminoacetamide hydrochloride, THF instead of the methanol solvent, and ethyl 2-bromoacetate instead of benzyl 2-bromoacetate.
Yield: 70.8 mg (0.130 mmol), Percentage yield: 65%
MS (ESI) m/z 544 [M+H]⁺
¹H NMR (400 MHz, DMSO-d₆) δ 8.19 (d, J = 2.3 Hz, 1H), 7.90 - 7.79 (m, 2H), 7.69 - 7.12 (m, 8H), 5.32 - 5.17 (m, 2H), 4.20 - 4.02 (m, 2H), 3.94 - 3.46 (m, 4H), 3.12 - 2.81 (m, 3H).

### Example 9

### 2-[[3-[[4-(4,5-Difluorobenzofuran-7-yl)phenoxy]methyl]benzo yl]-(tetrahydropyran-4-ylmethyl)amino]acetic Acid

To 4-(aminomethyl)tetrahydropyran (100 mg, 0.87 mmol), acetonitrile (4 mL) and potassium carbonate (120 mg, 0.87 mmol) were added, followed by cooling to -10°C to -15°C. Then, benzyl 2-bromoacetate (0.136 mL, 0.87 mmol) diluted with acetonitrile (1 mL) was added dropwise, followed by stirring for 2.5 hours. After insoluble matters were separated by filtration, the filtrate was concentrated under reduced pressure. To the obtained residue, THF (4 mL), Intermediate 1-B (50 mg, 0.13 mmol), and triethylamine (0.240 mL, 1.74 mmol) were added, followed by stirring at room temperature for 2 hours. To the reaction liquid, methanol (4 mL) and a 1 N aqueous sodium hydroxide solution (2 mL) were added, followed by stirring at room temperature for 2 hours. The reaction liquid was neutralized, and the organic solvent was evaporated under reduced pressure. Then, the obtained residue was purified by reversed phase HPLC in the same manner as in Step 2 of Example 1 to obtain the title compound.
Yield: 32 mg (0.060 mmol), Percentage yield: 46%
MS (ESI) m/z 536 [M+H]⁺

### Example 10

### 2-[(2-Amino-2-oxo-ethyl)-[3-[[4-(4,5-difluoro-2-methylsulfa nyl-phenyl)phenoxy]methyl]benzoyl]amino]acetic Acid

The title compound was obtained by conducting the same operation as in Example 1 by using Intermediate 2-B instead of Intermediate 1-B.
Yield: 44.6 mg (0.0891 mmol), Percentage yield: 36%
MS (ESI) m/z 501 [M+H]⁺
¹H NMR (400 MHz, DMSO-d₆) δ 12.95 (br s, 1H), 7.61 - 7.45 (m, 4H), 7.40 - 7.15 (m, 6H), 7.12 - 7.05 (m, 2H), 5.18 (s, 2H), 4.12 - 3.86 (m, 4H), 2.39 (s, 3H).

### Example 11

### 2-[[3-[[4-(4,5-Difluorobenzofuran-7-yl)phenoxy]methyl]benzo yl]-[2-(dimethylamino)-2-oxo-ethyl]amino]acetic Acid

### Step 1

### Synthesis of Benzyl 2-[[3-[[4-(4,5-Difluorobenzofuran-7-yl)phenoxy]methyl]benzo yl]-[2-(dimethylamino)-2-oxo-ethyl]amino]acetate

2-Amino-N,N-dimethylacetamide (51.1 mg, 0.500 mmol) was dissolved in acetonitrile (4 mL), and potassium carbonate (104 mg, 0.750 mmol) was added thereto, followed by cooling to -10°C to -15°C. Then, benzyl 2-bromoacetate (0.086 mL, 0.550 mmol) diluted with acetonitrile (1 mL) was added dropwise, and the temperature was gradually raised to room temperature, followed by stirring overnight. After insoluble matters were separated by filtration, the filtrate was concentrated under reduced pressure. The obtained residue was diluted with dichloromethane (2.5 mL), and DIPEA (0.044 mL, 0.250 mmol) and Intermediate 1-B (100 mg, 0.251 mmol) were added under ice cooling, followed by stirring at room temperature for 2 hours. To the reaction liquid, 0.5 N hydrochloric acid was added, followed by extraction with dichloromethane. Then, the organic layer was washed with a saturated aqueous sodium hydrogen carbonate solution and saturated aqueous sodium chloride, and dried over anhydrous magnesium sulfate. The solvent was evaporated under reduced pressure to obtain the title compound without purification.
Yield: 207 mg
MS (ESI) m/z 613 [M+H]⁺

### Step 2

### Synthesis of Example 11

The title compound was obtained by conducing the same operation as in Step 2 of Example 1 by using the compound obtained in Step 1 instead of benzyl 2-[(2-amino-2-oxo-ethyl)-[3-[[4-(4,5-difluorobenzofuran-7-y l)phenoxy]methyl]benzoyl]amino]acetate.
Yield: 99.5 mg (0.190 mmol), Percentage yield: 76%
MS (ESI) m/z 523 [M+H]⁺
¹H NMR (400 MHz, DMSO-d₆) δ 13.18 - 12.63 (m, 1H), 8.19 (d, J = 2.3 Hz, 1H), 7.86 - 7.80 (m, 2H), 7.66 - 7.55 (m, 2H), 7.53 - 7 . 43 (m, 2H), 7.31 - 7.25 (m, 1H), 7.24 (d, J = 2.3 Hz, 1H), 7.21 - 7.15 (m, 2H), 5.26 - 5.18 (m, 2H), 4.37 - 3.91 (m, 4H), 3.04 - 2.65 (m, 6H).

### Example 12

### 2-[[3-[[4-(4,5-Difluorobenzofuran-7-yl)phenoxy]methyl]benzo yl]-(4-piperidylmethyl)amino]acetic Acid Trifluoroacetate

To tert-butyl 4-(aminomethyl)piperidine-1-carboxylate (550 mg, 2.57 mmol), acetonitrile (4 mL) and potassium carbonate (354 mg, 2.57 mmol) were added, followed by cooling to -10°C to -15°C. Then, benzyl 2-bromoacetate (0.4 mL, 2.57 mmol) diluted with acetonitrile (1 mL) was added dropwise, followed by stirring for 2.5 hours. After insoluble matters were separated by filtration, the filtrate was concentrated under reduced pressure. To the obtained residue, THF (8 mL), Intermediate 1-B (250 mg, 0.65 mmol), and triethylamine (1.20 mL, 8.7 mmol) were added, followed by stirring at room temperature for 2 hours. To the reaction liquid, methanol (8 mL) and a 1 N aqueous sodium hydroxide solution (4 mL) were added, followed by stirring at room temperature for 2 hours. The reaction liquid was neutralized, and the solvent was evaporated under reduced pressure. Then, a 4 N hydrochloric acid/1,4-dioxane solution (10 mL) was added under ice cooling, followed by stirring for 1 hour. After the solvent was evaporated under reduced pressure, the obtained residue was purified by reversed phase HPLC in the same manner as in Step 2 of Example 1 to obtain the title compound.
Yield: 232 mg (0.36 mmol), Percentage yield: 55%
MS (ESI) m/z 535 [M+H]⁺

### Example 13

### 2-[[3-[[4-(4,5-Difluorobenzofuran-7-yl)phenoxy]methyl]benzo yl]-(2-dimethylaminoethyl)amino]acetic Acid Trifluoroacetate

### Step 1

### Synthesis of Benzyl 2-[[3-[[4-(4,5-Difluorobenzofuran-7-yl)phenoxy]methyl]benzo yl]-(2-dimethylaminoethyl)amino]acetate

N,N-Dimethyl-1,2-ethanediamine (44.1 mg, 0.5 mmol) was dissolved in acetonitrile (1.0 mL), followed by cooling to 0°C. To this solution, potassium carbonate (104 mg, 0.55 mmol) was added, and then benzyl 2-bromoacetate (126 mg, 0.55 mmol) was added. While the temperature was being allowed to rise, the mixture was stirred at room temperature for 15 hours. After insoluble matters were separated by filtration, the filtrate was concentrated under reduced pressure. The obtained residue was diluted with dichloromethane (2 mL), and DIPEA (0.087 mL, 0.50 mmol) and Intermediate 1-B (99.7 mg, 0.250 mmol) were added at room temperature, followed by stirring at room temperature for 2 hours. The reaction liquid was diluted with ethyl acetate, and washed with a saturated aqueous ammonium chloride solution, a saturated aqueous sodium hydrogen carbonate solution, and saturated aqueous sodium chloride. The organic layer was dried over anhydrous sodium sulfate, and the solvent was evaporated under reduced pressure to obtain the title compound without purification.
Yield: 168 mg
MS (ESI) m/z 599 [M+H]⁺

### Step 2

### Synthesis of Compound of Example 13

The compound obtained in Step 1 was dissolved in a solvent mixture of THF (1 mL) and methanol (1 mL), and a 2 N aqueous sodium hydroxide solution (1 mL) was added thereto at room temperature, followed by stirring at room temperature for 1 hour. After the reaction liquid was neutralized, the organic solvent was evaporated under reduced pressure, and then the obtained residue was purified by reversed phase HPLC in the same manner as in Step 2 of Example 1 to obtain the title compound. Yield: 72.2 mg (0.116 mmol), Percentage yield: 46%
MS (ESI) m/z 509 [M+H]⁺
¹H NMR (400 MHz, DMSO-d₆) δ 13.1 (bs, 1H), 9.38 (s, 1H), 8.19 (d, J = 2.3 Hz, 1H), 7.88 - 7.79 (m, 2H), 7.68 - 7.45 (m, 4H), 7.45-7.29 (m, 1H), 7.25 (d, J = 2.3 Hz, 1H), 7.22 - 7.14 (m, 2H), 5.29-5.19 (m, 1H), 4.19-3.99 (m, 1H), 3.86-3.78 (m, 2H), 3.61-3.25 (m, 2H), 2.91-2.56 (m, 6H).

### Example 14

### 2-[[3-[[4-(4,5-Difluoro-3-methyl-benzofuran-7-yl)phenoxy]me thyl]benzoyl]-(2-methylsulfonylethyl)amino]acetic Acid

The title compound was obtained by conducting the same operation as in Example 1 by using 2-methylsulfonylethanamine hydrochloride instead of 2-aminoacetamide hydrochloride, ethyl 2-bromoacetate instead of benzyl 2-bromoacetate, and Intermediate 3 instead of Intermediate 1-B.
Yield: 47.8 mg (0.0857 mmol), Percentage yield: 43%
MS (ESI) m/z 558 [M+H]⁺
¹H NMR (400 MHz, DMSO-d₆) δ 7. 91 (d, J = 1.6 Hz, 1H), 7.80 (d, J = 8.8 Hz, 2H), 7.63 - 7.39 (m, 5H) , 7.20 - 7. 12 (m, 2H), 5.29 - 5.16 (m, 2H), 4.19 - 4.02 (m, 3H), 3.88 - 3.45 (m, 4H), 3.09 - 2.81 (m, 3H), 2.34 (s, 3H).

### Example 15

### 2-[[3-[[4-(4,5-Difluorobenzofuran-7-yl)phenoxy]methyl]benzo yl]-[2-(methylamino)-2-oxo-ethyl]amino]acetic Acid

The title compound was obtained by conducting the same operation as in Example 1 by using 2-amino-N-methyl-acetamide hydrochloride (62.3 mg, 0.500 mmol) instead of 2-aminoacetamide hydrochloride.
Yield: 38.0 mg (0.0747 mmol), Percentage yield: 30%
MS (ESI) m/z 509 [M+H]⁺
¹H NMR (400 MHz, DMSO-d₆) δ 12.90 (bs, 1H), 8.19 (d, J = 2.3 Hz, 1H), 8.06 - 7.96 (m, 1H), 7.87 - 7.79 (m, 2H), 7.66 - 7.55 (m, 2H), 7.53 - 7.45 (m, 2H), 7.37 - 7.29 (m, 1H), 7.24 (d, J = 2.3 Hz, 1H), 7.21 - 7.14 (m, 2H), 5.22 (s, 2H), 4.14 - 3.85 (m, 4H), 2.67 - 2.57 (m, 3H).

### Example 16

### 2-[[3-[[4-(4,5-Difluorobenzofuran-7-yl)phenoxy]methyl]benzo yl]-[2-(methanesulfonamido)ethyl]amino]acetic Acid

### Step 1

### Synthesis of Benzyl 2-[2-(Methanesulfonamido)ethylamino]acetate

tert-Butyl N-[2-(methanesulfonamido)ethyl]carbamate (250 mg, 1.05 mmol) was dissolved in a 4 N hydrochloric acid/1,4-dioxane solution (1.1 mL) and 1,4-dioxane (5 mL), followed by stirring at room temperature for 17 hours. The mixture was concentrated under reduced pressure, and freeze dried. The obtained residue was dissolved in acetonitrile (3 mL) and DMF (3 mL), and cooled to 0°C. To this solution, potassium carbonate (173 mg, 1.25 mmol) was added, and then benzyl 2-bromoacetate (252 mg, 1.10 mmol) was added. While the temperature was being allowed to rise, the mixture was stirred at room temperature for 16 hours. After insoluble matters were separated by filtration, the filtrate was concentrated under reduced pressure to obtain the title compound without purification.
MS (ESI) m/z 287 [M+H]⁺

### Step 2

### Synthesis of Compound of Example 16

The compound obtained in Step 1 was dissolved in dichloromethane (2 mL), and DIPEA (0.087 mL, 0.50 mmol) and Intermediate 1-B (79.7 mg, 0.200 mmol) were added at room temperature, followed by stirring at room temperature for 17 hours. The reaction liquid was diluted with ethyl acetate, and washed with a saturated aqueous ammonium chloride solution, a saturated aqueous sodium hydrogen carbonate solution, and saturated aqueous sodium chloride. The organic layer was dried over anhydrous sodium sulfate, and the solvent was evaporated under reduced pressure. The obtained residue was dissolved by adding THF (1 mL) andmethanol (1 mL) thereto. To this solution, a 2 N aqueous sodium hydroxide solution (0.3 mL) was added, followed by stirring at room temperature for 1.5 hours. After the reaction liquid was neutralized, the organic solvent was evaporated under reduced pressure, and then the obtained residue was purified by reversed phase HPLC in the same manner as in Step 2 of Example 1 to obtain the title compound.
Yield: 57.6 mg (0.103 mmol), Percentage yield: 52%
MS (ESI) m/z 559 [M+H]⁺
¹H NMR (400 MHz, DMSO-d₆) δ 12.85 (s, 1H), 8.18 (d, J = 2.2 Hz, 1H), 7.83 (d, J = 8.31 Hz, 2H), 7.66 - 7.44 (m, 4H), 7.41-7.27 (m, 1H), 7.23 (d, J = 2.2 Hz, 1H), 7.21 - 7.03 (m, 3H), 5.28-5.19 (m, 2H), 4.19-4.01 (m, 1H), 3.60-3.51 (m, 1H), 3.41-3.32 (m, 1H), 3.30-3.20 (m, 1H), 3.17-3.07 (m, 1H), 2.98-2.81 (m, 3H).

### Example 17

### 2-[[3-[[4-(4,5-Difluoro-2-methylsulfanyl-phenyl)phenoxy]met hyl]benzoyl]-[2-(methanesulfonamido)ethyl]amino]acetic Acid

A half of the compound obtained in Step 1 of Example 16 was dissolved in dichloromethane (2 mL), and DIPEA (0.087 mL, 0.50 mmol) and Intermediate 2-B (81.0 mg, 0.200 mmol) were added thereto, followed by stirring at room temperature for 17 hour. The reaction liquid was diluted with ethyl acetate, and washed with a saturated aqueous ammonium chloride solution, a saturated aqueous sodium hydrogen carbonate solution, and saturated aqueous sodium chloride. The organic layer was dried over anhydrous sodium sulfate, and the solvent was evaporated under reduced pressure. The obtained residue was dissolved by adding THF (1 mL) and methanol (1 mL). To this solution, a 2 N aqueous sodium hydroxide solution (0.3 ml) was added, followed by stirring at room temperature for 1.5 hours. After the reaction liquid was neutralized, the organic solvent was evaporated under reduced pressure, and then the obtained residue was purified by reversed phase HPLC in the same manner as in Step 2 of Example 1 to obtain the title compound.
Yield: 62.5 mg (0.111 mmol), Percentage yield: 55%
MS (ESI) m/z 565 [M+H]⁺
¹H NMR (400 MHz, DMSO-d₆) δ 12. 94 (bs, 1H), 7.61 - 7.43 (m, 3H), 7.41-7.25 (m, 3H), 7.21-7.02 (m, 3H), 5.24-5.15 (m, 1H), 4.18-3.99 (m, 1H), 3.59-3.50 (m, 1H), 3.51-3.30 (m, 1H), 3.29-3.19 (m, 1H), 3.16-3.06 (m, 1H), 2.97-2.80 (m, 3H), 2.40 (s, 3H).

### Example 18

### 2-[[3-[[4-(4,5-Difluorobenzofuran-7-yl)phenoxy]methyl]benzo yl]-(3-hydroxy-3-methyl-butyl)amino]acetic Acid

To 4-amino-2-methylbutan-2-ol (100 mg, 0.87 mmol), acetonitrile (4 mL) and potassium carbonate (120 mg, 0.87 mmol) were added, followed by cooling to -10°C to -15°C. Then, benzyl 2-bromoacetate (0.136 mL, 0.87 mmol) diluted with acetonitrile (1 mL) was added dropwise, followed by stirring for 2.5 hours. After insoluble matters were separated by filtration, the filtrate was concentrated under reduced pressure. To the obtained residue, THF (4 mL), Intermediate 1-B (50 mg, 0.13 mmol), and triethylamine (0.240 mL, 1.74 mmol) were added, followed by stirring at room temperature for 2 hours. To the reaction liquid, methanol (4 mL) and a 1 N aqueous sodium hydroxide solution (2 mL) were added, followed by stirring at room temperature for 2 hours. The reaction liquid was neutralized, and the organic solvent was evaporated under reduced pressure, and then the obtained residue was purified by reversed phase HPLC in the same manner as in Step 2 of Example 1 to obtain the title compound.
Yield: 32 mg (0.06 mmol), Percentage yield: 46%
MS (ESI) m/z 524 [M+H]⁺

### Example 19

### 2-[[3-[[4-(4,5-Difluoro-2-methylsulfanyl-phenyl)phenoxy]met hyl]benzoyl]-(4-pyridylmethyl)amino]acetic Acid Trifluoroacetate

To 4-pyridinecarboxaldehyde (0.15 mL, 1.6 mmol), dichloromethane (5 mL), glycine benzyl ester p-toluenesulfonate (540 mg, 1.6 mmol), and acetic acid (0.18 mL, 3.2 mmol) were added, followed by stirring at room temperature for 30 minutes. Then, sodium triacetoxyborohydride (680 mg, 3.2 mmol) was added, followed by stirring overnight. After purification by reversed phase HPLC in the same manner as in Intermediate 4, THF (4 mL), Intermediate 2-B (150 mg, 0.39 mmol), and triethylamine (0.720 mL, 5.22 mmol) were added, followed by stirring at room temperature for 2 hours. To the reaction liquid, methanol (6 mL) and a 1 N aqueous sodium hydroxide solution (3 mL) were added, followed by stirring at room temperature for 2 hours. The reaction liquid was neutralized, and the organic solvent was evaporated under reduced pressure. Then, the obtained residue was purified by reversed phase HPLC in the same manner as in Step 2 of Example 1 to obtain the title compound.
Yield: 55 mg (0.085 mmol), Percentage yield: 22%
MS (ESI) m/z 535 [M+H]⁺

### Example 20

### 2-[[3-[[4-(4,5-Difluoro-2-methylsulfanyl-phenyl)phenoxy]met hyl]benzoyl]-(2-pyridylmethyl)amino]acetic Acid Trifluoroacetate

The title compound was obtained by conducting the same operation as in Example 6 by using 2-pyridylmethanamine instead of 2-methoxyethanamine.
Yield: 58.6 mg (0.0903 mmol), Percentage yield: 45%
MS (ESI) m/z 535 [M+H]⁺
¹H NMR (400 MHz, DMSO-d₆) δ 8.68 - 8.54 (m, 1H), 8.08 - 7.17 (m, 11H), 7.17 - 6.92 (m, 2H), 5.29 - 5.10 (m, 2H), 4.89 - 4.55 (m, 2H), 4.20 - 4.04 (m, 2H), 2.39 (s, 3H).

### Example 21

### 2-[[3-[[4-(4,5-Difluoro-2-methylsulfanyl-phenyl)phenoxy]met hyl]benzoyl]-(3-hydroxybutyl)amino]acetic Acid

The title compound was obtained by conducting the same operation as in Example 11 by using 4-aminobutan-2-ol (44.6 mg, 0.500 mmol) instead of 2-amino-N,N-dimethylacetamide and Intermediate 2-B (103 mg, 0.254 mmol) instead of Intermediate 1-B.
Yield: 83.0 mg (0.161 mmol), Percentage yield: 64%
MS (ESI) m/z 516 [M+H]⁺
¹H NMR (400 MHz, DMSO-d₆) δ 12.79 (br s, 1H), 7.59 - 7.23 (m, 8H), 7.12 - 7.04 (m, 2H), 5.28 - 5.13 (m, 2H), 4.17 - 3.90 (m, 2H), 3.77 - 3.12 (m, 4H), 2.39 (s, 3H), 1.79 - 1.47 (m, 2H), 1.15 - 0.88 (m, 3H).

### Example 22

### 2-[[(1S)-2-Amino-1-methyl-2-oxo-ethyl]-[3-[[4-(4,5-difluoro -2-methylsulfanyl-phenyl)phenoxy]methyl]benzoyl]amino]aceti c Acid

### Step 1

### Synthesis of Benzyl 2-[[(1S)-2-Amino-1-methyl-2-oxo-ethyl]-[3-[[4-(4,5-difluoro -2-methylsulfanyl-phenyl)phenoxy]methyl]benzoyl]amino]aceta te

L-Alanineamide hydrochloride (62.3 mg, 0.500 mmol) was dissolved in acetonitrile (3 mL), and potassium carbonate (173 mg, 1.25 mmol) was added thereto, followed by cooling to -10°C to -15°C. Then, benzyl 2-bromoacetate (0.086 mL, 0.550 mmol) diluted with acetonitrile (1 mL) was added dropwise. The temperature was allowed to rise to room temperature, followed by stirring overnight. After insoluble matters were separated by filtration, the filtrate was concentrated under reduced pressure. The obtained residue was diluted with dichloromethane (3 mL), and DIPEA (0.088 mL, 0.50 mmol) and Intermediate 2-B (103 mg, 0.254 mmol) were added under ice cooling, followed by stirring at room temperature for 2 hours. To the reaction liquid, 0.5 N hydrochloric acid was added, followed by extraction with dichloromethane. Then, the organic layer was washed with a saturated aqueous sodium hydrogen carbonate solution and saturated aqueous sodium chloride, and dried over anhydrous magnesium sulfate. The solvent was evaporated under reduced pressure to obtain the title compound without purification.
Yield: 178 mg
MS (ESI) m/z 605 [M+H]⁺

### Step 2

### Synthesis of Compound of Example 22

The title compound was obtained by conducting the same operation as in Step 2 of Example 1 by using the compound obtained in Step 1 instead of benzyl 2-[(2-amino-2-oxo-ethyl)-[3-[[4-(4,5-difluorobenzofuran-7-y 1)phenoxy]methyl]benzoyl]amino]acetate.
Yield: 41.6 mg (0.0808 mmol), Percentage yield: 32%
MS (ESI) m/z 515 [M+H]⁺
¹H NMR (400 MHz, DMSO-d₆) δ 12.95 (br s, 1H), 7.65 - 7.44 (m, 4H), 7.42 - 7.25 (m, 6H), 7.14 - 7.05 (m, 2H), 5.24 - 5.13 (m, 2H), 4.52 - 3.83 (m, 3H), 2.39 (s, 3H), 1.48 - 1.28 (m, 3H).

### Example 23

### 2-[[3-[[4-(4,5-Difluoro-2-methylsulfanyl-phenyl)phenoxy]met hyl]benzoyl]-(2-methoxy-2-methyl-propyl)amino]acetic Acid

The title compound was obtained by conducting the same operation as in Example 6 by using 2-methoxy-2-methyl-propane-1-amine instead of 2-methoxyethanamine.
Yield: 47.6 mg (0.0899 mmol), Percentage yield: 45%
MS (ESI) m/z 530 [M+H]⁺
¹H NMR (400 MHz, DMSO-d₆) δ 7.56 - 7.22 (m, 8H), 7.12 - 7.03 (m, 2H), 5.31 - 5.11 (m, 2H), 4.29 - 4.04 (m, 2H), 3.57 - 3.25 (m, 2H), 3.15 - 2.93 (m, 3H), 2.39 (s, 3H), 1.23 - 0.82 (m, 6H).

### Example 24

### 2-[2-Diethylaminoethyl-[3-[[4-(4,5-difluoro-2-methylsulfany 1-phenyl)phenoxy]methyl]benzoyl]amino]acetic Acid Trifluoroacetate

The title compound was obtained by conducting the same operation as in Example 6 by using N,N-diethyl-1,2-ethanediamine instead of 2-methoxyethanamine.
Yield: 98.7 mg (0.150 mmol), Percentage yield: 75%
MS (ESI) m/z 543 [M+H]⁺
¹H NMR (400 MHz, DMSO-d₆) δ 9.31 (s, 1H), 7.78 - 7.22 (m, 8H), 7.22 - 6.99 (m, 2H), 5.32 - 5.02 (m, 2H), 4.27 - 3.96 (m, 2H), 3.89 - 3.50 (m, 3H), 3.31 - 2.89 (m, 5H), 2.40 (s, 3H), 1.43 - 0.88 (m, 6H).

### Example 25

### 2-[[3-[[4-(4,5-Difluoro-2-methylsulfanyl-phenyl)phenoxy]met hyl]benzoyl]-(2-oxo-2-pyrrolidin-1-yl-ethyl)amino]acetic Acid

### Step 1

### Synthesis of tert-Butyl N-(2-Oxo-2-pyrrolidin-1-yl-ethyl)carbamate

In dichloromethane (20 mL), 2-(tert-butoxycarbonylamino)acetic acid (1.05 g, 6.00 mmol), WSC hydrochloride (1.15 g, 6.00 mmol), and HOBt monohydrate (919 mg, 6.00 mmol) were dissolved, and triethylamine (0.836 mL, 6.00 mmol) and pyrrolidine (0.496 mL, 6.00 mmol) were added thereto, followed by stirring at room temperature overnight. The reaction liquid was diluted with dichloromethane, and washed with a saturated aqueous sodium hydrogen carbonate solution, 1 N hydrochloric acid, and saturated aqueous sodium chloride. Then, the organic layer was dried over anhydrous magnesium sulfate, and concentrated under reduced pressure. To the obtained residue, a hexane/ethyl acetate solvent mixture was added, followed by stirring. The deposited solid was filtered to obtain the title compound.
Yield: 598 mg (2.62 mmol), Percentage yield: 44%
MS (ESI) m/z 229 [M+H]⁺

### Step 2

### Synthesis of 2-Amino-1-pyrrolidin-1-yl-ethanone Hydrochloride

The compound of Step 1 (598 mg, 2.62 mmol) was dissolved in 1,4-dioxane (3 mL), and a 4 N hydrochloric acid/1,4-dioxane solution (10 mL) was added, followed by stirring at room temperature for 1.5 hours. The reaction liquid was concentrated under reduced pressure, and suspended in toluene. This suspension was concentrated under reduced pressure, and ethyl acetate was added to the obtained residue, followed by stirring. The deposited solid was filtered to obtain the title compound.
Yield: 437 mg (2.65 mmol), Percentage yield: Quantitative

### Step 3

### Synthesis of Compound of Example 25

The title compound was obtained by conducting the same operation as in Example 22 by using the compound obtained in Step 2 (82.3 mg, 0.500 mmol) instead of L-alanineamide hydrochloride.
Yield: 98.5 mg (0.178 mmol), Percentage yield: 71%
MS (ESI) m/z 555 [M+H]⁺
¹H NMR (400 MHz, DMSO-d₆) δ 13.16 - 12.73 (m, 1H), 7.59 - 7.54 (m, 1H), 7.52 - 7.44 (m, 2H), 7.39 - 7.25 (m, 5H), 7.11 - 7.05 (m, 2H), 5.23 - 5.15 (m, 2H), 4.29 - 3.94 (m, 4H), 3.51 - 3.00 (m, 4H), 2.39 (s, 3H), 1.97 - 1.61 (m, 4H).

### Example 26

### 2-[[3-[[4-(4,5-Difluoro-2-methylsulfanyl-phenyl)phenoxy]met hyl]benzoyl]-[3-(dimethylamino)propyl]amino]acetic Acid Trifluoroacetate

The title compound was obtained by conducting the same operation as in Example 6 by using N,N-dimethylpropane-1,3-diamine instead of 2-methoxyethanamine.
Yield: 46.8 mg (0.0728 mmol), Percentage yield: 36%
MS (ESI) m/z 529 [M+H]⁺
¹H NMR (400 MHz, DMSO-d₆) δ 9.37 (s, 1H), 7.68 - 7.43 (m, 3H), 7.43 - 7.23 (m, 5H), 7.15 - 7.02 (m, 2H), 5.27 - 5.09 (m, 2H), 4.19 - 3.95 (m, 2H), 3.49 - 3.07 (m, 5H), 2.97 - 2.62 (m, 7H), 2.40 (s, 3H), 2.03 - 1.82 (m, 2H).

### Example 27

### 2-[Carboxymethyl-[3-[[4-(4,5-difluoro-2-methylsulfanyl-phen yl)phenoxy]methyl]benzoyl]amino]acetic Acid

To iminodiacetic acid (200 mg, 1.50 mmol), a 1 N aqueous sodium hydroxide solution (3 mL) and dichloromethane (2 mL) were added, and under ice cooling a dichloromethane solution (1 mL) of Intermediate 2-B (103 mg, 0.254 mmol) was added thereto, followed by stirring at room temperature for 3.5 hours. After the reaction liquid was neutralized, the organic solvent was evaporated under reduced pressure, and the obtained residue was purified by reversed phase HPLC in the same manner as in Step 2 of Example 1 to obtain the title compound.
Yield: 13.3 mg (0.00265 mmol), Percentage yield: 10%
MS (ESI) m/z 502 [M+H]⁺

### Example 28

### 2-[[3-[[4-(4,5-Difluoro-2-methylsulfanyl-phenyl)phenoxy]met hyl]benzoyl]-[2-(methylamino)ethyl]amino]acetic Acid Trifluoroacetate

### Step 1

### Synthesis of Ethyl 2-[2-(tert-Butoxycarbonylamino)ethyl-[3-[[4-(4,5-difluoro-2 -methylsulfanyl-phenyl)phenoxy]methyl]benzoyl]amino]acetate

The title compound was obtained by conducting the same operation as in Step 1 of Example 6 by using tert-butyl N-(2-aminoethyl)carbamate instead of 2-methoxyethanamine.
Yield: 94.6 mg (0.121 mmol), Percentage yield: 49%
MS (ESI) m/z 615 [M+H]⁺

### Step 2

### Synthesis of 2-[2-[tert-Butoxycarbonyl(methyl)amino]ethyl-[3-[[4-(4,5-di fluoro-2-methylsulfanyl-phenyl)phenoxy]methyl]benzoyl]amino ]acetic Acid

The compound obtained in Step 1 (94.6 mg, 0.121 mmol) was dissolved in THF (3 mL), and sodium hydride (16 mg, 0.364 mmol) and methyl iodide (0.038 mL, 0.605 mmol) were added thereto, followed by stirring at room temperature for 2 hours under an argon atmosphere. Further, sodium hydride (16 mg, 0.364 mmol) and methyl iodide (0.038 mL, 0.605 mmol) were added, followed by stirring at room temperature overnight. The reaction liquid was cooled, and then quenched with a saturated aqueous ammonium chloride solution. The reaction liquid was evaporated under reduced pressure, and then the obtained residue was purified by reversed phase HPLC in the same manner as in Step 2 of Example 1 to obtain the title compound.
Yield: 41.3 mg (0.0687 mmol), Percentage yield: 57%
MS (ESI) m/z 601 [M+H]⁺

### Step 3

### Synthesis of Compound of Example 28

The compound obtained in Step 2 (50.0 mg, 0.0833 mmol) was dissolved in dichloromethane, and trifluoroacetic acid (0.032 mL, 0.412 mmol) was added thereto, followed by stirring at room temperature for 6 hours. Trifluoroacetic acid (0.032 mL, 0.412 mmol) was further added, followed by stirring at room temperature overnight. After the reaction liquid was concentrated under reduced pressure, the obtained residue was purified by reversed phase HPLC in the same manner as in Step 2 of Example 1 to obtain the title compound.
Yield: 44.4 mg (0.0722 mmol), Percentage yield: 87%
MS (ESI) m/z 501 [M+H]⁺
¹H NMR (400 MHz, DMSO-d₆) δ 13.10 (s, 1H), 8.46 - 8.21 (m, 2H), 7.67 - 7.44 (m, 3H), 7.45 - 7.23 (m, 5H), 7.14 - 7.02 (m, 2H), 5.26 - 5.09 (m, 2H), 4.21 - 3.98 (m, 2H), 3.78 - 3.51 (m, 2H), 3.25 - 3.05 (m, 2H), 2.66 - 2.46 (m, 3H), 2.40 (s, 3H).

### Example 29

### (2S,4R)-1-[3-[[4-(4,5-Difluoro-2-methylsulfanyl-phenyl)phen oxy]methyl]benzoyl]-4-hydroxy-pyrrolidine-2-carboxylic Acid

Intermediate 2-A (58.0 mg, 0.150 mmol) and methyl (2S,4R)-4-hydroxypyrrolidine-2-carboxylate (22.0 mg, 0.150 mmol) were dissolved in dichloromethane (1.5 mL), and WSC hydrochloride (57.0 mg, 0.300 mmol), 1-hydroxy-7-azabenzotriazole (hereinafter, HOAt) (41.0 mg, 0.300 mmol), and triethylamine (0.063 mL, 0.45 mmol) were added thereto, followed by stirring at room temperature for 1. 5 hours. The reaction liquid was concentrated under reduced pressure to obtain a residue. The obtained residue was dissolved in a solvent mixture of THF (1 mL) and methanol (1 mL). Under ice cooling, a 2 N aqueous sodium hydroxide solution (0.5 mL) was added thereto, followed by stirring at room temperature for 1 hour. After the reaction liquid was neutralized, the organic solvent was evaporated under reduced pressure, and the obtained residue was purified by reversed phase HPLC in the same manner as in Step 2 of Example 1 to obtain the title compound.
Yield: 7.80 mg (0.0156 mmol), Percentage yield: 13%
MS (ESI) m/z 500[M+H]⁺

### Example 30

### 2-[[3-[[4-(4,5-Difluoro-2-methylsulfanyl-phenyl)phenoxy]met hyl]benzoyl]-(3-methoxypropyl)amino]acetic Acid

### Step 1

### Synthesis of Benzyl 2-[[3-[[4-(4,5-Difluoro-2-methylsulfanyl-phenyl)phenoxy]met hyl]benzoyl]-(3-methoxypropyl)amino]acetate

To a solution of 3-methoxypropane-1-amine (51 µL, 0.50 mmol) in acetonitrile (4 mL), potassium carbonate (69 mg, 0.50 mmol) was added, followed by cooling to -10°C to -15°C. Then, benzyl 2-bromoacetate (78 µL, 0.50 mmol) diluted with acetonitrile (1 mL) was added dropwise, followed by stirring for 3 hours. Insoluble matters were separated by filtration, and the filtrate was concentrated under reduced pressure. The obtained residue was diluted with dichloromethane (4 mL), and DIPEA (87 µL, 0.50 mmol) and Intermediate 2-B (81 mg, 0.20 mmol) were added thereto, followed by stirring at room temperature for 1.5 hours. To the reaction liquid, water was added, followed by extraction with dichloromethane. The organic layer was washed with a saturated aqueous sodium hydrogen carbonate solution and saturated aqueous sodium chloride, and dried over anhydrous magnesium sulfate. The solvent was evaporated under reduced pressure to obtain the title compound without purification.
MS (ESI) m/z 606 [M+H]⁺

### Step 2

### Synthesis of Compound of Example 30

The compound obtained in Step 1 was dissolved in 1,4-dioxane (3 mL), and a 1 N aqueous lithium hydroxide solution (0.6 mL) was added thereto, followed by stirring at room temperature for 1 hour. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 49.9 mg (0.0968 mmol), Percentage yield: 48%
MS (ESI) m/z 516 [M+H]⁺
¹H NMR (400 MHz, DMSO-d₆) δ 7.60 - 7.21 (m, 8H), 7.12 - 7.02 (m, 2H), 5.25 - 5.11 (m, 2H), 4.15 - 3.88 (m, 2H), 3.52 - 3.00 (m, 7H), 2.39 (s, 3H), 1.88 - 1.65 (m, 2H).

### Example 31

### 2-[[3-[[4-(4,5-Difluoro-2-methylsulfanyl-phenyl)phenoxy]met hyl]benzoyl]-(2-methylsulfonylethyl)amino]acetic Acid

### Step 1

### Synthesis of Ethyl 2-[[3-[[4-(4,5-Difluoro-2-methylsulfanyl-phenyl)phenoxy]met hyl]benzoyl]-(2-methylsulfonylethyl)amino]acetate

To 2-methylsulfonylethanamine hydrochloride (192 mg, 1.20 mmol), tetrahydrofuran (12 mL) and a 25% by weight sodium methoxide/methanol solution (0.274 mL) were added, followed by stirring at room temperature for 30 minutes. Then, insoluble matters were filtered off, and the solvent was evaporated under reduced pressure. The obtained residue was diluted with acetonitrile (9.6 mL), and potassium carbonate (166 mg, 1.20 mmol) was added thereto, followed by cooling to -10°C to -15°C. Then, ethyl 2-bromoacetate (0.133 mL, 1.20 mmol) diluted with acetonitrile (2.4 mL) was added dropwise. The temperature was gradually raised to room temperature, followed by stirring overnight. After insoluble matters were separated by filtration, the filtrate was concentrated under reduced pressure. A half of the obtained residue was diluted with dichloromethane (4 mL), and DIPEA (87 µL, 0.50 mmol) and Intermediate 2-B (81 mg, 0.20 mmol) were added thereto, followed by stirring at room temperature for 1 hour. To the reaction liquid, water was added, followed by extraction with dichloromethane. The organic layer was washed with a saturated aqueous sodium hydrogen carbonate solution and saturated aqueous sodium chloride, and dried over anhydrous magnesium sulfate. The solvent was evaporated under reduced pressure to obtain the title compound without purification.
MS (ESI) m/z 578 [M+H]⁺

### Step 2

### Synthesis of Compound of Example 31

The compound obtained in Step 1 was dissolved in 1,4-dioxane (3 mL), and a 1 N aqueous lithium hydroxide solution (0.6 mL) was added, followed by stirring at room temperature for 1 hour. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 67.8 mg (0.0123 mmol), Percentage yield: 62%
MS (ESI) m/z 550 [M+H]⁺
¹H NMR (400 MHz, DMSO-d₆) δ 7.66 - 7.20 (m, 8H), 7.14 - 7.01 (m, 2H), 5.33 - 5.06 (m, 2H), 4.19 - 4.02 (m, 2H), 3.92 - 3.43 (m, 4H), 3.16 - 2.76 (m, 3H), 2.39 (s, 3H).

### Example 32

### 2-[[3-[[4-(4,5-Difluoro-2-methylsulfanyl-phenyl)phenoxy]met hyl]benzoyl]-(3-methylsulfonylpropyl)amino]acetic Acid

### Step 1

### Synthesis of Ethyl 2-[[3-[[4-(4,5-Difluoro-2-methylsulfanyl-phenyl)phenoxy]met hyl]benzoyl]-(3-methylsulfonylpropyl)amino]acetate

To a solution of 3-methylsulfonylpropane-1-amine (183 mg, 1.00 mmol) in acetonitrile (8 mL), potassium carbonate (138 mg, 1.00 mmol) was added, followed by cooling to -10°C to -15°C. Then, ethyl 2-bromoacetate (111 µL, 1.00 mmol) diluted with acetonitrile (2 mL) was added dropwise, followed by stirring for 2.5 hours. The temperature was returned to room temperature, followed by stirring for 5.5 hours. Then, insoluble matters were separated by filtration, and the filtrate was concentrated under reduced pressure. A half of the obtained residue was diluted with dichloromethane (4 mL), and DIPEA (87 µL, 0.50 mmol) and Intermediate 2-B (80 mg, 0.20 mmol) were added thereto, followed by stirring at room temperature overnight. To the reaction liquid, water was added, followed by extraction with dichloromethane. The organic layer was washed with a saturated aqueous sodium hydrogen carbonate solution and saturated aqueous sodium chloride, and dried over anhydrous magnesium sulfate. The solvent was evaporated under reduced pressure to obtain the title compound without purification.
MS (ESI) m/z 592 [M+H]⁺

### Step 2

### Synthesis of Compound of Example 32

The compound obtained in Step 1 was dissolved in 1,4-dioxane (3 mL), and a 1 N aqueous lithium hydroxide solution (0.6 mL) was added thereto, followed by stirring at room temperature for 1.5 hours. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 74.9 mg (0.0133 mmol), Percentage yield: 66%
MS (ESI) m/z 564 [M+H]⁺
¹H NMR (400 MHz, DMSO-d₆) δ 7.64 - 7.18 (m, 8H), 7.17 - 7.00 (m, 2H), 5.32 - 5.01 (m, 2H), 4.26 - 3.93 (m, 2H), 3.59 - 2.83 (m, 7H), 2.39 (s, 3H), 2.11 - 1.86 (m, 2H).

### Example 33

### (2S,3S)-1-[3-[[4-(4,5-Difluoro-2-methylsulfanyl-phenyl)phen oxy]methyl]benzoyl]-3-methoxy-pyrrolidine-2-carboxylic Acid

A THF solution (1 mL) of methyl (2S,3S)-1-[3-[[4-(4,5-difluoro-2-methylsulfanyl-phenyl)phen oxy]methyl]benzoyl]-3-hydroxy-pyrrolidine-2-carboxylate (50 mg, 0.094 mmol) obtained as an intermediate in Example 5 was cooled to 0°C, and sodium hydride (13 mg, 0.29 mmol) and THF (0.5 mL) were added thereto, followed by stirring at room temperature for 1.5 hours. Methyl iodide (18 µL, 0.29 mmol) was added dropwise, followed by stirring at room temperature for 20 minutes. Methyl iodide (30 µL, 0.49 mmol) was further added, followed by stirring for 30 minutes. Then, water was added to the reaction liquid. The obtained residue was purified by reversed phase HPLC in the same manner as in Step 2 of Example 1 to obtain the title compound.
Yield: 25.2 mg (0.0491 mmol), Percentage yield: 50%
MS (ESI) m/z 514 [M+H]⁺
¹H NMR (400 MHz, DMSO-d₆) δ 7.69 - 7.21 (m, 8H), 7.16 - 7.02 (m, 2H), 5.27 - 5.12 (m, 2H), 4.50 - 4.18 (m, 1H), 4.07 - 3.96 (m, 1H), 3.70 - 3.36 (m, 2H), 3.34 - 3.19 (m, 3H), 2.39 (s, 3H), 2.08 - 1.85 (m, 2H).

### Example 34

### 2-[[3-[[4-(4,5-Difluoro-2-methylsulfanyl-phenyl)phenoxy]met hyl]benzoyl]-(tetrahydropyran-4-ylmethyl)amino]acetic Acid

After 4- (aminomethyl) tetrahydropyran (350 mg, 3. 04 mmol) was dissolved in acetonitrile (25 mL), potassium carbonate (420 mg, 3.04 mmol) was added, and benzyl bromoacetate (0.48 mL, 3.04 mmol) was added slowly at -20°C. At the same temperature, the mixture was stirred for 1 hour. Then, the temperature was returned to room temperature, followed by stirring overnight. After insoluble matters were separated by filtration, the solvent was evaporated under reduced pressure. To a half of the obtained residue, THF (5 mL), Intermediate 2-B (300 mg, 0.750 mmol), and triethylamine (0.21 mL, 1. 5 mmol) were added, followed by stirring at room temperature for 2 hours. Then, a 1 N aqueous sodium hydroxide solution (5 mL) and methanol (5 mL) were added thereto, followed by stirring overnight. The solvent was evaporated under reduced pressure. The obtained residue was diluted with ethyl acetate, then washed with 1 N hydrochloric acid and saturated aqueous sodium chloride, and dried over anhydrous magnesium sulfate. The solvent was evaporated under reduced pressure, and the obtained residue was purified by reversed phase HPLC in the same manner as in Step 2 of Example 1 to obtain the title compound.
Yield: 120 mg (0.220 mmol), Percentage yield: 14%
MS (ESI) m/z 542 [M+H]⁺

### Example 35

### 2-[[3-[[4-(4,5-Difluoro-2-methylsulfanyl-phenyl)phenoxy]met hyl]benzoyl]-(4-piperidylmethyl)amino]acetic Acid Trifluoroacetate

After 1-N-BOC-4-(aminomethyl)piperidine (550 mg, 2.57 mmol) was dissolved in acetonitrile (25 mL), potassium carbonate (354 mg, 2.57 mmol) was added thereto, and benzyl bromoacetate (0.400 mL, 2.57 mmol) was slowly added at -20°C. At the same temperature, the mixture was stirred for 1 hour. Then, the temperature was returned to room temperature, followed by stirring overnight. Insoluble matters were separated by filtration, and then the solvent was evaporated under reduced pressure. To a half of the obtained residue, THF (5 mL), Intermediate 2-B (300 mg, 0.750 mmol), and triethylamine (0.21 mL, 1.5 mmol) were added, followed by stirring at room temperature for 2 hours. Then, a 1 N aqueous sodium hydroxide solution (5 mL) and methanol (5 mL) were added, followed by stirring overnight. After neutralization with 2N hydrochloric acid, the solvent was evaporated under reduced pressure. To the obtained residue, a 4 N hydrochloric acid/1,4-dioxane solution was added at 0°C, and the mixture was stirred at the same temperature for 2 hours. The solvent was evaporated under reduced pressure, and the obtained residue was purified by reversed phase HPLC in the same manner as in Step 2 of Example 1 to obtain the title compound.
Yield: 130 mg (0.190 mmol), Percentage yield: 15%
MS (ESI) m/z 541 [M+H]⁺

### Example 36

### 2-[[3-[[4-(4,5-Difluoro-2-methylsulfanyl-phenyl)phenoxy]met hyl]benzoyl]-(4-piperidyl)amino]acetic Acid Trifluoroacetate

The title compound was obtained by using 4-amino-1-BOC-piperidine (513 mg, 2.56 mmol) instead of 1-N-BOC-4-(aminomethyl)piperidine of Example 35 by conducting substantially the same operation.
Yield: 120 mg (0.190 mmol), Percentage yield: 14%
MS (ESI) m/z 527 [M+H]⁺

### Example 37

### 2-[[3-[[4-(4,5-Difluoro-2-methylsulfanyl-phenyl)phenoxy]met hyl]benzoyl]-[(1-methyl-4-piperidyl)methyl]amino]acetic Acid Trifluoroacetate

To the compound of Example 35 (20 mg, 0.030 mmol), ethanol (2 mL), acetic acid (0.0080 mL, 0.12 mmol), and paraformaldehyde (4.0 mg, 0.12 mmol) were added, followed by stirring at room temperature for 30 minutes. Then, sodium triacetoxyborohydride (13 mg, 0.060 mmol) was added thereto, followed by stirring at room temperature overnight. The solvent was evaporated under reduced pressure, and the obtained residue was purified by reversed phase HPLC in the same manner as in Step 2 of Example 1 to obtain the title compound.
Yield: 5 mg (0.007 mmol), Percentage yield: 25%
MS (ESI) m/z 555 [M+H]⁺

### Example 38

### 2-[[3-[[4-(4,5-Difluoro-2-methylsulfanyl-phenyl)phenoxy]met hyl]benzoyl]-[(1-ethyl-4-piperidyl)methyl]amino]acetic Acid Trifluoroacetate

The title compound was obtained by conducting the same operation as in Example 37 by using acetaldehyde instead of paraformaldehyde.
Yield: 3 mg (0.004 mmol), Percentage yield: 15%
MS (ESI) m/z 569 [M+H]⁺

### Example 39

### 2-[[3-[[4-(4,5-Difluoro-2-methylsulfanyl-phenyl)phenoxy]met hyl]benzoyl]-(3-pyridylmethyl)amino]acetic Acid Trifluoroacetate

### Step 1

### Synthesis of Ethyl 2-[[3-[[4-(4,5-Difluoro-2-methylsulfanyl-phenyl)phenoxy]met hyl]benzoyl]-(3-pyridylmethyl)amino]acetate

To a solution of 3-pyridylmethanamine (51 µL, 0.50 mmol) in acetonitrile (4 mL), potassium carbonate (69 mg, 0.50 mmol) was added, followed by cooling to -10°C to -15°C. Then, ethyl 2-bromoacetate (55 µL, 0.50 mmol) diluted with acetonitrile (1 mL) was added dropwise, and the temperature was gradually raised to room temperature, followed by stirring overnight. Insoluble matters were separated by filtration, and the filtrate was concentrated under reduced pressure. The obtained residue was diluted with dichloromethane (4 mL), and DIPEA (87 µL, 0.50 mmol) and Intermediate 2-B (80 mg, 0.20 mmol) were added thereto, followed by stirring at room temperature for 1.5 hours. The reaction liquid was concentrated under reduced pressure to obtain the title compound without purification.
MS (ESI) m/z 563 [M+H]⁺

### Step 2

### Synthesis of Compound of Example 39

The compound obtained in Step 1 was dissolved in 1,4-dioxane (3 mL), and a 1 N aqueous lithium hydroxide solution (0.6 mL) was added, followed by stirring at room temperature for 1 hour. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 14.5 mg (0.0224 mmol), Percentage yield: 11%
MS (ESI) m/z 535 [M+H]⁺
¹H NMR (400 MHz, DMSO-d₆) δ 8.84 - 8.42 (m, 3H), 8.15 - 7.15 (m, 10H), 7.15 - 6.93 (m, 2H), 5.18 (s, 2H), 4.82 - 4.52 (m, 2H), 4.13 - 3.97 (m, 2H), 2.39 (s, 3H).

### Example 40

### 2-[[3-[[4-(4,5-Difluoro-2-methylsulfanyl-phenyl)phenoxy]met hyl]benzoyl]-(2-pyrrolidin-1-ylethyl)amino]acetic Acid Trifluoroacetate

### Step 1

### Synthesis of Benzyl 2-[[3-[[4-(4,5-Difluoro-2-methylsulfanyl-phenyl)phenoxy]met hyl]benzoyl]-(2-pyrrolidin-1-ylethyl)amino]acetate

2-Pyrrolidin-1-ylethanamine (0.063 mL, 0.50 mmol) was diluted with acetonitrile (4 mL), and potassium carbonate (104 mg, 0.750 mmol) was added thereto, followed by cooling to -10°C. Then, benzyl 2-bromoacetate (0.086 mL, 0.55 mmol) diluted with acetonitrile (1 mL) was added dropwise, followed by stirring overnight. After insoluble matters were separated by filtration, the filtrate was concentrated under reduced pressure to obtain a residue. The obtained residue was diluted with dichloromethane (3 mL), and DIPEA (0.044 mL, 0.25 mmol) and Intermediate 2-B (103 mg, 0.254 mmol) were added under ice cooling, followed by stirring at room temperature for 1 hour. The reaction solution was diluted by adding dichloromethane. The organic layer was washed with 1 N hydrochloric acid, a saturated aqueous sodium hydrogen carbonate solution, and saturated aqueous sodium chloride in this order, and then dried over anhydrous magnesium sulfate. The organic solvent was concentrated under reduced pressure to obtain a crude product of Step 2.
Yield: 193 mg
MS (ESI) m/z 631 [M+H]⁺

### Step 2

### Synthesis of Compound of Example 40

The crude product obtained in Step 1 was dissolved in a solvent mixture of THF (2 mL) and methanol (2 mL), and a 1 N aqueous lithium hydroxide solution (1 mL) was added thereto under ice cooling, followed by stirring at room temperature for 1.5 hours. After the reaction liquid was neutralized, the organic solvent was evaporated under reduced pressure, and then the obtained residue was purified by reversed phase HPLC in the same manner as in Step 2 of Example 1 to obtain the title compound.
Yield: 91.6 mg (0.140 mmol), Percentage yield: 56% (Step 1 included)
MS (ESI) m/z 541 [M+H]⁺
¹H NMR (400 MHz, DMSO-d₆) δ 13.1 (s, 1H), 9.63 (s, 1H), 7.67 - 7.23 (m, 8H), 7.14 - 7.00 (m, 2H), 5.28 - 5.11 (m, 2H), 4.24 - 3.99 (m, 2H), 3.87 - 3.00 (m, 8H), 2.40 (s, 3H), 2.12 - 1.69 (m, 4H).

### Example 41

### 2-[[3-[[4-(4,5-Difluoro-2-methylsulfanyl-phenyl)phenoxy]met hyl]benzoyl]-[2-(1,1-dioxo-1,4-thiazinan-4-yl)ethyl]amino]a cetic Acid Trifluoroacetate

### Step 1

### Synthesis of Ethyl 2-[[3-[[4-(4,5-Difluoro-2-methylsulfanyl-phenyl)phenoxy]met hyl]benzoyl]-[2-(1,1-dioxo-1,4-thiazinan-4-yl)ethyl]amino]a cetate

To a solution of 2-(1,1-dioxo-1,4-thiazinan-4-yl)ethanamine (89 mg, 0.50 mmol) in acetonitrile (4 mL), potassium carbonate (69 mg, 0.50 mmol) was added, followed by cooling to -10°C to -15°C. Then, ethyl 2-bromoacetate (55 µL, 0.50 mmol) diluted with acetonitrile (1 mL) was added dropwise. The temperature was gradually raised to room temperature, and the mixture was stirred overnight. Insoluble matters were separated by filtration, and the filtrate was concentrated under reduced pressure. The obtained residue was diluted with dichloromethane (4 mL), and DIPEA (87 µL, 0.50 mmol) and Intermediate 2-B (81 mg, 0.20 mmol) were added thereto, followed by stirring at room temperature for 1.5 hours. The reaction liquid was concentrated under reduced pressure to obtain the title compound without purification.
MS (ESI) m/z 633 [M+H]⁺

### Step 2

### Synthesis of Compound of Example 41

The compound obtained in Step 1 was dissolved in 1,4-dioxane (3 mL), and a 1 N aqueous lithium hydroxide solution (0.8 mL) was added thereto, followed by stirring at room temperature overnight. Further, a 1 N aqueous lithium hydroxide solution (0.2 mL) was added, followed by stirring. The reaction liquid was neutralized with a 1 N aqueous trifluoroacetic acid solution, and evaporated under reduced pressure. The obtained residue was purified by reversed phase HPLC in the same manner as in Step 2 of Example 1 to obtain the title compound.
Yield: 15.5 mg (0.0216 mmol), Percentage yield: 11%
MS (ESI) m/z 605 [M+H]⁺
¹H NMR (400 MHz, DMSO-d₆) δ 7.69 - 7.04 (m, 10H), 5.31 - 5.11 (m, 2H), 4.21 - 4.00 (m, 2H), 3.73 - 2.69 (m, 12H), 2.40 (s, 3H)

### Example 42

### 2-[[3-[[4-(4,5-Difluoro-2-methylsulfanyl-phenyl)phenoxy]met hyl]benzoyl]-(oxazol-2-ylmethyl)amino]acetic Acid

### Step 1

### Synthesis of Ethyl 2-[[3-[[4-(4,5-Difluoro-2-methylsulfanyl-phenyl)phenoxy]met hyl]benzoyl]-(oxazol-2-ylmethyl)amino]acetate

A solution of oxazol-2-ylmethanamine hydrochloride (83 mg, 0.60 mmol) in methanol (2 mL) and a 25% by weight sodium methoxide/methanol solution (0.137 mL) were added, followed by stirring at room temperature for 15 minutes. Then, insoluble matters were filtered off, and the solvent was evaporated under reduced pressure. The obtained residue was diluted with acetonitrile (4 mL), and potassium carbonate (83 mg, 0.60 mmol) was added thereto, followed by cooling to -10°C to -15°C. Then, ethyl 2-bromoacetate (0.066 mL, 0.60 mmol) diluted with acetonitrile (1 mL) was added dropwise. The temperature was gradually raised to room temperature, and the mixture was stirred overnight. After insoluble matters were separated by filtration, the filtrate was concentrated under reduced pressure. The obtained residue was diluted with dichloromethane (4 mL), and DIPEA (104 µL, 0.600 mmol) and Intermediate 2-B (81 mg, 0.20 mmol) were added thereto, followed by stirring at room temperature for 1 hour. The reaction liquid was evaporated under reduced pressure to remove the solvent, and the obtained residue was purified by silica gel chromatography (hexane/ethyl acetate) to obtain the title compound.
MS (ESI) m/z 553 [M+H]⁺

### Step 2

### Synthesis of Compound of Example 42

The compound obtained in Step 1 was dissolved in 1,4-dioxane (3 mL), and a 1 N aqueous lithium hydroxide solution (0.3 mL) was added thereto, followed by stirring at room temperature overnight. The reaction liquid was neutralized with a 1 N aqueous trifluoroacetic acid solution, and evaporated under reduced pressure. The obtained residue was purified by reversed phase HPLC in the same manner as in Step 2 of Example 1 to obtain the title compound.
Yield: 54.3 mg (0.104 mmol), Percentage yield: 52%
MS (ESI) m/z 525 [M+H]⁺
¹H NMR (400 MHz, DMSO-d₆) δ 8.10 (s, 1H), 7. 64 - 7. 01 (m, 11H), 5.26 - 5.12 (m, 2H), 4.87 - 4.56 (m, 2H), 4.18 - 4.02 (m, 2H), 2.39 (s, 3H).

### Example 43

### 2-[[3-[[4-(4,5-Difluoro-2-methylsulfanyl-phenyl)phenoxy]met hyl]benzoyl]-[2-(ethylamino)ethyl]amino]acetic Acid Trifluoroacetate

### Step 1

### Synthesis of Ethyl 2-[2-(tert-Butoxycarbonylamino)ethyl-[3-[[4-(4,5-difluoro-2 -methylsulfanyl-phenyl)phenoxy]methyl]benzoyl]amino]acetate

To a solution of tert-butyl N-(2-aminoethyl)carbamate (111 µL, 0.700 mmol) in acetonitrile (6mL), potassium carbonate (97 mg, 0.70 mmol) was added, followed by cooling to -10°C to -15°C. Then, ethyl 2-bromoacetate (77 µL, 0.70 mmol) diluted with acetonitrile (1 mL) was added dropwise. The temperature was gradually raised to room temperature, and the mixture was stirred overnight. Insoluble matters were separated by filtration, and the filtrate was concentrated under reduced pressure. The obtained residue was diluted with dichloromethane (6 mL), and DIPEA (122 µL, 0.700 mmol) and Intermediate 2-B (100 mg, 0.247 mmol) were added thereto, followed by stirring at room temperature for 1 hour. To the reaction liquid, water was added, followed by extraction with dichloromethane. The organic layer was washed with a saturated aqueous sodium hydrogen carbonate solution and saturated aqueous sodium chloride, and dried over anhydrous magnesium sulfate. The solvent was evaporated under reduced pressure, and then the obtained residue was purified by silica gel chromatography (hexane/ethyl acetate) to obtain the title compound.
MS (ESI) m/z 615 [M+H]⁺

### Step 2

### Synthesis of 2-[2-[tert-Butoxycarbonyl(ethyl)amino]ethyl-[3-[[4-(4,5-dif luoro-2-methylsulfanyl-phenyl)phenoxy]methyl]benzoyl]amino] acetic Acid

The compound obtained in Step 1 was dissolved in a THF solution (5 mL), and sodium hydride (26 mg, 0.60 mmol) and ethyl iodide (80 µL, 1.0 mmol) were added dropwise, followed by stirring at room temperature for 3 hours. Further, sodium hydride (26 mg, 0.60 mmol) and ethyl iodide (64 µL, 0.80 mmol) were added, followed by stirring overnight. Still further, sodium hydride (75 mg, 1.7 mmol) and ethyl iodide (144 µL, 1.80 mmol) were added, followed by stirring overnight. A saturated aqueous ammonium chloride solution was added to the reaction liquid, which was then concentrated under reduced pressure. The obtained residue was purified by reversed phase HPLC in the same manner as in Step 2 of Example 1 to obtain the title compound.
MS (ESI) m/z 615 [M+H]⁺

### Step 3

### Synthesis of Compound of Example 43

The compound obtained in Step 2 was dissolved in dichloromethane (2 mL), and trifluoroacetic acid (80 µL, 1.1 mmol) was added dropwise thereto, followed by stirring overnight. The mixture was concentrated under reduced pressure, and the obtained residue was purified by reversed phase HPLC in the same manner as in Step 2 of Example 1 to obtain the title compound.
Yield: 54.8 mg (0.0872 mmol), Percentage yield: 35%
MS (ESI) m/z 515 [M+H]⁺
¹H NMR (400 MHz, DMSO-d₆) δ 13.31 - 12.80 (m, 1H), 8.58 - 8.12 (m, 2H), 7.80 - 6.91 (m, 10H), 5. 35 - 5.02 (m, 2H), 4 .24 - 3.97 (m, 2H), 3. 80 - 3.52 (m, 2H), 3.27 - 2.81 (m, 4H), 2.40 (s, 3H), 1.32 - 0.97 (m, 3H).

### Example 44

### 2-[2-Acetamidoethyl-[3-[[4-(4,5-difluoro-2-methylsulfanyl-p henyl)phenoxy]methyl]benzoyl]amino]acetic Acid

### Step 1

### Synthesis of Benzyl 2-(2-Acetamidoethylamino)acetate

N-(2-Aminoethyl)acetamide (102 mg, 1.00 mmol) was diluted with acetonitrile (4 mL), and potassium carbonate (207 mg, 1.50 mmol) was added thereto, followed by cooling to 0°C. Then, benzyl 2-bromoacetate (0.172 mL, 1.50 mmol) diluted with acetonitrile (1 mL) was added dropwise, followed by stirring for 5 hours. After insoluble matters were separated by filtration, the filtrate was concentrated under reduced pressure to obtain a crude product of Step 1.
Yield: 265 mg
MS (ESI) m/z 251 [M+H]⁺

### Step 2

### Synthesis of Compound of Example 44

The crude product obtained in Step 1 (265 mg, 1.00 mmol) was diluted with dichloromethane (5 mL), and DIPEA (0.165 mL, 1.00 mmol) and Intermediate 2-B (202 mg, 0.500 mmol) were added thereto under ice cooling, followed by stirring at room temperature for 1 hour. The reaction liquid was concentrated under reduced pressure, and then the obtained residue was dissolved in a solvent mixture of THF (2 mL) and methanol (2 mL). Under ice cooling, a 1 N aqueous sodium hydroxide solution (1.5 mL) was added thereto, followed by stirring at room temperature for 1 hour. After the reaction liquid was neutralized, the organic solvent was evaporated under reduced pressure, and then the obtained residue was purified by reversed phase HPLC in the same manner as in Step 2 of Example 1 to obtain the title compound.
Yield: 103 mg (0.194 mmol), Percentage yield: 38.8%
MS (ESI) m/z 529 [M+H]⁺
¹H NMR (400 MHz, DMSO-d₆) δ 8.00 - 7.80 (m, 1H), 7.59 - 7.22 (m, 8H), 7.12 - 7.03 (m, 2H), 5.30 - 5.08 (m, 2H), 4.24 - 3.84 (m, 2H), 3.58 - 3.39 (m, 2H), 3.33 - 3.12 (m, 2H), 2.39 (s, 3H), 1.90 - 1.64 (m, 3H).

### Example 45

### 2-[[3-[[4-(4,5-Difluoro-2-methylsulfanyl-phenyl)phenoxy]met hyl]benzoyl]-(2-methoxypropyl)amino]acetic Acid

### Step 1

### Synthesis of Ethyl 2-[[3-[[4-(4,5-Difluoro-2-methylsulfanyl-phenyl)phenoxy]met hyl]benzoyl]-(2-methoxypropyl)amino]acetate

To a solution of 2-methoxypropane-1-amine hydrochloride (75 mg, 0.60 mmol) in acetonitrile (4 mL), triethylamine (0.184 mL, 1.32 mmol) was added, followed by cooling to -10°C to -15°C. Then, ethyl 2-bromoacetate (0.066 mL, 0.60 mmol) diluted with acetonitrile (1 mL) was added dropwise thereto. The temperature was gradually raised to room temperature, and the mixture was stirred overnight. After insoluble matters were separated by filtration, the filtrate was concentrated under reduced pressure. The obtained residue was diluted with dichloromethane (4 mL), and DIPEA (104 µL, 0.600 mmol) and Intermediate 2-B (81 mg, 0.20 mmol) were added thereto, followed by stirring at room temperature for 1 hour. To the reaction liquid, water was added, followed by extraction with dichloromethane. The organic layer was washed with saturated aqueous sodium chloride, and dried over anhydrous magnesium sulfate. The solvent was evaporated under reduced pressure, and then the obtained residue was purified by silica gel chromatography (hexane/ethyl acetate) to obtain the title compound.
MS (ESI) m/z 544 [M+H]⁺

### Step 2

### Synthesis of Compound of Example 45

The compound obtained in Step 1 was dissolved in 1,4-dioxane (1 mL), and a 1 N aqueous lithium hydroxide solution (0.18 mL) was added thereto, followed by stirring at room temperature for 2 hours. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 35.3 mg (0.0680 mmol), Percentage yield: 34%
MS (ESI) m/z 516 [M+H]⁺
¹H NMR (400 MHz, DMSO-d₆) δ 7.63 - 7.19 (m, 8H), 7.08 (d, J = 8.6 Hz, 2H), 5.30 - 5.08 (m, 2H), 4.36 - 3.89 (m, 3H), 3.40 - 3.11 (m, 5H), 2.40 (s, 3H), 1.19 - 0.79 (m, 3H).

### Example 46

### 2-[[3-[[4-(4,5-Difluoro-2-methylsulfanyl-phenyl)phenoxy]met hyl]benzoyl]-[(1,1-dioxothiolan-3-yl)methyl]amino]acetic Acid

### Step 1

### Synthesis of Ethyl 2-[[3-[[4-(4,5-Difluoro-2-methylsulfanyl-phenyl)phenoxy]met hyl]benzoyl]-[(1,1-dioxothiolan-3-yl)methyl]amino]acetate

To a solution of (1,1-dioxothiolan-3-yl)methanamine (79 mg, 0.50 mmol) in acetonitrile (4 mL), potassium carbonate (69 mg, 0.50 mmol) was added, followed by cooling to -10°C to -15°C. Then, ethyl 2-bromoacetate (55 µL, 0.50 mmol) diluted with acetonitrile (1 mL) was added dropwise thereto. The temperature was gradually raised to room temperature, and the mixture was stirred overnight. Insoluble matters were separated by filtration, and the filtrate was concentrated under reduced pressure. The obtained residue was diluted with dichloromethane (5 mL), and DIPEA (87 µL, 0.50 mmol) and Intermediate 2-B (81 mg, 0.20 mmol) were added thereto, followed by stirring at room temperature for 1 hour and 45 minutes. The reaction liquid was concentrated under reduced pressure to obtain the title compound without purification.
MS (ESI) m/z 604 [M+H]⁺

### Step 2

### Synthesis of Compound of Example 46

The compound obtained in Step 1 was dissolved in 1,4-dioxane (3 mL), and a 1 N aqueous lithium hydroxide solution (0.8 mL) was added thereto, followed by stirring at room temperature overnight. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 58.3 mg (0.101 mmol), Percentage yield: 51%
MS (ESI) m/z 576 [M+H]⁺
¹H NMR (400 MHz, DMSO-d₆) δ 13.35 - 12.55 (m, 1H), 7. 67 - 7.17 (m, 8H), 7.17 - 7.02 (m, 2H), 5.31 - 5.09 (m, 2H), 4.23 - 1.41 (m, 11H).

### Example 47

### 2-[[3-[[4-(4,5-Difluoro-2-methylsulfanyl-phenyl)phenoxy]met hyl]benzoyl]-(oxazol-5-ylmethyl)amino]acetic Acid

### Step 1

### Synthesis of Benzyl 2-(Oxazol-5-ylmethylamino)acetate

Oxazol-5-ylmethylamine hydrochloride (67.3 mg, 0.500 mmol) was diluted with acetonitrile (3 mL), and potassium carbonate (173 mg, 1.25 mmol) was added thereto, followed by cooling to 0°C. Then, benzyl 2-bromoacetate (0.078 mL, 0.50 mmol) diluted with acetonitrile (1 mL) was added dropwise thereto. While the temperature was gradually returned to room temperature, the mixture was stirred for 15 hours. After insoluble matters were separated by filtration, the filtrate was concentrated under reduced pressure to obtain a crude product of Step 1.
Yield: 100 mg
MS (ESI) m/z 247 [M+H]⁺

### Step 2

### Synthesis of Compound of Example 47

The crude product obtained in Step 1 (100 mg, 1.00 mmol) was diluted with dichloromethane (4 mL), and DIPEA (0.350 mL, 2.00 mmol) and Intermediate 2-B (101 mg, 0.250 mmol) were added thereto under ice cooling, followed by stirring at room temperature for 1.5 hours. The reaction liquid was concentrated under reduced pressure, and then the obtained residue was dissolved in a solvent mixture of THF (2 mL) and methanol (2 mL). Under ice cooling, a 1 N aqueous sodium hydroxide solution (1.0 mL) was added thereto, followed by stirring at room temperature for 2 hours. After the reaction liquid was neutralized, the organic solvent was evaporated under reduced pressure, and then the obtained residue was purified by reversed phase HPLC in the same manner as in Step 2 of Example 1 to obtain the title compound.
Yield: 88.3 mg (0.168 mmol), Percentage yield: 67.3%
MS (ESI) m/z 525 [M+H]⁺
¹H NMR (400 MHz, DMSO-d₆) δ 11.46 - 11.05 (m, 1H), 8.35 (s, 1H), 7.66 - 7.12 (m, 9H), 7.12 - 7.04 (m, 2H), 5.33 - 5.09 (m, 2H), 4.86 - 4.46 (m, 2H), 3.98 - 3.71 (m, 2H), 2.39 (s, 3H).

### Example 48

### 2-[(4-Aminocyclohexyl)-[3-[[4-(4,5-difluoro-2-methylsulfany 1-phenyl)phenoxy]methyl]benzoyl]amino]acetic Acid Trifluoroacetate

The title compound was obtained by conducting the same operation as in Example 35 by using N-BOC-trans-1,4-cyclohexanediamine (585 mg, 2.73 mmol) instead of 1-N-BOC-4-(aminomethyl)piperidine.
Yield: 73 mg (0.11 mmol), Percentage yield: 8%
MS (ESI) m/z 541 [M+H]⁺

### Example 49

### 2-[[3-[[4-(4,5-Difluoro-2-methylsulfanyl-phenyl)phenoxy]met hyl]benzoyl]-[2-(2-methoxyethoxy)ethyl]amino]acetic Acid

The title compound was obtained by conducting the same operation as in Example 34 by using 2-(2-methoxyethoxy)ethanamine (250 mg, 2.10 mmol) instead of 4-(aminomethyl)tetrahydropyran.
Yield: 15 mg (0.027 mmol), Percentage yield: 3%
MS (ESI) m/z 546 [M+H]⁺

### Example 50

### 2-[[3-[[4-(4,5-Difluoro-2-methylsulfanyl-phenyl)phenoxy]met hyl]benzoyl]-(3-piperidylmethyl)amino]acetic Acid Trifluoroacetate

The title compound was obtained by conducting the same operation as in Example 35 by using N-BOC-3-(aminomethyl)piperidine (470 mg, 2.20 mmol) instead of 1-N-BOC-4-(aminomethyl)piperidine.
Yield: 34 mg (0.05 mmol), Percentage yield: 5%
MS (ESI) m/z 541 [M+H]⁺

### Example 51

### 2-[[3-[[4-(4,5-Difluoro-2-methylsulfanyl-phenyl)phenoxy]met hyl]benzoyl]-(2-sulfamoylethyl)amino]acetic Acid

### Step 1

### Synthesis of Ethyl 2-[[3-[[4-(4,5-Difluoro-2-methylsulfanyl-phenyl)phenoxy]met hyl]benzoyl]-(2-sulfamoylethyl)amino]acetate

To 2-aminoethanesulfonamide hydrochloride (96 mg, 0.60 mmol), methanol (2 mL) and a 25% by weight sodium methoxide/methanol solution (0.137 mL) were added, followed by stirring at room temperature for 15 minutes. The reaction liquid was concentrated under reduced pressure, and acetonitrile (4 mL), DMF (2 mL), and potassium carbonate (83 mg, 0.60 mmol) were added thereto, followed by cooling to -10°C to -15°C. Then, ethyl 2-bromoacetate (0.066 mL, 0.60 mmol) diluted with acetonitrile (1 mL) was added dropwise. The temperature was gradually raised to room temperature, and the mixture was stirred overnight. After insoluble matters were separated by filtration, the filtrate was concentrated under reduced pressure. The obtained residue was diluted with dichloromethane (5 mL), and DIPEA (104 µL, 0.600 mmol) and Intermediate 2-B (81 mg, 0.20 mmol) were added thereto, followed by stirring at room temperature for 2 hours. The reaction liquid was concentrated under reduced pressure to obtain the title compound without purification.
MS (ESI) m/z 579 [M+H]⁺

### Step 2

### Synthesis of Compound of Example 51

The compound obtained in Step 1 was dissolved in 1,4-dioxane (3 mL), and a 1 N aqueous lithium hydroxide solution (0.8 mL) was added thereto, followed by stirring at room temperature for 2 hours. Further, a 1 N aqueous lithium hydroxide solution (0.3 mL) was added, followed by stirring overnight. Then, the reaction liquid was neutralized with a 1 N aqueous trifluoroacetic acid solution, and evaporated under reduced pressure. The obtained residue was purified by reversed phase HPLC in the same manner as in Step 2 of Example 1 to obtain the title compound.
Yield: 65.5 mg (0.119 mmol), Percentage yield: 59%
MS (ESI) m/z 551 [M+H]⁺
¹H NMR (400 MHz, DMSO-d₆) δ 7.74 - 7.19 (m, 8H), 7.14 - 6.80 (m, 4H), 5.30 - 5.06 (m, 2H), 4.23 - 3.97 (m, 2H), 3.88 - 3.61 (m, 4H), 2.40 (s, 3H).

### Example 52

### 2-[[3-[[4-(4,5-Difluoro-2-methylsulfanyl-phenyl)phenoxy]met hyl]benzoyl]-(oxazol-4-ylmethyl)amino]acetic Acid

### Step 1

### Synthesis of Ethyl 2-[[3-[[4-(4,5-Difluoro-2-methylsulfanyl-phenyl)phenoxy]met hyl]benzoyl]-(oxazol-4-ylmethyl)amino]acetate

To a solution of oxazol-2-ylmethanamine hydrochloride (67 mg, 0.50 mmol) in acetonitrile (5 mL), potassium carbonate (173 mg, 1.25 mmol) and DMF (1 mL) were added, followed by stirring at room temperature. After the mixture was cooled to a temperature between -10°C and -15°C, ethyl 2-bromoacetate (55 µL, 0.50 mmol) diluted with acetonitrile (1 mL) was added dropwise thereto. The temperature was gradually raised to room temperature, and the mixture was stirred overnight. Insoluble matters were separated by filtration, and the filtrate was concentrated under reduced pressure. The obtained residue was diluted with dichloromethane (5 mL), and DIPEA (87 µL, 0.50 mmol) and Intermediate 2-B (81 mg, 0.20 mmol) were added thereto, followed by stirring at room temperature for 1 hour. The reaction liquid was concentrated under reduced pressure to obtain the title compound without purification.
MS (ESI) m/z 553 [M+H]⁺

### Step 2

### Synthesis of Compound of Example 52

The compound obtained in Step 1 was dissolved in 1,4-dioxane (3 mL), and a 1 N aqueous lithium hydroxide solution (1.0 mL) was added thereto, followed by stirring at room temperature overnight. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 44.0 mg (0.0839 mmol), Percentage yield: 42%
MS (ESI) m/z 525 [M+H]⁺
¹H NMR (400 MHz, DMSO-d₆) δ 8.45 - 8.35 (m, 1H), 8.12 (s, 1H), 7.71 - 7.23 (m, 9H), 7.08 (d, J = 8.8 Hz, 2H), 5.30 - 5.09 (m, 2H), 4.65 - 4.31 (m, 2H), 4.07 - 3.94 (m, 2H), 2.40 (s, 3H).

### Example 53

### 2-[[3-[[4-(4,5-Difluoro-2-methylsulfanyl-phenyl)phenoxy]met hyl]benzoyl]-(pyrimidin-4-ylmethyl)amino]acetic Acid

### Step 1

### Synthesis of Ethyl 2-[[3-[[4-(4,5-Difluoro-2-methylsulfanyl-phenyl)phenoxy]met hyl]benzoyl]-(pyrimidin-4-ylmethyl)amino]acetate

To a solution of pyrimidin-4-ylmethanamine (55 mg, 0.50 mmol) in acetonitrile (4 mL), potassium carbonate (69 mg, 0.50 mmol) was added. After the mixture was cooled to a temperature between -10°C and -15°C, ethyl 2-bromoacetate (55 µL, 0.50 mmol) diluted with acetonitrile (1 mL) was added dropwise thereto. The temperature was gradually raised to room temperature, and the mixture was stirred for 4 hours. Insoluble matters were separated by filtration, and the filtrate was concentrated under reduced pressure. The obtained residue was diluted with dichloromethane (4 mL), and DIPEA (87 µL, 0.50 mmol) and Intermediate 2-B (81 mg, 0.20 mmol) were added thereto, followed by stirring at room temperature for 1 hour. The reaction liquid was concentrated under reduced pressure to obtain the title compound without purification.
MS (ESI) m/z 564 [M+H]⁺

### Step 2

### Synthesis of Compound of Example 53

The compound obtained in Step 1 was dissolved in 1,4-dioxane (3 mL), and a 1 N aqueous lithium hydroxide solution (0.8 mL) was added thereto, followed by stirring at room temperature for 2 hours. The reaction liquid was neutralized with a 1 N aqueous trifluoroacetic acid solution, and then evaporated under reduced pressure. The obtained residue was purified by reversed phase HPLC in the same manner as in Step 2 of Example to obtain the title compound.
Yield: 54.7 mg (0.102 mmol), Percentage yield: 51%
MS (ESI) m/z 536 [M+H]⁺
¹H NMR (400 MHz, DMSO-d₆) δ 9.14 (d, J = 3.7, 1.4 Hz, 1H), 8.76 (t, J = 5.7 Hz, 1H), 7.66 - 7.17 (m, 9H), 7.09 (d, J = 8.7 Hz, 1H), 6.99 (d, J = 8.7 Hz, 1H), 5.26 - 5.07 (m, 2H), 4.80 - 4.57 (m, 2H), 4.20 - 4.09 (m, 2H), 2.39 (s, 3H).

### Example 54

### 2-[[3-[[4-(4,5-Difluoro-2-methylsulfanyl-phenyl)phenoxy]met hyl]benzoyl]-(1H-pyrazol-5-ylmethyl)amino]acetic Acid

### Step 1

### Synthesis of Benzyl 2-[[3-[[4-(4,5-Difluoro-2-methylsulfanyl-phenyl)phenoxy]met hyl]benzoyl]-(1H-pyrazol-5-ylmethyl)amino]acetate

To 1H-pyrazole-5-carbaldehyde (48 mg, 0.50 mmol), benzyl 2-aminoacetate tosylate (186 mg, 0.550 mmol), acetic acid (1 mL), and dichloromethane (1.5 mL) were added, followed by stirring at room temperature for 15 minutes. After the mixture was cooled to 0°C, sodium triacetoxyborohydride (159 mg, 0.750 mmol) and dichloromethane (2 mL) were added thereto, followed by stirring at room temperature for 6 hours. Saturated aqueous sodium hydrogen carbonate was added, followed by extraction with dichloromethane and ethyl acetate. The organic layer was washed with water and saturated aqueous sodium chloride, dried over anhydrous magnesium sulfate, and concentrated under reduced pressure. The obtained residue was diluted with dichloromethane (3.5 mL), and DIPEA (48 µL, 0.281 mmol) and Intermediate 2-B (38 mg, 0.094 mmol) were added thereto, followed by stirring at room temperature for 2 hours. The reaction liquid was concentrated under reduced pressure to obtain the title compound without purification.
MS (ESI) m/z 614 [M+H]⁺

### Step 2

### Synthesis of Compound of Example 54

The compound obtained in Step 1 was dissolved in 1,4-dioxane (1 mL), and a 1 N aqueous lithium hydroxide solution (0.45 mL) was added thereto, followed by stirring at room temperature for 1 hour. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 9.7 mg (0.019 mmol), Percentage yield: 20%
MS (ESI) m/z 524 [M+H]⁺
¹H NMR (400 MHz, DMSO-d₆) δ 7.75 - 7.16 (m, 10H), 7.07 (d, J = 8.6, 1.7 Hz, 2H), 6.30 - 6.16 (m, 1H), 5.25 - 5.13 (m, 2H), 4.70 - 4.38 (m, 2H), 4.07 - 3.82 (m, 2H), 2.39 (s, 3H).

### Example 55

### 2-[[3-[[4-(4,5-Difluoro-2-methylsulfanyl-phenyl)phenoxy]met hyl]benzoyl]-[(2-methylpyrazol-3-yl)methyl]amino]acetic Acid

### Step 1

### Synthesis of Benzyl 2-[[3-[[4-(4,5-Difluoro-2-methylsulfanyl-phenyl)phenoxy]met hyl]benzoyl]-[(2-methylpyrazol-3-yl)methyl]amino]acetate

To 2-methylpyrazole-3-carbaldehyde (55 mg, 0.50 mmol), benzyl 2-aminoacetate tosylate (186 mg, 0.550 mmol), acetic acid (1 mL), and dichloromethane (1.5 mL) were added, followed by stirring at room temperature. Sodium triacetoxyborohydride (159 mg, 0.750 mmol) was added thereto, followed by stirring at room temperature overnight. The reaction liquid was diluted with dichloromethane, and saturated aqueous sodium hydrogen carbonate was added thereto. After extraction with dichloromethane, the organic layer was washed with water and saturated aqueous sodium chloride, dried over anhydrous magnesium sulfate, and concentrated under reduced pressure. The obtained residue was diluted with dichloromethane (3 mL), and DIPEA (61 µL, 0.35 mmol) and Intermediate 2-B (57 mg, 0.14 mmol) were added thereto, followed by stirring at room temperature for 2 hours. The reaction liquid was concentrated under reduced pressure to obtain the title compound without purification.

### Step 2

### Synthesis of Compound of Example 55

The compound obtained in Step 1 was dissolved in 1,4-dioxane (1 mL), and a 1 N aqueous lithium hydroxide solution (0.563 mL) was added thereto, followed by stirring at room temperature overnight. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 10.0 mg (0.0190 mmol), Percentage yield: 13%
MS (ESI) m/z 538 [M+H]⁺
¹H NMR (400 MHz, DMSO-d₆) δ 7.75 - 6.97 (m, 11H), 6.36 - 6.27 (m, 1H), 5.18 (s, 2H), 4.85 - 4.49 (m, 2H), 4.12 - 3.76 (m, 2H), 3.51 (s, 3H), 2.39 (s, 3H).

### Example 56

### 2-[[3-[[4-(4,5-Difluoro-2-methylsulfanyl-phenyl)phenoxy]met hyl]benzoyl]-[2-(methylsulfamoyl)ethyl]amino]acetic Acid

### Step 1

### Synthesis of Ethyl 2-[[3-[[4-(4,5-Difluoro-2-methylsulfanyl-phenyl)phenoxy]met hyl]benzoyl]-[2-(methylsulfamoyl)ethyl]amino]acetate

To a solution of 2-amino-N-methyl-ethanesulfonamide (59 mg, 0.50 mmol) in acetonitrile (4 mL), DMF (1 mL) and potassium carbonate (69 mg, 0.50 mmol) were added. After the mixture was cooled to a temperature between -10°C and -15°C, ethyl 2-bromoacetate (55 µL, 0.50 mmol) diluted with acetonitrile (1 mL) was added dropwise thereto. The temperature was gradually raised to room temperature, and the mixture was stirred overnight. Insoluble matters were separated by filtration, and the filtrate was concentrated under reduced pressure. The obtained residue was diluted with dichloromethane (4 mL), and DIPEA (87 µL, 0.50 mmol) and Intermediate 2-B (81 mg, 0.20 mmol) were added thereto, followed by stirring at room temperature for 1 hour. The reaction liquid was concentrated under reduced pressure to obtain the title compound without purification. MS (ESI) m/z 593 [M+H]⁺

### Step 2

### Synthesis of Compound of Example 56

The compound obtained in Step 1 was dissolved in 1,4-dioxane (3 mL), and a 1 N aqueous lithium hydroxide solution (0.8 mL) was added thereto, followed by stirring at room temperature overnight. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 69.9 mg (0.124 mmol), Percentage yield: 62%
MS (ESI) m/z 565 [M+H]⁺
¹H NMR (400 MHz, DMSO-d₆) δ 7.74 - 6.80 (m, 11H), 5.30 - 5.01 (m, 2H), 4.24 - 3.95 (m, 2H), 3.83 - 3.28 (m, 4H), 2.70 - 2.57 (m, 2H), 2.44 - 2.33 (m, 4H).

### Example 57

### (2S, 5R) or (2S,5S)-5-Carbamoyl-1-[3-[[4-(4,5-difluoro-2-methylsulfanyl -phenyl)phenoxy]methyl]benzoyl]pyrrolidine-2-carboxylic Acid

Ethyl (2S,5R) or (2S,5S)-5-cyanopyrrolidine-2-carboxylate (285 mg, 1.00 mmol) obtained in Step 1 of Example 4 was diluted with dichloromethane (5 mL), and DIPEA (0.440 mL, 2.50 mmol) and Intermediate 2-B (202 mg, 0.500 mmol) were added thereto, followed by stirring at room temperature for 18 hours. The reaction liquid was concentrated under reduced pressure, and then the obtained residue was dissolved in a solvent mixture of THF (3 mL) and methanol (3 mL). Under ice cooling, a 1 N aqueous sodium hydroxide solution (2.5 mL) was added thereto, followed by stirring at room temperature for 1 hour. After the reaction liquid was neutralized, the organic solvent was evaporated under reduced pressure, and then the obtained residue was purified by reversed phase HPLC in the same manner as in Step 2 of Example 1 to obtain the title compound.
Yield: 50.3 mg (0.0955 mmol), Percentage yield: 19.1%
MS (ESI) m/z 527 [M+H]⁺
¹H NMR (400 MHz, DMSO-d₆) δ 8.11 - 7.24 (m, 10H), 7.09 (d, J = 8.1 Hz, 2H), 5.17 (s, 2H), 4.63 - 4.14 (m, 2H), 2.39 (s, 3H), 2.38 - 2.20 (m, 2H), 2.06 - 1.76 (m, 2H).

### Example 58

### (2S,5S) or (2S,5R)-5-Carbamoyl-1-[3-[[4-(4,5-difluoro-2-methylsulfanyl -phenyl)phenoxy]methyl]benzoyl]pyrrolidine-2-carboxylic Acid

Ethyl (2S,5R) or (2S,5S)-5-cyanopyrrolidine-2-carboxylate (443 mg, 1.56 mmol) obtained in Step 1 of Example 4 was diluted with dichloromethane (5 mL), and DIPEA (0. 68 mL, 3. 89 mmol) and Intermediate 2-B (157 mg, 0.369 mmol) were added thereto, followed by stirring at room temperature for 21 hours. The reaction liquid was concentrated under reduced pressure, and then the obtained residue was dissolved in a solvent mixture of THF (4 mL) and methanol (2 mL). Under ice cooling, a 1 N aqueous sodium hydroxide solution (2.0 mL) was added thereto, followed by stirring at room temperature for 1 hour. After the reaction liquid was neutralized, the organic solvent was evaporated under reduced pressure. Then, the obtained residue was purified by reversed phase HPLC in the same manner as in Step 2 of Example 1 to obtain the title compound as an isomer of Example 57.
Yield: 18.7 mg (0.0355 mmol), Percentage yield: 9.62%
MS (ESI) m/z 527 [M+H]⁺

### Example 59

### 2-[[3-[[4-(4,5-Difluoro-2-methylsulfanyl-phenyl)phenoxy]met hyl]benzoyl]-[2-(dimethylsulfamoyl)ethyl]amino]acetic Acid

### Step 1

### Synthesis of Ethyl 2-[[3-[[4-(4,5-Difluoro-2-methylsulfanyl-phenyl)phenoxy]met hyl]benzoyl]-[2-(dimethylsulfamoyl)ethyl]amino]acetate

To a solution of 2-amino-N,N-dimethyl-ethanesulfonamide hydrochloride (94 mg, 0.50 mmol) in acetonitrile (4 mL), DMF (1 mL) and potassium carbonate (173 mg, 1.25 mmol) were added. After the mixture was cooled to a temperature between -10°C and -15°C, ethyl 2-bromoacetate (55 µL, 0.50 mmol) diluted with acetonitrile (1 mL) was added dropwise thereto. The temperature was gradually raised to room temperature, and the mixture was stirred for 6 hours. Insoluble matters were separated by filtration, and the filtrate was concentrated under reduced pressure. The obtained residue was diluted with dichloromethane (4 mL), and DIPEA (87 µL, 0.50 mmol) and Intermediate 2-B (81 mg, 0.20 mmol) were added thereto, followed by stirring at room temperature for 1 hour. The reaction liquid was concentrated under reduced pressure to obtain the title compound without purification.
MS (ESI) m/z 607 [M+H]⁺

### Step 2

### Synthesis of Compound of Example 59

The compound obtained in Step 1 was dissolved in 1,4-dioxane (3 mL), and a 1 N aqueous lithium hydroxide solution (0.8 mL) was added thereto, followed by stirring at room temperature for 2 hours. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 27.0 mg (0.0466 mmol), Percentage yield: 23%
MS (ESI) m/z 579 [M+H]⁺
¹H NMR (400 MHz, DMSO-d₆) δ 7.65 - 7.20 (m, 8H), 7.14 - 7.01 (m, 2H), 5.32 - 5.11 (m, 2H), 4.25 - 4.02 (m, 2H), 3.83 - 3.38 (m, 4H), 2.82 (s, 3H), 2.61 (s, 3H), 2.39 (s, 3H).

### Example 60

### 2-[[3-[[4-(4,5-Difluoro-2-methylsulfanyl-phenyl)phenoxy]met hyl]benzoyl]-[2-(5-methyl-1,2,4-oxadiazol-3-yl)ethyl]amino] acetic Acid

### Step 1

### Synthesis of Ethyl 2-[2-Cyanoethyl-[3-[[4-(4,5-difluoro-2-methylsulfanyl-pheny 1)phenoxy]methyl]benzoyl]amino]acetate

The compound of Example 7 (258 mg, 0.520 mmol) was dissolved in ethanol (10 mL) and dichloromethane (5 mL), and WSC hydrochloride (110 mg, 0.572 mmol), HOBt monohydrate (87.6 mg, 0.572 mmol), and triethylamine (0.0800 mL, 0.572 mmol) were added thereto, followed by stirring at room temperature overnight. The reaction liquid was diluted with ethyl acetate. The organic layer was washed with a saturated aqueous sodium hydrogen carbonate solution, a saturated aqueous ammonium chloride solution, and saturated aqueous sodium chloride in this order, and then dried over anhydrous magnesium sulfate. The solvent was removed under reduced pressure to obtain a crude product of Step 1.
Yield: 281 mg
MS (ESI) m/z 525 [M+H]⁺

### Step 2

### Synthesis of Ethyl 2-[[3-[[4-(4,5-Difluoro-2-methylsulfanyl-phenyl)phenoxy]met hyl]benzoyl]-[3-(hydroxyamino)-3-imino-propyl]amino]acetate Trifluoroacetate

The crude product obtained in Step 1 (281 mg) was dissolved in ethanol (6 mL), and a 50% aqueous hydroxyamine solution (0.064 mL, 0.52 mmol) was added thereto, followed by stirring at 90°C overnight. The reaction liquid was evaporated under reduced pressure, and then the obtained residue was purified by reversed phase HPLC in the same manner as in Step 2 of Example 1 to obtain the title compound.
Yield: 175 mg (0.260 mmol), Percentage yield: 50% (from Step 1)
MS (ESI) m/z 558 [M+H]⁺

### Step 3

### Synthesis of Ethyl 2-[[3-[[4-(4,5-Difluoro-2-methylsulfanyl-phenyl)phenoxy]met hyl]benzoyl]-[2-(5-methyl-1,2,4-oxadiazol-3-yl)ethyl]amino] acetate

The compound obtained in Step 2 (80.6 mg, 0.120 mmol) was dissolved in acetonitrile (2 mL), and p-toluenesulfonic acid monohydrate (6.9 mg, 0.036 mmol) and zinc chloride (4.9 mg, 0.036 mmol) were added thereto, followed by stirring at 90°C overnight. The reaction liquid was diluted with dichloromethane. The organic layer was washed with a 0.5 N aqueous hydrochloric acid solution, a saturated aqueous sodium hydrogen carbonate solution, and saturated aqueous sodium chloride in this order, and then dried over anhydrous magnesium sulfate. The solvent was removed under reduced pressure to obtain a crude product of Step 3.
Yield: 64.3 mg
MS (ESI) m/z 582 [M+H]⁺

### Step 4

### Synthesis of Compound of Example 60

The compound obtained in Step 3 (64.3 mg) was dissolved in a solvent mixture of THF (2 mL) and methanol (2 mL). Under ice cooling, a 1 N aqueous lithium hydroxide solution (0.5 mL) was added thereto, followed by stirring at room temperature for 1 hour. After the reaction liquid was neutralized, the organic solvent was evaporated under reduced pressure, and then the obtained residue was purified by reversed phase HPLC in the same manner as in Step 2 of Example 1 to obtain the title compound.
Yield: 27.6 mg (0.0499 mmol), Percentage yield: 42% (from Step 3)
MS (ESI) m/z 554 [M+H]⁺
¹H NMR (400 MHz, DMSO-d₆) δ 13.1 - 12.6 (m, 1H), 7.58 - 7.52 (m, 1H), 7.52 - 7.44 (m, 1H), 7.44 - 7.40 (m, 1H), 7.40 - 7.22 (m, 5H), 7.12 - 7.04 (m, 2H), 5.25 - 5.12 (m, 2H), 4.22 - 3.95 (m, 2H), 3.82 - 3.58 (m, 2H), 3.10 - 2.91 (m, 2H), 2.60 - 2.45 (m, 3H), 2.39 (s, 3H).

### Example 61

### (2S,4R)-4-Carbamoyl-1-[3-[[4-(4,5-difluoro-2-methylsulfanyl -phenyl)phenoxy]methyl]benzoyl]pyrrolidine-2-carboxylic Acid

### Step 1

### Synthesis of Ethyl (2S,4R)-4-Carbamoylpyrrolidine-2-carboxylate Hydrochloride

To a solution of N-Boc-cis-4-hydroxy-L-proline ethyl ester (540 mg, 2.08 mmol) in dichloromethane (9 mL), triethylamine (0.870 mL, 6.24 mmol) was added, and the mixture was cooled to 0°C. Then, p-toluenesulfonyl chloride (595 mg, 3.12 mmol) was added, followed by stirring at room temperature for 5 hours. 4-Dimethylaminopyridine (hereinafter, DMAP) (17 mg, 0.139 mmol) was added thereto, followed by stirring at room temperature overnight. Dichloromethane and 0.1 N hydrochloric acid were added, followed by extraction. The organic layer was washed with saturated aqueous sodium hydrogen carbonate and saturated aqueous sodium chloride, and dried over anhydrous magnesium sulfate. After concentration under reduced pressure, the obtained residue was purified by reversed phase HPLC in the same manner as in Step 2 of Example 1. To the obtained compound, dimethyl sulfoxide (2 mL) and potassium cyanide (25 mg, 0.38 mmol) were added, followed by stirring at 80°C overnight. The mixture was diluted with ethyl acetate. The organic layer was washed with saturated aqueous sodium hydrogen carbonate and saturated aqueous sodium chloride, and dried over anhydrous magnesium sulfate. The solvent was evaporated under reduced pressure, and then the residue was purified by silica gel chromatography (hexane/ethyl acetate). To the obtained compound, 1,4-dioxane (0.7 mL) and 4 N hydrochloric acid/1,4-dioxane (0.165 mL) were added, followed by stirring at room temperature. Then, while the progress of the reaction was being monitored, 4 N hydrochloric acid/1,4-dioxane was added portionwise. After the mixture was stirred for three days and the reaction was found to be completed, the mixture was concentrated under reduced pressure to obtain the title compound without purification.

### Step 2

### Synthesis of Ethyl (2S,4R)-4-Carbamoyl-1-[3-[[4-(4,5-difluoro-2-methylsulfanyl -phenyl)phenoxy]methyl]benzoyl]pyrrolidine-2-carboxylate

The compound obtained in Step 1 was diluted with dichloromethane (2 mL), and DIPEA (92 µL, 0.53 mmol) and Intermediate 2-B (53 mg, 0.13 mmol) were added thereto, followed by stirring at room temperature overnight. The reaction liquid was concentrated under reduced pressure to obtain the title compound without purification.
MS (ESI) m/z 555 [M+H]⁺

### Step 2

### Synthesis of Compound of Example 61

The compound obtained in Step 2 was dissolved in 1,4-dioxane (1 mL), and a 1 N aqueous lithium hydroxide solution (0.211 mL) was added thereto, followed by stirring at room temperature for 2 hours. Further, while the progress of the reaction was being monitored, a 1 N aqueous lithium hydroxide solution was added portionwise, followed by stirring. After the reaction was found to be completed, the mixture was neutralized with a 1 N aqueous trifluoroacetic acid solution, and evaporated under reduced pressure. The obtained residue was purified by reversed phase HPLC in the same manner as in Step 2 of Example 1 to obtain the title compound.
Yield: 32.2 mg (0.0611 mmol), Percentage yield: 3%
MS (ESI) m/z 527 [M+H]⁺
¹H NMR (400 MHz, DMSO-d₆) δ 7.80 - 7.17 (m, 9H), 7.17 - 6.89 (m, 3H), 5.30 - 5.10 (m, 2H), 4.58 - 4.35 (m, 1H), 3.84 - 3.64 (m, 1H), 3.19 - 2.87 (m, 2H), 2.39 (s, 3H), 2.21 - 1.88 (m, 2H).

### Example 62

### (2S,5S)-1-[3-[[4-(4,5-Difluoro-2-methylsulfanyl-phenyl)phen oxy]methyl]benzoyl]-5-(methoxymethyl)pyrrolidine-2-carboxyl ic Acid

### Step 1

### Synthesis of [(2S)-5-Oxopyrrolidin-2-yl]methyl Benzoate

(5S)-5-(Hydroxymethyl)pyrrolidin-2-one (1.00 g, 8.69 mmol) was dissolved in dichloromethane (45 mL), and benzoyl chloride (1.47 g, 10.4 mmol) and triethylamine (2.20 mL, 21.7 mmol) were added thereto, followed by stirring at room temperature for 62 hours. The reaction liquid was evaporated under reduced pressure, and then diluted by adding ethyl acetate. The organic layer was washed with an aqueous ammonium chloride solution, water, and saturated aqueous sodium chloride in this order, and dried over anhydrous sodium sulfate. The organic solvent was concentrated under reduced pressure, and the obtained residue was purified by silica gel column chromatography (dichloromethane/methanol) to obtain the title compound.
Yield: 1.35 g (6.26 mmol), Percentage yield: 71%
MS (ESI) m/z 220 [M+H]⁺

### Step 2

### Synthesis of [(2S,5S)-5-Cyanopyrrolidin-2-yl]methyl Benzoate

The compound of Step 1 (1.35 g, 6.16 mmol) was dissolved in THF (60 mL). Under ice cooling, zirconocene chloride hydride (2.07 g, 8.01 mmol) was added thereto. While the temperature was gradually returned to room temperature, the mixture was stirred for 2 hours. To the reaction liquid, trimethylsilyl cyanide (0.734 mL, 7.40 mmol) was added, followed by stirring at room temperature for 17 hours. The reaction liquid was evaporated under reduced pressure, and then diluted by adding dichloromethane. The organic layer was washed with an aqueous sodium hydrogen carbonate solution, water, and saturated aqueous sodium chloride in this order, and dried over anhydrous sodium sulfate. The organic solvent was concentrated under reduced pressure, and the obtained residue was purified by silica gel column chromatography (dichloromethane/methanol) to obtain the title compound.
Yield: 1.26 g (5.44 mmol), Percentage yield: 89%
MS (ESI) m/z 231 [M+H]⁺

### Step 3

### Synthesis of Ethyl (2S,5S)-5-(Benzoyloxymethyl)pyrrolidine-2-carboxylate Hydrochloride

The compound of Step 2 (1.00 g, 4.34 mmol) was dissolved in a mixture solution of a 4 N hydrochloric acid/1,4-dioxane solution (5.1 mL) and ethanol (1.3 mL), followed by stirring at room temperature for 17 hours. The reaction liquid was evaporated under reduced pressure, and then the obtained residue was dissolved in a mixture solution of ethanol (2.5 mL) and water (2.5 mL), followed by stirring at room temperature for 3 hours. The reaction liquid was evaporated under reduced pressure, and then the obtained residue was purified by reversed phase HPLC in the same manner as in Step 2 of Example 1 to obtain a residue. The obtained residue was dissolved in a 0.05 N aqueous hydrochloric acid solution and freeze dried to obtain the title compound.
Yield: 640 mg (2.05 mmol), Percentage yield: 47%
MS (ESI) m/z 278 [M+H]⁺

### Step 4

### Synthesis of (2S,5S)-1-[3-[[4-(4,5-Difluoro-2-methylsulfanyl-phenyl)phen oxy]methyl]benzoyl]-5-(hydroxymethyl)pyrrolidine-2-carboxyl iC Acid

The compound obtained in Step 3 (313 mg, 1.00 mmol) was diluted with dichloromethane (10 mL), and DIPEA (0.63 mL, 3.64 mmol) and Intermediate 2-B (368 mg, 0.909 mmol) were added thereto, followed by stirring at room temperature for 16 hours . The reaction liquid was concentrated under reduced pressure, and then the obtained residue was dissolved in a solvent mixture of THF (5 mL) and methanol (5 mL). Under ice cooling, a 1 N aqueous sodium hydroxide solution (4.6 mL) was added thereto, followed by stirring at room temperature for 2 hours. After the reaction liquid was neutralized, the organic solvent was evaporated under reduced pressure, and then the obtained residue was purified by reversed phase HPLC in the same manner as in Step 2 of Example 1 to obtain the title compound.
Yield: 106 mg (0.207 mmol), Percentage yield: 22.8%
MS (ESI) m/z 514 [M+H]⁺

### Step 5

### Synthesis of Compound of Example 62

The compound obtained in Step 4 (106 mg, 0.207 mmol) was dissolved in THF (2.0 mL), and the solution was cooled to 0°C. To this solution, sodium hydride (41.4 mg, 1.04 mmol) was added, followed by stirring for 30 minutes. Then, methyl iodide (0.130 mL, 2.07 mmol) was added. While the temperature was being returned gradually to room temperature, the mixture was stirred for 5 hours. After the reaction liquid was neutralized, the organic solvent was evaporated under reduced pressure, and then the obtained residue was purified by reversed phase HPLC in the same manner as in Step 2 of Example 1 to obtain the title compound.
Yield: 87.1 mg (0.165 mmol), Percentage yield: 79.8%
MS (ESI) m/z 528 [M+H]⁺
¹H NMR (400 MHz, DMSO-d₆) δ 7.61 - 7.23 (m, 8H), 7.11 - 7.04 (m, 2H), 5.30 - 5.12 (m, 2H), 4.54 - 4.04 (m, 2H), 3.57 - 3.36 (m, 1H), 3.34 - 2.83 (m, 3H), 3.00 - 2.86 (m, 1H), 2.46 - 2.35 (m, 3H), 2.34 - 1.99 (m, 1H), 1.98 - 1.75 (m, 3H).

### Example 63

### (2S,4R)-1-[3-[[4-(4,5-Difluoro-2-methylsulfanyl-phenyl)phen oxy]methyl]benzoyl]-4-(dimethylamino)pyrrolidine-2-carboxyl ic Acid Trifluoroacetate

### Step 1

### Synthesis of 02-Methyl O1-tert-Butyl (2S,4S)-4-(p-Tolylsulfonyl)pyrrolidine-1,2-dicarboxylic Acid

To a solution of N-Boc-cis-4-hydroxy-L-proline methyl ester (500 mg, 2.04 mmol) in dichloromethane (10 mL), triethylamine (0.853 mL, 6.12 mmol), DMAP (25 mg, 0.20 mmol), and p-toluenesulfonyl chloride (583 mg, 3.06 mmol) were added, followed by stirring at room temperature overnight. After DMAP (25 mg, 0.20 mmol) was added, the mixture was stirred overnight once again. Dichloromethane and 0.1 N hydrochloric acid were added. After extraction, the organic layer was washed with saturated aqueous sodium hydrogen carbonate and saturated aqueous sodium chloride, and dried over anhydrous magnesium sulfate. After concentration under reduced pressure, the obtained residue was purified by silica gel chromatography (hexane/ethyl acetate) to obtain the title compound.
Yield: 801 mg (2.01 mmol), Percentage yield: 98%

### Step 2

### Synthesis of Methyl (2S,4R)-4-(Dimethylamino)pyrrolidine-2-carboxylate Trifluoroacetate

To the compound obtained in Step 1 (300 mg, 0.752 mmol) , acetonitrile (2 mL) and a 2 M dimethylamine/tetrahydrofuran solution (4 mL) were added, and stirred for 3 hours at 160°C under microwave irradiation. The reaction liquid was evaporated under reduced pressure, and the obtained residue was purified by reversed phase HPLC in the same manner as in Step 2 of Example 1. Trifluoroacetic acid (0.276 mL) diluted with dichloromethane (5 mL) was added dropwise to the resulting purified product, followed by stirring at room temperature for 7 hours. After concentration under reduced pressure, acetonitrile and water were added, followed by freeze drying. Thus, the title compound was obtained without purification.

### Step 3

### Synthesis of Methyl (2S,4R)-1-[3-[[4-(4,5-Difluoro-2-methylsulfanyl-phenyl)phen oxy]methyl]benzoyl]-4-(dimethylamino)pyrrolidine-2-carboxyl ate

The compound obtained in Step 2 was diluted with dichloromethane (5 mL), and DIPEA (313 µL, 1.80 mmol) and Intermediate 2-B (146 mg, 0.360 mmol) were added thereto, followed by stirring at room temperature for 2 hours. The reaction liquid was concentrated under reduced pressure to obtain the title compound without purification.
MS (ESI) m/z 541 [M+H]⁺

### Step 4

### Synthesis of Compound of Example 63

To the compound obtained in Step 3, 1,4-dioxane (2 mL) was added, and then a 1 N aqueous lithium hydroxide solution (0.576 mL) was added, followed by stirring for 1.5 hours. Further, a 1N aqueous lithium hydroxide solution (1.15 mL) and water (1 mL) were added, followed by stirring for 1.5 hours. After neutralization with a 1 N aqueous trifluoroacetic acid solution and evaporation under reduced pressure, the obtained residue was purified by reversed phase HPLC in the same manner as in Step 2 of Example 1 to obtain the title compound.
Yield: 139 mg (0.216 mmol), Percentage yield: 60%
MS (ESI) m/z 527 [M+H]⁺
¹H NMR (400 MHz, DMSO-d₆) δ 10.27 - 9.81 (m, 1H), 7.83 - 6.86 (m, 10H), 5.36 - 5.00 (m, 2H), 4.76 - 4.47 (m, 1H), 4.08 - 3.64 (m, 3H), 2.96 - 2.63 (m, 6H), 2.63 - 2.36 (m, 5H).

### Example 64

### (2S,5R)-1-[3-[[4-(4,5-Difluoro-2-methylsulfanyl-phenyl)phen oxy]methyl]benzoyl]-5-(hydroxymethyl)pyrrolidine-2-carboxyl ic Acid

### Step 1

### Synthesis of [(2R)-5-Cyanopyrrolidin-2-yl]methyl Benzoate

To [(3S)-5-oxopyrrolidin-3-yl]methyl benzoate (1.11 g, 5.09 mmol), tetrahydrofuran (50 mL) was added, followed by cooling to 0°C. Then, zirconocene chloride hydride (1.71 g, 6.61 mmol) was added, followed by stirring for 1 hour. The temperature was returned to room temperature. After stirring for 1 hour, trimethylsilyl cyanide (0.757 mL, 6.10 mmol) was added, followed by stirring at room temperature overnight. Saturated aqueous sodium hydrogen carbonate was added, followed by extraction with dichloromethane. Then, the organic layer was washed with saturated aqueous sodium chloride, dried over anhydrous magnesium sulfate, and concentrated under reduced pressure. The obtained residue was purified by silica gel chromatography (hexane/ethyl acetate) to obtain the title compound.
Yield: 835 mg (3.63 mmol), Percentage yield: 71%

### Step 2

### Synthesis of Ethyl (5R)-5-(Benzoyloxymethyl)pyrrolidine-2-carboxylate Hydrochloride

To the compound obtained in Step 1 (835 mg, 3.63 mmol), ethanol (1.07 mL) and 4 N hydrochloric acid/1,4-dioxane (4.5 mL) were added, followed by stirring at room temperature overnight. After concentration under reduced pressure and drying in a vacuum, ethanol/water (4.2 mL) was added, followed by stirring at room temperature for 1.5 hours. Then, the mixture was concentrated under reduced pressure. The obtained residue was purified by reversed phase HPLC in the same manner as in Step 2 of Example 1 to obtain the title compound.
Yield: 218 mg (0.696 mmol), Percentage yield: 19%

### Step 3

### Synthesis of Ethyl (2S,5R)-1-[3-[[4-(4,5-Difluoro-2-methylsulfanyl-phenyl)phen oxy]methyl]benzoyl]-5-(hydroxymethyl)pyrrolidine-2-carboxyl ate

The compound obtained in Step 2 (218 mg, 3.63 mmol) was diluted with dichloromethane (10 mL), and DIPEA (505 µL, 2.78 mmol) and Intermediate 2-B (212 mg, 3.63 mmol) were added thereto, followed by stirring at room temperature for 2 hours. The reaction liquid was concentrated under reduced pressure to obtain the title compound without purification.

### Step 4

### Synthesis of Compound of Example 64

To the compound obtained in Step 3, 1,4-dioxane (9 mL) was added, and then a 1 N aqueous lithium hydroxide solution (2.44 mL) was added, followed by stirring at room temperature overnight. Further, a 1 N aqueous lithium hydroxide solution (1.74 mL) was added, followed by stirring for 3 hours. After neutralization with a 1 N aqueous trifluoroacetic acid solution and evaporation under reduced pressure, the obtained residue was purified by reversed phase HPLC in the same manner as in Step 2 of Example 1 to obtain the title compound.
Yield: 99.4 mg (0.194 mmol), Percentage yield: 28%
MS (ESI) m/z 514 [M+H]⁺

### Example 65

### (2S,5R)-1-[3-[[4-(4,5-Difluoro-2-methylsulfanyl-phenyl)phen oxy]methyl]benzoyl]-5-(methoxymethyl)pyrrolidine-2-carboxyl ic Acid

A THF solution (1.5 mL) of the compound of Example 64 (65.4 mg, 0.127 mmol) was cooled to 0°C, and then sodium hydride (22 mg, 0.51 mmol) was added thereto. After stirring for 1 hour, methyl iodide (40 µL, 0.64 mmol) was added dropwise, followed by stirring at room temperature for 3 hours. Further, methyl iodide (40 µL, 0.64 mmol) was added, followed by stirring for 2 hours. Then, water (300 µL) and methanol (300 µL) were added, followed by stirring for 30 minutes. After neutralization with a 1 N aqueous trifluoroacetic acid solution and purification by reversed phase HPLC in the same manner as in Step 2 of Example 1, the title compound was obtained.
Yield: 45.9 mg (0.0870 mmol), Percentage yield: 69%
MS (ESI) m/z 528 [M+H]⁺
¹H NMR (400 MHz, DMSO-d₆) δ 7.80 - 6.93 (m, 10H), 5.33 - 5.04 (m, 2H), 4.54 - 4.21 (m, 2H), 3.39 - 2. 89 (m, 5H), 2.39 (s, 3H), 2.36 - 1.69 (m, 4H).

### Example 66

### 2-[[3-[[4-(4,5-Difluoro-2-methylsulfanyl-phenyl)phenoxy]met hyl]benzoyl]-(isoxazol-3-ylmethyl)amino]acetic Acid

### Step 1

### Synthesis of Benzyl 2-[[3-[[4-(4,5-Difluoro-2-methylsulfanyl-phenyl)phenoxy]met hyl]benzoyl]-(isoxazol-3-ylmethyl)amino]acetate

To a solution of isoxazol-3-ylmethanamine hydrochloride (71 mg, 0.50 mmol) in acetonitrile (3 mL), potassium carbonate (173 mg, 1.25 mmol) and DMF (1 mL) were added, followed by stirring at room temperature. After the mixture was cooled to a temperature between -10°C and -15°C, benzyl 2-bromoacetate (78 µL, 0.50 mmol) diluted with acetonitrile (1 mL) was added dropwise thereto. The temperature was gradually raised to room temperature, and the mixture was stirred overnight. Insoluble matters were separated by filtration, and the filtrate was concentrated under reduced pressure. The obtained residue was diluted with dichloromethane (5 mL), and DIPEA (87 µL, 0.50 mmol) and Intermediate 2-B (81 mg, 0.20 mmol) were added thereto, followed by stirring at room temperature for 1 hour. The reaction liquid was concentrated under reduced pressure to obtain the title compound without purification.
MS (ESI) m/z 615 [M+H]⁺

### Step 2

### Synthesis of Compound of Example 66

The compound obtained in Step 1 was dissolved in 1, 4-dioxane (3 mL), and a 1 N aqueous lithium hydroxide solution (0.8 mL) was added thereto, followed by stirring at room temperature for 30 minutes. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 66.3 mg (0.126 mmol), Percentage yield: 63%
MS (ESI) m/z 525 [M+H]⁺
¹H NMR (400 MHz, DMSO-d₆) δ 8.91 (d, J = 7.7 Hz, 1H), 7.72 - 6.87 (m, 10H), 6.71 - 6.50 (m, 1H), 5.19 (s, 2H), 4.81 - 4.53 (m, 2H), 4. 12 - 3. 97 (m, 2H), 2.39 (s, 3H).

### Example 67

### 2-[[3-[[4-(4,5-Difluoro-2-methylsulfanyl-phenyl)phenoxy]met hyl]benzoyl]-[(2-methyloxazol-4-yl)methyl]amino]acetic Acid

### Step 1

### Synthesis of Ethyl 2-[[3-[[4-(4,5-Difluoro-2-methylsulfanyl-phenyl)phenoxy]met hyl]benzoyl]-[(2-methyloxazol-4-yl)methyl]amino]acetate

Glycine ethyl ester hydrochloride (209 mg, 1.50 mmol) was dissolved in a liquid mixture of THF (5 mL) and methanol (1 mL), and 2-methyloxazole-4-carbaldehyde (167 mg, 1.50 mmol) and a small amount of acetic acid were added thereto, followed by stirring at 50°C overnight. After the reaction liquid was evaporated under reduced pressure, the obtained residue was suspendered in THF (10 mL), and sodium triacetoxyborohydride (954 mg, 4.50 mmol) was added thereto, followed by stirring at room temperature for 3 hours. The reaction liquid was diluted with ethyl acetate, and the organic layer was washed with a saturated aqueous sodium hydrogen carbonate solution and saturated aqueous sodium chloride in this order, and then dried over anhydrous magnesium sulfate. After the organic solvent was removed under reduced pressure, the obtained residue was dissolved in dichloromethane (5mL), and Intermediate 2-B (101 mg, 0.250 mmol) and DIPEA (0.088 mL, 0.50 mmol) were added thereto, followed by stirring at room temperature for 1 hour. After the reaction liquid was evaporated under reduced pressure, the obtained residue was purified by reversed phase HPLC in the same manner as in Step 2 of Example 1 to obtain the target compound of Step 1.
Yield: 25.3 mg (0.0447 mmol), Percentage yield: 18%
MS (ESI) m/z 567 [M+H]⁺

### Step 2

### Synthesis of Compound of Example 67

The compound obtained in Step 1 (25.7 mg, 0.0463 mmol) was dissolved in a solvent mixture of THF (2 mL) and methanol (2 mL). Under ice cooling, a 1 N aqueous lithium hydroxide solution (0.5 mL) was added, followed by stirring at room temperature for 1 hour. After the reaction liquid was neutralized, the organic solvent was evaporated under reduced pressure, and then the obtained residue was purified by reversed phase HPLC in the same manner as in Step 2 of Example 1 to obtain the title compound.
Yield: 15.7 mg (0.0298 mmol), Percentage yield: 64%
MS (ESI) m/z 539 [M+H]⁺
¹H NMR (400 MHz, DMSO-d6) δ 12.8 (brs, 1H), 7.98 - 7. 91 (m, 1H), 7. 71 - 7.42 (m, 4H), 7.40 - 7.23 (m, 4H), 7.12 - 7.03 (m, 2H), 5.29 - 5.12 (m, 2H), 4.55 - 4.24 (m, 2H), 4.09 - 3.91 (m, 2H), 2.42 - 2.37 (m, 6H).

### Example 68

### 2-[[3-[[4-(4,5-Difluoro-2-methylsulfanyl-phenyl)phenoxy]met hyl]benzoyl]-[(3-methyl-1,2,4-oxadiazol-5-yl)methyl]amino]a cetic Acid

### Step 1

### Synthesis of Ethyl 2-[(3-Methyl-1,2,4-oxadiazol-5-yl)methylamino]acetate

Glycine ethyl ester hydrochloride (68.5 mg, 0.491 mmol) was dissolved in acetonitrile (4 mL), and potassium carbonate (204 mg, 1.47 mmol) was added thereto. After the mixture was cooled to 0°C, 5-(chloromethyl)-3-methyl-1,2,4-oxadiazole (65.0 mg, 0.491 mmol) diluted with acetonitrile (1 mL) and sodium iodide (74 mg, 0.49 mmol) were added thereto. While the temperature was being gradually returned to room temperature, the mixture was stirred for 16 hours. After insoluble matters were separated by filtration, the filtrate was concentrated under reduced pressure to obtain a crude product of Step 1.
Yield: 122 mg
MS (ESI) m/z 200 [M+H]⁺

### Step 2

### Synthesis of Ethyl 2-[[3-[[4-(4,5-Difluoro-2-methylsulfanyl-phenyl)phenoxy]met hyl]benzoyl]-[(3-methyl-1,2,4-oxadiazol-5-yl)methyl]amino]a cetate

The crude product obtained in Step 1 (61 mg, 0.25 mmol) was dissolved in dichloromethane (5 mL), and Intermediate 2-B (81.0 mg, 0.200 mmol) and DIPEA (0.044 mL, 0.25 mmol) were added thereto, followed by stirring at room temperature overnight. After the reaction liquid was evaporated under reduced pressure, the obtained residue was diluted with ethyl acetate. The organic layer was washed with a 0.5 N aqueous hydrochloric acid solution, a saturated aqueous sodium hydrogen carbonate solution, and saturated aqueous sodium chloride in this order, and then dried over anhydrous magnesium sulfate. The organic solvent was removed under reduced pressure to obtain a crude product of Step 2.
Yield: 121 mg
MS (ESI) m/z 568 [M+H]⁺

### Step 3

### Synthesis of Compound of Example 68

The crude product obtained in Step 2 (121 mg) was dissolved in a solvent mixture of THF (2 mL) and methanol (2 mL), and a 1 N aqueous lithium hydroxide solution (1 mL) was added thereto under ice cooling, followed by stirring at room temperature for 1 hour. After the reaction liquid was neutralized, the organic solvent was evaporated under reduced pressure, and then the obtained residue was purified by reversed phase HPLC in the same manner as in Step 2 of Example 1 to obtain the title compound.
Yield: 84.5 mg (0.157 mmol), Percentage yield: 79% (from Step 1)
MS (ESI) m/z 540 [M+H]⁺
¹H NMR (400 MHz, DMSO-d₆) δ 13. 1 (brs, 1H), 7 . 64 - 7.56 (m, 1H), 7.55 - 7.46 (m, 2H), 7.41 - 7.25 (m, 5H), 7.13 - 7.02 (m, 2H), 5.19 (s, 2H), 4.97 - 4.74 (m, 2H), 4.24 - 4.13 (m, 2H), 2.39 (s, 3H), 2.37 - 2.32 (m, 3H).

### Example 69

### 2-[[3-[[4-(4,5-Difluoro-2-methylsulfanyl-phenyl)phenoxy]met hyl]benzoyl]-[(5-methyl-1,2,4-oxadiazol-3-yl)methyl]amino]a cetic Acid

### Step 1

### Synthesis of Ethyl 2-[[(5-Methyl-1,2,4-oxadiazol-3-yl)methylamino]acetate

Glycine ethyl ester hydrochloride (105 mg, 0.754 mmol) was dissolved in acetonitrile (10 mL), and potassium carbonate (313 mg, 2.26 mmol) was added thereto. After the mixture was cooled to 0°C, 3-(chloromethyl)-5-methyl-1,2,4-oxadiazole (100 mg, 0.754 mmol) diluted with acetonitrile (1 mL) and sodium iodide (113 mg, 0.754 mmol) were added thereto. While the temperature was being gradually returned to room temperature, the mixture was stirred overnight. After insoluble matters were separated by filtration, the filtrate was concentrated under reduced pressure to obtain a crude product of Step 1.
Yield: 272 mg
MS (ESI) m/z 200 [M+H]⁺

### Step 2

### Synthesis of Ethyl 2-[[3-[[4-(4,5-Difluoro-2-methylsulfanyl-phenyl)phenoxy]met hyl]benzoyl]-[(5-methyl-1,2,4-oxadiazol-3-yl)methyl]amino]a cetate

Intermediate 2-B (81.0 mg, 0.200 mmol) was dissolved in dichloromethane (3 mL), and the crude product obtained in Step 1 (90.6 mg, 0.251 mmol) and DIPEA (0.0440 mL, 0.250 mmol) were added thereto, followed by stirring at room temperature for 2 hours. After the reaction liquid was evaporated under reduced pressure, the obtained residue was diluted with ethyl acetate. The organic layer was washed with a 0.5 N aqueous hydrochloric acid solution, a saturated aqueous sodium hydrogen carbonate solution, and saturated aqueous sodium chloride in this order, and then dried over anhydrous magnesium sulfate. The organic solvent was removed under reduced pressure to obtain a crude product of Step 2.
Yield: 121 mg
MS (ESI) m/z 568 [M+H]⁺

### Step 3

### Synthesis of Compound of Example 69

The crude product obtained in Step 2 (121 mg) was dissolved in a solvent mixture of THF (2 mL) and methanol (2 mL) . Under ice cooling, a 1 N aqueous lithium hydroxide solution (1 mL) was added thereto, followed by stirring at room temperature for 1 hour. After the reaction liquid was neutralized, the organic solvent was evaporated under reduced pressure, and then the obtained residue was purified by reversed phase HPLC in the same manner as in Step 2 of Example 1 to obtain the title compound.
Yield: 78.7 mg (0.146 mmol), Percentage yield: 73% (from Step 2)
MS (ESI) m/z 540 [M+H]⁺
¹H NMR (400 MHz, DMSO-d₆) δ 13.3 - 12.7 (m, 1H), 7.62 - 7.42 (m, 3H), 7.40 - 7.24 (m, 5H), 7.11 - 7.03 (m, 2H), 5.24 - 5.16 (m, 2H), 4.84 - 4.51 (m, 2H), 4.19 - 4.00 (m, 2H), 2.62 - 2.57 (m, 3H), 2.39 (s, 3H).

### Example 70

### 2-[[3-[[4-(4,5-Difluoro-2-methylsulfanyl-phenyl)phenoxy]met hyl]benzoyl]-[2-(1,2,4-triazol-1-yl)ethyl]amino]acetic Acid

### Step 1

### Synthesis of 2-[2-(1,2,4-Triazol-1-yl)ethyl]isoindoline-1,3-dione

A DMF solution (6 mL) of 1H-1,2,4-triazole (1.00 g, 14.5 mmol) was cooled to 0°C, and a 50% aqueous sodium hydroxide solution (1.27 mL) was added thereto, followed by stirring at room temperature for 3 hours. A DMF solution (4 mL) of 2-(2-bromoethyl)isoindoline-1,3-dione (3.88 g, 15.3 mmol) was added, followed by stirring at 60°C overnight. After stirring at room temperature for further two days, ethyl acetate and water were added. The organic layer was extracted with ethyl acetate, washed with saturated aqueous sodium chloride, dried over anhydrous magnesium sulfate, and concentrated under reduced pressure. The residue was purified by silica gel column chromatography (hexane/ethyl acetate) to obtain the title compound.
Yield: 324 mg (1.34 mmol), Percentage yield: 9%

### Step 2

### Synthesis of 2-(1,2,4-Triazol-1-yl)ethanamine Trifluoroacetate

To an ethanol solution (3.7 mL) of the compound obtained in Step 1 (324 mg, 1.34 mmol), hydrazine monohydrate (194 µL, 4.01 mmol) was added, followed by stirring at 70°C for 1 hour. Ethanol (3.7 mL) was added, followed by stirring at 70°C for 1hour. Then, the temperature was returned to room temperature, and insoluble matters were separated by filtration using ethanol. The filtrate was concentrated under reduced pressure, and the obtained residue was purified by reversed phase HPLC in the same manner as in Step 2 of Example 1 to obtain the title compound.
Yield: 82.8 mg (0.366 mmol), Percentage yield: 27%

### Step 3

### Synthesis of Benzyl 2-[[3-[[4-(4,5-Difluoro-2-methylsulfanyl-phenyl)phenoxy]met hyl]benzoyl]-[2-(1,2,4-triazol-1-yl)ethyl]amino]acetate

To a solution of the compound obtained in Step 2 (82.8 mg, 0.366 mmol) in acetonitrile (3mL), potassium carbonate (126 mg, 0.915 mmol) was added. After the mixture was cooled to a temperature between -10°C and -15°C, benzyl 2-bromoacetate (57 µL, 0.37 mmol) diluted with acetonitrile (1 mL) was added dropwise thereto. The temperature was gradually raised to room temperature, and the mixture was stirred overnight. Insoluble matters were separated by filtration, and the filtrate was concentrated under reduced pressure. The obtained residue was diluted with dichloromethane (3 mL), and DIPEA (64 µL, 0.37 mmol) and Intermediate 2-B (81 mg, 0.20 mmol) were added thereto, followed by stirring at room temperature for 2 hours. The reaction liquid was concentrated under reduced pressure, and the obtained residue was purified by reversed phase HPLC in the same manner as in Step 2 of Example 1 to obtain the title compound.
Yield: 25 mg (0.046 mmol), Percentage yield: 23%
MS (ESI) m/z 629 [M+H]⁺

### Step 4

### Synthesis of Compound of Example 70

The compound obtained in Step 3 was dissolved in 1,4-dioxane (0.5 mL), and a 1 N aqueous lithium hydroxide solution (0.074 mL) was added thereto, followed by stirring at room temperature for 1 hour and 30 minutes. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 7.8 mg (0.015 mmol), Percentage yield: 31%
MS (ESI) m/z 539 [M+H]⁺
¹H NMR (400 MHz, DMSO-d₆) δ 8.54 (d, J = 59.2 Hz, 1H), 7.96 (d, J = 39.5 Hz, 1H), 7.58 - 6.92 (m, 10H), 5.16 (s, 2H), 4.54 - 4.27 (m, 2H), 4.17 - 3.60 (m, 4H), 2.39 (s, 3H).

### Example 71

### 2-[[3-[[4-(4,5-Difluoro-2-methylsulfanyl-phenyl)phenoxy]met hyl]benzoyl]-(thiazol-4-ylmethyl)amino]acetic Acid

### Step 1

### Synthesis of Ethyl 2-[[3-[[4-(4,5-Difluoro-2-methylsulfanyl-phenyl)phenoxy]met hyl]benzoyl]-(thiazol-4-ylmethyl)amino]acetate

To a solution of thiazol-4-ylmethanamine hydrochloride (100 mg, 0.664 mmol) in acetonitrile (3mL), potassium carbonate (229 mg, 1.66 mmol) and DMF (2 mL) were added. After the mixture was cooled to a temperature between -10°C and -15°C, ethyl 2-bromoacetate (73 µL, 0.66 mmol) diluted with acetonitrile (1 mL) was added dropwise thereto. The temperature was gradually raised to room temperature, and the mixture was stirred overnight. Insoluble matters were separated by filtration, and the filtrate was concentrated under reduced pressure. A half of the obtained residue was diluted with dichloromethane (2 mL), and DIPEA (61 µL, 0.35 mmol) and Intermediate 2-B (61 mg, 0.15 mmol) were added thereto, followed by stirring at room temperature for 30 minutes. The reaction liquid was concentrated under reduced pressure, and the obtained residue was purified by silica gel column chromatography (hexane/ethyl acetate) to obtain the title compound.
MS (ESI) m/z 569 [M+H]⁺

### Step 2

### Synthesis of Compound of Example 71

The compound obtained in Step 1 was dissolved in 1, 4-dioxane (1 mL), and a 1 N aqueous lithium hydroxide solution (0.139 mL) was added thereto, followed by stirring at room temperature overnight. The reaction liquid was neutralized with a 1 N aqueous trifluoroacetic acid solution, and then purified by reversed phase HPLC in the same manner as in Step 2 of Example 1 to obtain the title compound.
Yield: 9.4 mg (0.018 mmol), Percentage yield: 12%
MS (ESI) m/z 541 [M+H]⁺
¹H NMR (400 MHz, DMSO-d₆) δ 9.18 - 9.07 (m, 1H), 7.71 - 7.20 (m, 9H), 7.14 - 7.00 (m, 2H), 5.28 - 5.12 (m, 2H), 4.86 - 4.55 (m, 2H), 4.11 - 3.97 (m, 2H), 2.39 (s, 3H).

### Example 72

### (2S)-5-(Cyanomethyl)-1-[3-[[4-(4,5-difluoro-2-methylsulfany 1-phenyl)phenoxy]methyl]benzoyl]pyrrolidine-2-carboxylic Acid

Intermediate 2-B (155 mg, 0.380 mmol) was dissolved in THF (5 mL), and ethyl (2S,5S)-5-(cyanomethyl)pyrrolidine-2-carboxylate (100 mg, 0.46 mmol) and triethylamine (0.11 mL, 0.76 mmol) were added thereto, followed by stirring at room temperature for 2 hours. Then, a 1N aqueous sodium hydroxide solution (5 mL) and methanol (5 mL) were added thereto, followed by stirring overnight. After neutralization with 2N hydrochloric acid, the solvent was evaporated under reduced pressure. To the obtained residue, a 4 N hydrochloric acid/1, 4-dioxane solution was added at 0°C, followed by stirring at the same temperature for 2 hours. The solvent was evaporated under reduced pressure, and the obtained residue was purified by reversed phase HPLC in the same manner as in Step 2 of Example 1 to obtain the title compound.
Yield: 35 mg (0.074 mmol), Percentage yield: 19%
MS (ESI) m/z 523 [M+H]⁺

### Example 73

### 2-[[3-[[4-(4,5-Difluoro-2-methylsulfanyl-phenyl)phenoxy]met hyl]benzoyl]-[(5-methyl-1,3,4-oxadiazol-2-yl)methyl]amino]a cetic Acid

### Step 1

### Synthesis of Benzyl 2-[(5-Methyl-1,3,4-oxadiazol-2-yl)methylamino]acetate

(5-Methyl-1,3,4-oxadiazol-2-yl)methanamine (170 mg, 1.50 mmol) was dissolved in acetonitrile (12 mL), and potassium carbonate (311 mg, 2.25 mmol) was added thereto. After the mixture was cooled to -10°C, benzyl 2-bromoacetate (0.259 mL, 1.65 mmol) diluted with acetonitrile (3 mL) was added thereto. While the temperature was being gradually returned to room temperature, the mixture was stirred overnight. After insoluble matters were separated by filtration, the filtrate was concentrated under reduced pressure to obtain a crude product.
Yield: 372 mg
MS (ESI) m/z 262 [M+H]⁺

### Step 2

### Synthesis of Benzyl 2-[[3-[[4-(4,5-Difluoro-2-methylsulfanyl-phenyl)phenoxy]met hyl]benzoyl]-[(5-methyl-1,3,4-oxadiazol-2-yl)methyl]amino]a cetate

A crude product of Step 1 (124 mg, 0.500 mmol) was dissolved in dichloromethane (3 mL), and Intermediate 2-B (94.8 mg, 0.240 mmol) and DIPEA (0.044 mL, 0.25 mmol) were added thereto, followed by stirring at room temperature overnight. After the reaction liquid was evaporated under reduced pressure, the obtained residue was purified by reversed phase HPLC in the same manner as in Step 2 of Example 1 to obtain the title compound.
Yield: 54.6 mg (0.1033 mmol), Percentage yield: 43%
MS (ESI) m/z 630 [M+H]⁺

### Step 3

### Synthesis of Compound of Example 73

The compound obtained in Step 2 (54.6 mg, 0.103 mmol) was dissolved in a solvent mixture of THF (2 mL) and methanol (2 mL). Under ice cooling, a 1 N aqueous lithium hydroxide solution (0.5 mL) was added, followed by stirring at room temperature for 1 hour. After the reaction liquid was neutralized, the organic solvent was evaporated under reduced pressure, and then the obtained residue was purified by reversed phase HPLC in the same manner as in Step 2 of Example 1 to obtain the title compound.
Yield: 42.3 mg (0.0799 mmol), Percentage yield: 78%
MS (ESI) m/z 540 [M+H]⁺
¹H NMR (400 MHz, DMSO-d₆) δ 7.64 - 7.24 (m, 8H), 7.12 - 7.05 (m, 2H), 5.26 - 5.15 (m, 2H), 4.97 - 4.64 (m, 2H), 4.20 - 4.05 (m, 2H), 2.53 - 2.44 (m, 3H), 2.42 - 2.37 (m, 3H).

### Example 74

### 2-[[3-[[4-(4,5-Difluoro-2-methylsulfanyl-phenyl)phenoxy]met hyl]benzoyl]-[(1-methylimidazol-4-yl)methyl]amino]acetic Acid

### Step 1

### Synthesis of Benzyl 2-[(1-Methylimidazol-4-yl)methylamino]acetate

1-Methylimidazol-4-ylmethanamine (250 mg, 2.25 mmol) was diluted with acetonitrile (10 mL), and potassium carbonate (466 mg, 3.37 mmol) was added thereto. After the mixture was cooled to 0°C, benzyl 2-bromoacetate (0.390 mL, 2.47 mmol) diluted with acetonitrile (1 mL) was added dropwise thereto. While the temperature was being gradually returned to room temperature, the mixture was stirred for 14 hours. After insoluble matters were separated by filtration, the filtrate was concentrated under reduced pressure to obtain a crude product.
Yield: 350 mg
MS (ESI) m/z 260 [M+H]⁺

### Step 2

### Synthesis of Compound of Example 74

Intermediate 2-B (75.0 mg, 0.186 mmol) was dissolved by adding dichloromethane (2 mL) . To this solution, the compound obtained in Step 1 (96.5 mg, 0.372 mmol) and DIPEA (0.081 mL, 0.465 mmol) were added, followed by stirring at room temperature for 16 hours. The reaction liquid was concentrated under reduced pressure, and then the obtained residue was dissolved in a solvent mixture of THF (1 mL) and methanol (1 mL) . Under ice cooling, a 1 N aqueous sodium hydroxide solution (0.48 mL) was added, followed by stirring at room temperature for 3 hours. After the reaction liquid was neutralized, the organic solvent was evaporated under reduced pressure, and then the obtained residue was purified by reversed phase HPLC using ODS in the same manner as in Step 2 of Example 1 to obtain the title compound.
Yield: 26.7 mg (0.0497 mmol), Percentage yield: 30%
MS (ESI) m/z 538 [M+H]⁺
¹H NMR (400 MHz, DMSO-d₆) δ 9.28 - 8.62 (m, 1H), 7.77 - 7.30 (m, 9H), 7.14 (d, J = 7.9 Hz, 2H), 5.30 - 5.20 (m, 2H), 4.80 - 4.53 (m, 2H), 4.21 - 4.02 (m, 2H), 3.96 - 3.80 (m, 3H), 2.46 (s, 3H).

### Example 75

### 2-[2-Cyanoethyl-[3-[[4-(4,5-difluorobenzofuran-7-yl)phenoxy ]methyl]benzoyl]amino]acetic Acid

To Intermediate 1-B (50 mg, 0.13 mmol) and N- (2-cyanoethyl) glycine (80 mg, 0. 63 mmol), a 1 N aqueous sodium hydroxide solution (2 mL) and dichloromethane (2 mL) were added, followed by stirring overnight. After the solvent was evaporated under reduced pressure, the obtained residue was purified by reversed phase HPLC in the same manner as in Step 2 of Example 1 to obtain the title compound.
Yield: 28 mg (0.057 mmol), Percentage yield: 45%
MS (ESI) m/z 491 [M+H]⁺

### Example 76

### (2S,5S) or (2S,5R)-5-Carbamoyl-1-[3-[[4-(4,5-difluorobenzofuran-7-yl)p henoxy]methyl]benzoyl]pyrrolidine-2-carboxylic Acid

The title compound was obtained as an isomer in the step of Example 4.
Yield: 4.40 mg (0.00845 mmol), Percentage yield: 6.8%
MS (ESI) m/z 521 [M+H]+
¹H NMR (400 MHz, DMSO-d₆) δ 13.5 (s, 1H), 8.19 (d, J = 2.2 Hz, 1H), 8.11 - 7.87 (m, 1H), 7.83 (d, J = 8.4 Hz, 2H), 7.78 - 7.67 (m, 1H), 7.67 - 7.54 (m, 3H), 7.54 - 7.44 (m, 1H), 7.42 - 7.36 (m, 1H), 7.24 (d, J = 2.2 Hz, 1H), 7.19 (d, J = 8.4 Hz, 2H), 5.21 (s, 2H), 4.62 - 4.20 (m, 2H), 2.45 - 2.19 (m, 2H), 2.05 - 1.79 (m, 2H).

### Example 77

### 2-[[3-[[4-(4,5-Difluorobenzofuran-7-yl)phenoxy]methyl]benzo yl]-(3-methylsulfonylpropyl)amino]acetic Acid

### Step 1

### Synthesis of Ethyl 2-[[3-[[4-(4,5-Difluorobenzofuran-7-yl)phenoxy]methyl]benzo yl]-(3-methylsulfonylpropyl)amino]acetate

To a solution of 3-methylsulfonylpropane-1-amine (183 mg, 1.00 mmol) in acetonitrile (8mL), potassium carbonate (138 mg, 1.00 mmol) was added. After the mixture was cooled to a temperature between -10°C and -15°C, ethyl 2-bromoacetate (111 µL, 1.00 mmol) diluted with acetonitrile (2 mL) was added dropwise thereto, followed by stirring for 2.5 hours. The temperature was returned to room temperature, and the mixture was stirred for 5.5 hours. Then, insoluble matters were separated by filtration. The filtrate was concentrated under reduced pressure. A half of the obtained residue was diluted with dichloromethane (4 mL), and DIPEA (87 µL, 0.50 mmol) and Intermediate 1-B (80 mg, 0.20 mmol) were added thereto, followed by stirring at room temperature overnight. To the reaction liquid, water was added, followed by extraction with dichloromethane. The organic layer was washed with a saturated aqueous sodium hydrogen carbonate solution and saturated aqueous sodium chloride, and dried over anhydrous magnesium sulfate. The solvent was evaporated under reduced pressure to obtain the title compound without purification.
MS (ESI) m/z 586 [M+H]⁺

### Step 2

### Synthesis of Compound of Example 77

The compound obtained in Step 1 was dissolved in 1, 4-dioxane (3 mL), and a 1 N aqueous lithium hydroxide solution (0.6 mL) was added thereto, followed by stirring at room temperature for 1.5 hours. The reaction liquid was neutralized with a 1 N aqueous trifluoroacetic acid solution, and then evaporated under reduced pressure. The obtained residue was purified by reversed phase HPLC in the same manner as in Step 2 of Example 1 to obtain the title compound.
Yield: 65.2 mg (0.0117 mmol), Percentage yield: 58%
MS (ESI) m/z 558 [M+H]⁺
¹H NMR (400 MHz, DMSO-d₆) δ 8.19 (d, J = 2.3 Hz, 1H), 7.83 (d, J = 8.8 Hz, 2H), 7.70 - 7.38 (m, 4H), 7.40 - 7.07 (m, 4H), 5.35 - 5.12 (m, 2H), 4.16 - 3. 94 (m, 2H), 3. 62 - 2.79 (m, 7H), 2.11 - 1.81 (m, 2H).

### Example 78

### (2S,3S)-1-[3-[[4-(4,5-Difluorobenzofuran-7-yl)phenoxy]methy 1]benzoyl]-3-methoxy-pyrrolidine-2-carboxylic Acid

A THF solution (2 mL) of methyl (2S,3S)-1-[3-[[4-(4,5-difluorobenzofuran-7-yl)phenoxy]methy 1]benzoyl]-3-hydroxy-pyrrolidine-2-carboxylate (82 mg, 0.13 mmol) obtained in Step 1 of Example 2 was cooled to 0°C, and sodium hydride (11 mg, 0.24 mmol) was added thereto, followed by stirring at room temperature for 30 minutes. Methyl iodide (45 µL, 0.49 mmol) was added dropwise, followed by stirring at room temperature for 4 hours. Further, sodium hydride (11 mg, 0.24 mmol) and methyl iodide (45 µL, 0.49 mmol) were added thereto, followed by stirring for 1.5 hours. Water was added to the reaction liquid. The obtained residue was purified by reversed phase HPLC in the same manner as in Step 2 of Example 1 to obtain the title compound.
Yield: 23.9 mg (0.0471 mmol), Percentage yield: 29%
MS (ESI) m/z 508 [M+H]⁺
¹H NMR (400 MHz, DMSO-d₆) δ 8.19 (d, J = 2.2 Hz, 1H), 7.82 (d, J = 8.8 Hz, 2H), 7.65 - 7.43 (m, 5H), 7.32 - 7.14 (m, 3H), 5.32 - 5.20 (m, 2H), 4.48 - 4.21 (m, 1H), 4.04 - 3.98 (m, 1H), 3.69 - 3.46 (m, 2H), 3.36 - 3.20 (m, 3H), 2.09 - 1.90 (m, 2H).

### Example 79

### 2-[(3-Amino-3-oxo-propyl)-[3-[[4-(4,5-difluorobenzofuran-7-yl)phenoxy]methyl]benzoyl]amino]acetic Acid

### Step 1

### Synthesis of Benzyl 2-[(3-Amino-3-oxo-propyl)-[3-[[4-(4,5-difluorobenzofuran-7-yl)phenoxy]methyl]benzoyl]amino]acetate

3-Aminopropanamide (62.3 mg, 0.500 mmol) was diluted with acetonitrile (4 mL), and potassium carbonate (104 mg, 0.750 mmol) was added thereto. After the mixture was cooled to -15°C, benzyl 2-bromoacetate (0.086 mL, 0.550 mmol) diluted with acetonitrile (1 mL) was added dropwise thereto, followed by stirring overnight. After insoluble matters were separated by filtration, the filtrate was concentrated under reduced pressure to obtain a residue.
MS (ESI) m/z 237 [M+H]⁺

The obtained residue was diluted with dichloromethane (3 mL). Under ice cooling, DIPEA (0.044 mL, 0.25 mmol) and Intermediate 1-B (100 mg, 0.250 mmol) were added thereto, followed by stirring at room temperature for 1 hour. The reaction solution was diluted by adding dichloromethane. The organic layer was washed with 1 N hydrochloric acid, a saturated aqueous sodium hydrogen carbonate solution, and saturated aqueous sodium chloride in this order, and then dried over anhydrous magnesium sulfate. The organic solvent was concentrated under reduced pressure to obtain a crude product.
Yield: 200 mg
MS (ESI) m/z 599 [M+H]⁺

### Step 2

### Synthesis of Compound of Example 79

The crude product obtained in Step 1 was dissolved in a solvent mixture of THF (2 mL) and methanol (2 mL). Under ice cooling, a 1N aqueous lithium hydroxide solution (1.0 mL) was added thereto, followed by stirring at room temperature for 1 hour. After the reaction liquid was neutralized, the organic solvent was evaporated under reduced pressure, and then the obtained residue was purified by reversed phase HPLC in the same manner as in Step 2 of Example 1 to obtain the title compound.
Yield: 92.0 mg (0.181 mmol), Percentage yield: 72% (Step 1 was included)
MS (ESI) m/z 509 [M+H]⁺
¹H NMR (400 MHz, DMSO-d₆) δ 12.7 (s, 1H), 8.19 (d, J = 2.3 Hz, 1H), 7.87 - 7.78 (m, 2H), 7.67 - 7.12 (m, 9H), 6.93 - 6.81 (m, 1H), 5.31 - 5.16 (m, 2H), 4.19 - 3.93 (m, 2H), 3.64 - 3.40 (m, 2H), 2.49 - 2.33 (m, 2H)

### Example 80

### 2-[[3-[[4-(4,5-Difluorobenzofuran-7-yl)phenoxy]methyl]benzo yl]-[(1S)-2-methoxy-1-methyl-ethyl]amino]acetic Acid

N-Boc-L-Alaninol (250 mg, 1.43 mmol) was dissolved in DMF (1 mL), and silver oxide (660 mg, 2.86 mmol) and methyl iodide (0.890 mL, 14.3 mmol) were added thereto, followed by stirring at room temperature overnight. After insoluble matters were separated by filtration, the mixture was diluted with ethyl acetate, then washed with water and saturated aqueous sodium chloride, and dried over anhydrous magnesium sulfate. After the solvent was evaporated under reduced pressure, a 4 N hydrochloric acid/1, 4-dioxane solution (5 mL) was added to the obtained residue, followed by stirring at room temperature for 2 hours. After the solvent was evaporated under reduced pressure, the obtained residue was washed with ethyl acetate. A half of the obtained residue was dissolved in acetonitrile (15 mL), and then potassium carbonate (198 mg, 1.43 mmol) was added thereto, and benzyl 2-bromoacetate (0. 112 ml, 0.715 mmol) was slowly added at -20°C. After the mixture was stirred for 1 hour at the same temperature, the temperature was returned to room temperature, followed by stirring overnight. After insoluble matters were separated by filtration, the solvent was evaporated under reduced pressure. Then, THF (5 mL), Intermediate 1-B (100 mg, 0.250 mmol), and triethylamine (0.07 mL, 0.5 mmol) were added to the obtained residue, followed by stirring at room temperature for 2 hours. Then, a 1 N aqueous sodium hydroxide solution (5 mL) and methanol (5 mL) were added, followed by stirring overnight. The solvent was evaporated under reduced pressure. The obtained residue was diluted with ethyl acetate, then washed with 1 N hydrochloric acid and saturated aqueous sodium chloride, and dried over anhydrous magnesium sulfate. The solvent was evaporated under reduced pressure, and the obtained residue was purified by reversed phase HPLC in the same manner as in Step 2 of Example 1 to obtain the title compound.
Yield: 37 mg (0.073 mmol), Percentage yield: 10%
MS (ESI) m/z 510 [M+H]⁺

### Example 81

### 2-[(1-Acetyl-4-piperidyl)methyl-[3-[[4-(4,5-difluorobenzofu ran-7-yl)phenoxy]methyl]benzoyl]amino]acetic Acid

### Step 1

### Synthesis of 2-[(1-tert-Butoxycarbonyl -4-piperidyl)methyl-[3-[[4-(4,5-difluorobenzofuran-7-yl)phe noxy]methyl]benzoyl]amino]acetic Acid

1-N-BOC-4-(Aminomethyl)piperidine (550 mg, 2.57 mmol) was dissolved in acetonitrile (25 mL), and then potassium carbonate (354 mg, 2.57 mmol) was added thereto, and benzyl 2-bromoacetate (0.400 m, 2.57 mmol) was slowly added at -20°C. After the mixture was stirred at the same temperature for 1 hour, the temperature was returned to room temperature, followed by stirring overnight. After insoluble matters were separated by filtration, the solvent was evaporated under reduced pressure. Then, THF (5 mL), Intermediate 1-B (300 mg, 0.750 mmol), and triethylamine (0.21 mL, 1.5 mmol) were added to a half of the obtained residue, followed by stirring at room temperature for 2 hours. Then, a 1 N aqueous sodium hydroxide solution (5 mL) and methanol (5 mL) were added, followed by stirring overnight. The solvent was evaporated under reduced pressure. The obtained residue was diluted with ethyl acetate, then washed with 1 N hydrochloric acid and saturated aqueous sodium chloride, and dried over anhydrous magnesium sulfate. The solvent was evaporated under reduced pressure, and the obtained residue was purified by reversed phase HPLC in the same manner as in Step 2 of Example 1 to obtain the title compound.
Yield: 210 mg (0.33 mmol), Percentage yield: 25%

### Step 2

### Synthesis of 2-[[3-[[4-(4,5-Difluorobenzofuran-7-yl)phenoxy]methyl]benzo yl]-(4-piperidylmethyl)amino]acetic Acid

To the compound obtained in Step 1 (210 mg, 0.33 mmol) at 0°C, a 4 N hydrochloric acid/1, 4-dioxane solution (3 mL) was added, followed by stirring at the same temperature for 1 hour. The solvent was evaporated under reduced pressure, and the obtained residue was purified by reversed phase HPLC in the same manner as in Step 2 of Example 1 to obtain the title compound. Yield: 185 mg (0.290 mmol), Percentage yield: 87%

### Step 3

### Synthesis of Compound of Example 81

To the compound obtained in Step 2 (18 mg, 0.029 mmol), dichloromethane (2 mL), acetyl chloride (3.4 mg, 0.044 mmol), and triethylamine (0.014 mL, 0.10 mmol) were added, followed by stirring at room temperature for 1 hour. After the solvent was evaporated under reduced pressure, the obtained residue was purified by reversed phase HPLC in the same manner as in Step 2 of Example 1 to obtain the title compound.
Yield: 11 mg (0.019 mmol), Percentage yield: 45%
MS (ESI) m/z 577 [M+H]⁺

### Example 82

### 2-[[3-[[4-(4,5-Difluorobenzofuran-7-yl)phenoxy]methyl]benzo yl]-(2-oxopyrrolidin-3-yl)amino]acetic Acid

### Step 1

### Synthesis of Benzyl 2-[(2-Oxopyrrolidin-3-yl)amino]acetate

3-Aminopyrrolidin-2-one (50.1 mg, 0.500 mmol) was diluted with acetonitrile (4 mL), and potassium carbonate (104 mg, 0.750 mmol) was added thereto. After the mixture was cooled to -15°C, benzyl 2-bromoacetate (0.086 mL, 0.55 mmol) diluted with acetonitrile (1 mL) was added dropwise thereto, followed by stirring overnight. After insoluble matters were separated by filtration, the filtrate was concentrated under reduced pressure to obtain a residue.
MS (ESI) m/z 249 [M+H]⁺

### Step 2

### Synthesis of Benzyl 2-[[3-[[4-(4,5-Difluorobenzofuran-7-yl)phenoxy]methyl]benzo yl]-(2-oxopyrrolidin-3-yl)amino]acetate

The obtained residue was diluted with dichloromethane (3 mL), and DIPEA (0.044 mL, 0.25 mmol) and Intermediate 1-B (100 mg, 0.250 mmol) were added thereto under ice cooling, followed by stirring at room temperature for 1 hour. The reaction solution was diluted by adding dichloromethane. The organic layer was washed with a 1 N aqueous hydrochloric acid solution, a saturated aqueous sodium hydrogen carbonate solution, and saturated aqueous sodium chloride in this order, and then dried over anhydrous magnesium sulfate. The organic solvent was concentrated under reduced pressure to obtain a crude product.
Yield: 193 mg
MS (ESI) m/z 611 [M+H]⁺

### Step 3

### Synthesis of Compound of Example 82

The crude product obtained in Step 1 was dissolved in a solvent mixture of THF (2 mL) and methanol (2 mL), and a 1 N aqueous lithium hydroxide solution (1.0 mL) was added thereto under ice cooling, followed by stirring at room temperature for 1.5 hours. After the reaction liquid was neutralized, the organic solvent was evaporated under reduced pressure, and then the obtained residue was purified by reversed phase HPLC in the same manner as in Step 2 of Example 1 to obtain the title compound.
Yield: 30.8 mg (0.0592 mmol), Percentage yield: 24%
MS (ESI) m/z 521 [M+H]⁺
¹H NMR (400 MHz, DMSO-d₆) δ 8.19 (d, J = 2.3 Hz, 1H), 8.04 - 7.86 (m, 1H), 7.86 - 7.79 (m, 2H), 7.67 - 7.38 (m, 5H), 7.23 (d, J = 2.3 Hz, 1H), 7.21 - 7.15 (m, 2H), 5.32 - 5.21 (m, 2H), 4.45 - 4.33 (m, 1H), 4.30 - 4.18 (m, 1H), 4.06 - 3.86 (m, 1H), 3.18 - 2.94 (m, 2H), 2.33 - 2.08 (m, 2H).

Tables 2-1 to 2-11 show structural formulae and values of physical properties of the compounds obtained in Examples described above.

**Table 2-1**

| Example No | moistructure | Salt | Name of Compound (IUPAC) | Analysis data |
|---|---|---|---|---|
| 1 | 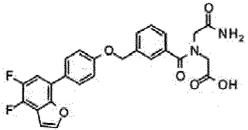 | - | **2-[(2-amino-2-oxo-ethyl)-[3-[[4-(4,5-difluorobenzofuran-7-yl)phenoxy]methyl]benzoyl]amin o]acetic acid** | **MS (ESI) m/z 495 (M+H)+ 1H NMR (400 MHz, DMSO-d6) δ 13.19 - 12.77 (m, 1H), 8.19 (d, J = 2.3 Hz, 1H), 7.66 - 7.80 (m, 2H), 7.66 - 7.46 (m, 5H), 7.37 - 7.26 (m, 2H), 7.24 (d, J = 2.3 Hz, 1H), 7.22 - 7.15 (m, 2H), 5,22 (s, 2H), 4.13 - 3.87 (m, 4H).** |
| 2 | 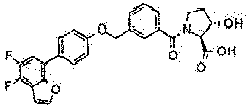 | - | **(2S,3S)-1-[3-[[4-(4.5-difluorobenzofuran-7-yl)phenoxy]methyl]benzoyl]-3-hydroxy-pyrrolidine-2-carboxykic acid** | **MS (ESI) m/z 494 (M+H)+** |
| 3 | 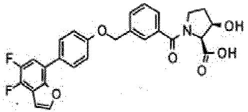 | - | **(2S,3R)-1-[3-[[4-(4,5-difluorobenzofuran-7-yl)phenoxy]methyl]benzoyl]-3-hydroxy-pyrrolidine-2-carboxylic acid** | **MS (ESI) m/z 494 (M+H)+ 1H NMR (400 MHz, DMSO-d6) δ 12.78 - 11.96 (m, 1H), 8.19 (d, J = 2.2 Hz, 1H), 7.87 - 7.79 (m, 2H), 7.67 - 7.63 (m, 1H), 7.63 - 7.57 (m, 2H), 7.54 - 7.47 (m, 2H), 7.23 (d, J = 2.2 Hz, 1H). 7.22 - 7 15 (m. 2H), 5.29 - 5.18 (m, 2H), 4.56 - 4.38 (m, 2H), 3.72 - 3.58 (m, 1H), 3.48 - 3.43 (m. 1H), 1.99 - 1.73 (m, 2H).** |
| 4 | 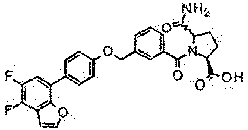 | - | **(2S,5R) or (2S,5S)-5-carbamoyl-1-[3-[[4-(4,5-difluorobenzofuran-7-yl)phenoxy]methyl]benzoyl]pyrrol idine-2-carboxylic acid** | **MS (ESI) m/z 521 (M+H)+ 1H NMR (400 MHz, DMSO-d6) δ 12.67 (bs, 1H), 8.19 (d, J = 22 Kz, 1H), 7-89 - 7.76 (m, 2H), 7.69 - 7.57 (m, 2H), 7.57 - 7.49 (m, 1H), 7.45 (m. 2H). 7.34- 7.27 (m, 1H). 7.24 (d, J = 2.2 Hz, 1H), 7.22 - 7.15 (m, 2H), 7.11 - 6.87 (m, 1M), 5.21 (m, 2H), 4.70 - 4.21 (m, 2H). 2.47 1.77 (m, 4H).** |
| 5 | 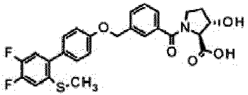 | - | **(2S, 3S)-1-[3-[[4-(4,5-difluoro-2-methylsulfanyl phenyl)phenoxy]methyl]benzoyl]-3-hydroxy-pyrrolidine-2-carboxylic acid** | **MS (ESI) m/z 500 (M-+H)+ 1H NMR (400 MHz, DMSO-d6) δ 7,43 - 7.43 (rg 4H). 7.40-7.25 (m, 4H), 7.12 - 7.05 (m, 2H), 5.25 - 5.15 (m, 2H), 4.34 - 4.09 (m, 2H), 3.66 - 3.55 (m, 1H). 3,51 - 3.40 (m. 1H). 2.39 (d, J = 1.6 Hz, 3H), 2.04 - 1.73 (m, 3H).** |
| 6 | 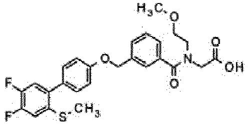 | - | **2-[[3-[[4-(4,5-difluoro-2-methylsulfanyl-phenyl)phenoxy]methyl]bezoyl]-(2-methoxyethyl)amino]acetic acid** | **MS (ESI) m/z 502 (M+H)+ 1H NMR (400 MHz, DMSO-d6) δ 7.60- 7.23 (m, 8H), 7.08 (d, J = 8.8 Hz, 2H), 5.29- 5.13 (m, 2H), 4.20 - 3.97 (m, 2H), 3.66 - 3.31 (m, 4H), 3.30 3.10 (m, 3H), 239 (s, 3H).** |
| 7 | 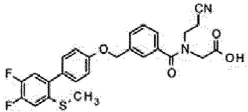 | - | **2-[2-cyanoethyl-[[4-(4.5-difluoro-2-methylsulfanyl-phenyl)phenoxy]methyl]benzoyl] amino]acetic acid** | **MS (ESI) m/z 497 (M+H)+** |
| 8 | 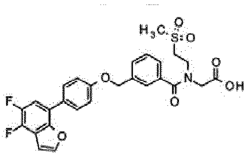 | - | **2-[[3-[[4-(4,5-difluorobenzofuran-7-yl)phenoxy]methyl]benzoyl]-(2-methylsulfonylethyl)amino]acetic acid** | **MS (ESI) m/z 544 (M+H)+ 1H NMR (400 MFtz, DMSO-d6) δ 8.19 (d, J = 2.3 Hz, 1H), 7.90 - 7.79 (m, 2H), 7,69 - 7.12 (m, 8H), 5.32 - 5.32 - 5.17 (m, 2H), 4.20 - 4.02 (m, 2H), 3.94 - 3.46 (m, 4H), 3.12 - 2.81 (m, 3H).** |

**Table 2-2**

| Example No. | moistructure Salt | | Name of Compound (IUPAC) | Analysis data |
|---|---|---|---|---|
| 9 | 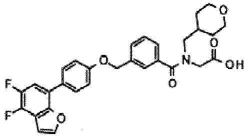 | - | 2-[[3-[[4-(4,5-difluorobenzofuran-7-yl)phenoxyl]-methyl]benzoyol]-(tetrahydropyran-4-ylmethyl)amino]acetic acid | MS (ESI) m/z 536 (M+H)+ |
| 10 | 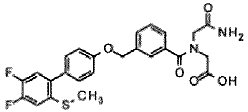 | - | 2-[(2-amino-2-oxo-ethyl)-[3-[[4-(4,5-difluoro-2-methylsufanyl-phenyl)phenoxy]methyl]benzoyl] amino]acetic acid | MS (ESI) m/z 501 (M+H)+ 1H NMR (400 MHz, DMSO-d6) δ 12.95 (bs, 1H). 7.61 - 7.46 (m, 4H), 7.40 - 7.15 (m, 6H), 7.12 - 7.05 (m, 2H), 5.18 (s, 2H), 4.12 - 3.86 (m, 4H), 2.39 (s, 3H). |
| 11 | 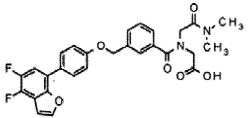 | - | 2-[[3-[[4-(4,5-difluorobenzofuran-7-yl)phenoxy]methyl]benzoyl]-[2-(dimethylamino)-2-oxoethyl]amino]acetic acid | MS (ESI) m/z 525 (M+H)+ 1H NMR (400 MHz, DMSO-d6) δ 13.18 - 12.63 (m, 1H), 8.19 (d, J = 2.3 Hz, 1H), 7.86 - 7.80 (m ,2H), 7.66 - 7.55 (m, 2H), 7.53 - 7.43 (m, 2H), 7 31 - 7.25 (m, 1H), 7.24 (d. J = 2.3 Hz, 1H), 7.21-7.15 (m, 2H), 5.26 - 5 18 (m, 2H), 4.37 - 3.91 (m, 4H), 3.04 - 2.65 (m, 6H). |
| 12 | 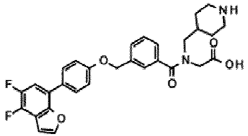 | TFA | 2-[[3-[[4-(4,5-difluorobenzofuran-7-yl)phenoxy]methyl]benzoyl]-(4-piperidylmethyl)amino]acetic acid trifluoroacetate | MS (ESI) m/z 535 (M+H)+ |
| 13 | 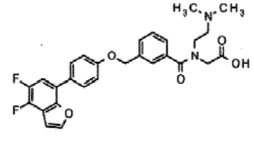 | TFA | 2-[[3-[[4-(4,5-difluorobenzofuran-7-yl)phenoxy]benzoyl]-(2-dimethylaminoethyl)amino]acetic acid trifluoroacetate | MS (ESI) m/z 509 (M+H)+ 1H NMR (400 MHz, DMSO-d6) δ13.1 (bs, 1H), 9.38 (s, 1H), 8.19 (d, J = 2.3 Hz, 1H). 7.88 - 7.79 (m, 2H). 7.68 - 7.45 (m, 4H), 7.45 - 7.29 (m, 1H). 7.25 (d, J = 2.3 Hz, 1H). 7.22 - 7.14 (m. 2H), 5.29 - 5.19 (m, 1H), 4.19 - 3.99 (m, 1H), 3.86 - 3.78 (m, 2H), 3.61 - 3.25 (m, 2H), 2.91 - 2.56 (m, 6H). |
| 14 | 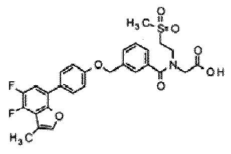 | - | 2-[[3-[[4-(4,5-difluoro-3-methyl-benzofuran-7-y)phenoxy]methyl]benzoyl]-(2-methylsulfonylethyl)amino]acetic acid | MS (ESI) m/z 558 (M+H)+ 1H NMR (400 MHz, DMSO-d6) δ 7.91 (d, J = 1.6 Hz, 1H), 7.80 (d, J = 8.8 Hz, 2H), 7.63 - 7.39 (m, 5H), 7.20 - 7 12 (m, 2H), 5.29- 5.16 (m, 2H), 4 19 - 4.02 (m, 3H), 3.88 - 3.45 (m, 4H), 3.09 - 2.81 (m, 3H), 2.34 (s, 3H) |
| 15 | 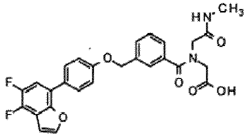 | - | 2-[[3-[[4-(4,5-difluorobenzofuran-7-yl)phenoxy]methyl]benzoyl]-[2-(methylamino)-2-oxoethyl]amino]acetic acid | MS (ESI) m/z 509 (M+H)+ 1H NMR(400 MHz, DMSO-d6) δ 12.90 (bs, 1H), 8.19 (d, J = 2.3 Hz, 1H), 8.06 - 7.96 (m. 1H), 7.87 7.79 (m, 2H), 7.66 - 7.55 (m, 2H), 7.53 - 7,45 (m. 2H), 7.37 - 7.29 (m, 1H), 7.24 (d, J = 2.3 Hz, 1H), 7.21 - 7.14 (m, 2H), 5 22 (s, 2H), 4.14 - 3.85 (m, 4H), 2.67 - 2.57 (m, 3H) |
| 16 | 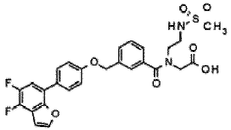 | - | 2-[[3-[[4-(4,5-difluorobenzofuran-7-yl)phenoxy]methyl]benzoyl]-[2-(methanesulfonamido)ethyl]amin o]acetic acid | MS (ESI) m/z 559 (M+H)+ 1H NMR (400 MHz, DMSO-d6) δ 12.85 (s, 1H), 8.18 (d, J = 2.2 Hz, 1H), 7.83 (d, J = 8.31 Hz, 2H), 7.66 - 7.44 (m, 4H), 7.41 - 7 27 (m, 1H), 7.23 (d, J = 2.2 Hz, 1H), 7.21 - 7.03 (m, 3H), 5 28 - 5 19 (m, 2H). 4.19 - 4.01 (m, 1H), 3.60 - 3.51 (m, 1H), 3.41 - 3.32 (m, 1H), 3.30 - 3.20 (m, 1H), 3.17-3.07 (m, 1H). 2.98 - 2.81 (m, 3H). |

**Table 2-3**

| Example No. | moistructure | Salt | Name of Compound (IUPAC) | Analysis data |
|---|---|---|---|---|
| 17 | 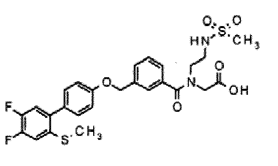 | - | 2-[[3-[[4-(4,5-difluoro-2-methylsulfanyl-phenyl)phenoxy]methyl]benzoyl]-[2-(methanesulfonamido)ethyl]amin o]acetic acid | MS (ESI) m/z 565 (M+H)+ 1H NMR (400 MHz, DMSO-d6) δ12.94 (bs, 1H), 7.61 - 7.43 (m, 3H), 7.41 - 7.25 (m, 3H), 7.21 - 7.02 (m, 3H), 5.24 - 5.15 (m, 1H), 4.18 - 3.99 (m, 1H), 3.59 - 3.50 (m, 1H), 3.51 - 3.30 (m, 1H), 3.29 - 3.19 (m, 1H), 3.16 - 3.06 (m, 1H), 2.97-2.80 (m, 3H), 2 40 (s, 3H) |
| 18 | 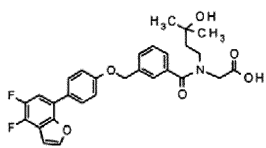 | - | 2-[[3-[[4-(4,5-diflurobenzofuran-7-yl)phenoxy]methyl]benzoyl]-(3-hydroxy-3-methyl-butyl)amino]acetic acid | MS (ESI) *m*/*z* 524 (M+H)+ |
| 19 | 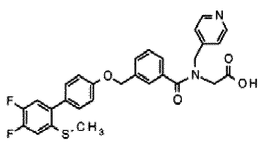 | TFA | 2-[[3-[[4-(4,5-difluoro-2-methylsulfanyl-phenyl)phenoxy]methyl]benzoyl] -(4-pyridylmethyl)amino]acetic acid trifluoroacetate | MS (ESI) m/z 535 (M+H)+ |
| 20 | 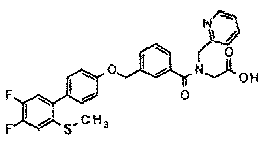 | TFA | 2-[[3-[[4-(4,5-difluoro-2-methylsulfanyl-phenyl)phenoxy]methyl]benzoyl]-(2-pyridylmethyl)amino]acetic acid trifluoroacetate | MS (ESI) m/z 535 (M+H)+ 1H NMR (400 MHz, DMSO-d6) δ 8.68 - 8.54 (m, 1H), 8.08 - 7.17 (m, 11H), 7.17 - 6.92 (m, 2H), 5.29 - 5.10 (m, 2H), 4.89 - 4.55 (m, 2H), 4.20 - 4.04 (m, 2H), 2.39 (s, 3H). |
| 21 | 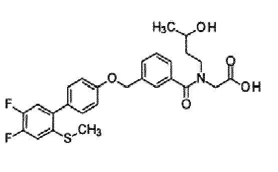 | - | 2-[[3-[[4-(4,5-difluoro-2-methylsulfanyl-phenyl)phenoxy]methyl]benzoyl]-(3-hydroxybutyl)amino]acetic acid | MS (ESI) m/z 516 (M+H)+ 1H NMR (400 MHz, DMSO-d6) δ 12.79 (bs, 1H), 7.59 - 7.23 (m, 8H), 7.12 - 7.04 (m, 2H), 5.28 - 5.13 (m, 2H), 4.17 - 3.90 (m, 2H), 3.77 - 3.12 (m, 4H), 2.39 (s, 3H), 1.79 - 1.47 (m, 2H), 1.15 - 0.88 (m, 3H). |
| 22 | 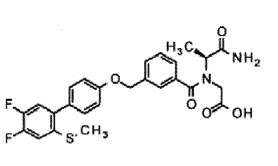 | - | 2-[[(1S)-2-amino-1-methyl-2-oxo-ethyl]-[3-[[4-(4,5-difluoro-2-methylsulfanyl-phenyl)phenoxy]methyl]benzoyl] amino]acetic acid | MS (ESI) m/z 515 (M+H)+ 1H NMR (400 MHz, DMSO-d6) δ 12.95 (bs, 1H), 7.65 - 7.44 (m, 4H), 7.42 - 7.25 (m, 6H), 7.14 - 7.05 (m, 2H), 5.24 - 5.13 (m, 2H), 4.52 - 3.83 (m, 3H), 2.39 (s, 3H), 1.48 - 1.28 (m, 3H). |
| 23 | 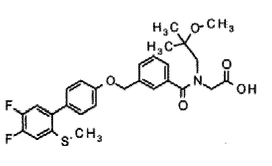 | - | 2-[[3-[[4-(4,5-difluoro-2-methylsulfanyl-phenyl)phenoxy]methyl]benzoyl]-(2-methoxy-2-methyl-propyl)amino]acetic acid | MS (ESI) m/z 530 (M+H)+ 1H NMR (400 MHz, DMSO-d6) δ 7.56 - 7.22 (m, 8H), 7.12 - 7.03 (m, 2H), 5.31 - 5.11 (m, 2H), 4 29 - 4.04 (m, 2H), 3.57 - 3.25 (m, 2H), 3.15 - 2.93 (m, 3H), 2.39 (s, 3H), 1.3 - 0.82 (m, 6H). |

**Table 2-4**

| Example No | moistructure | Salt | Name of Compound (IUPAC) | Analysis data |
|---|---|---|---|---|
| 24 | 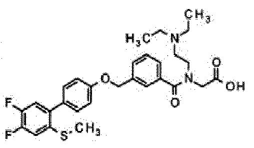 | TFA | 2-[2-diethylaminoethyl-[3-[[4-(4,5-difluoro-2-methylsulfanyl-phenyl)phenoxy]methyl]benzoyl] amino]acetic acid trifluoroacetate | MS (ESI) m/z 543 (M+H)+ 1H NMR (400 MHz, DMSO-d6) δ 9 31 (s, 1H), 7 78 7.22 (m, 8H), 7.22 - 6.99 (m, 2H), 5.32 - 5.02 (m, 2H), 4.27 - 3.96 (m, 2H), 3.89- 3.50 (m, 3H), 3.31 - 2.89 (m, 5H), 2.40 (s, 3H), 1.43 - 0.88 (m, 6H). |
| 25 | 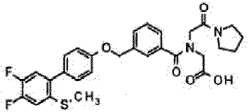 | - | 2-[[3-[[4-(4,5-difluoro-2-methylsulfanyl-phenyl)phenoxy]methyl]benzoyl]-(2-oxo-2-pyrrolidin-1-yl-ethyl)amino]acetic acid | MS (ESI) m/z 555 (M+H)+ 1H NMR (400 MHz, DMSO-d6) δ 13.16 - 12.73 (m, 1H), 7.59- 7.54 (m, 1H), 7.52 - 7.44 (m, 2H), 7.39 - 7.25 (m, 5H), 7.11 - 7.05 (m, 2H), 5.23 - 5.15 (m, 2H), 4.29 3.94 (m, 4H), 3.51 - 3.00 (m, 4H), 2.39 (s, 3H), 1.97 - 1.61 (m, 4H). |
| 26 | 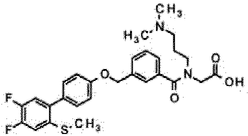 | TFA | 2-[[3-[[4-(4,5-difluoro-2-methylsulfanyl-phenyl)phenoxy]methyl]benzoyl]-[3-(dimethylamino)propyl]amino]ac etic acid trifluoroacetate | MS (ESI) m/z 529 (M+H)+ 1H NMR (400 MHz, DMSO-d6) δ 9.37 (s, 1H), 7.68 7.43 (m, 3H), 7.43 - 7.23 (m, 5H), 7.15 - 7.02 (m, 2H), 5.27- 5.09 (m, 2H), 4.19 - 3.95 (m, 2H), 3.49 3 07 (m, 5H), 2.97 - 2.62 (m, 7H), 2.40 (s, 3H), 2.03 - 1.82 (m, 2H). |
| 27 | 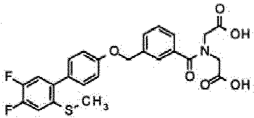 | - | 2-[carboxymethyl-[3-[[4-(4,5-difluoro-2-methylsulfanyl-phenyl)phenoxy]methyl]benzoyl] amino]acetic acid | MS (ESI) m/z 502 (M+H)+ |
| 28 | 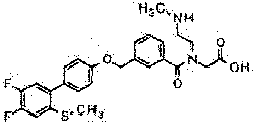 | TFA | 2-[[3-[[4-(4,5-difluoro-2-methylsulfanyl-phenyl)phenoxy]methyl]benzoyl]-[2-(methylamino)ethyl]amino]acetic acid trifluoroacetate | MS (ESI) m/z 501 (M+H)+ 1H NMR (400 MHz, DMSO-d6) δ 13.10 (s, 1H), 8.46 - 8.21 (m, 2H), 7.67 - 7.44 (m, 3H), 7.45 - 7.23 (m, 5H), 7.14-7.02 (m, 2H), 5.26-5.09 (m, 2H), 4.21 - 3.98 (m, 2H), 3.78 - 3.51 (m, 2H), 3 25 - 3 05 (m, 2H), 2.66 - 2.46 (m, 3H), 2.40 (s, 3H). |
| 29 | 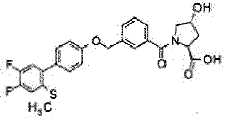 | - | (2S,4R)-1-[3-[[4-(4,5-difluoro-2-methylsulfanyl-phenyl)phenoxy]methyl]benzoyl]-4-hydroxy-pyrrolidine-2-carboxylic acid | MS (ESI) m/z 500 (M+H)+ |
| 30 | 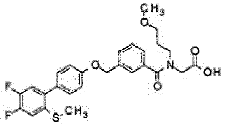 | - | 2-[[3-[[4-(4,5-difluoro-2-methylsulfanyl-phenyl)phenoxy]methyl]benzoyl]-(3-methoxypropyl)amino]acetic acid | MS (ESI) m/z 516 (M+H)+ 1H NMR (400 MHz, DMSO-d6) δ 7.60 - 7.21 (m, 8H), 7.12-7.02 (m, 2H), 5.25 - 5.11 (m, 2H), 4.15 - 3.88 (m. 2H). 3.52 - 3.00 (m, 7H), 2.39 (s, 3H), 1.88 - 1.65 (m, 2H), |
| 31 | 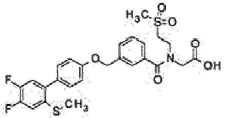 | - | 2-[[3-[[4-(4,5-difluoro-2-methylsulfanyl-phenyl)phenoxy]methyl]benzoyl]-(2-methylsulfonylethyl)amino]acetic acid | MS (ESI) m/z 550 (M+H)+ 1H NMR (400 MHz DMSO-d6) δ 7.66 - 7.20 (m, 8H), 7.14 - 7.01 (m, 2H), 5.33 - 5.06 (m, 2H), 4.19 - 4.02 (m, 2H), 3.92 - 3 43 (m, 4H), 3.16 - 2.76 (m, 3H), 2.39 (s, 3H). |

**Table 2-5**

| Example No | moistructure | Salt | Name of Compound (IUPAC) | Analysis data |
|---|---|---|---|---|
| 32 | 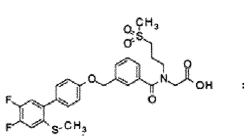 | - | 2-[[3-[[4-(4,5-difluoro-2-methylsulfanyl-phenyl)phenoxy]methyl]benzoyl]-(3-methylsulfonylpropyl)amino]aceti c acid | MS (ESI) m/z 564 (M+H)+ 1H NMR (400 MHz, DMSO-d6) δ 7.64-7.18 (m, 8H), 7.17 - 7.00 (m, 2H), 5.32 - 5.01 (m, 2H), 4.26 - 3.93 (m, 2H), 3.59 - 2.83 (m, 7H), 2.39 (s, 3H), 2.11 - 1.86 (m, 2H). |
| 33 | 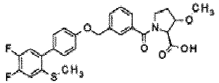 | - | (2S,3S)-1-[3-[[4-(4,5-difluoro-2-methylsulfanyl-phenyl)phenoxy]methyl]benzoyl]-3-methoxy-pyrrolidine-2-carboxylic acid | MS (ESI) m/z 514 (M+H)+ 1H NMR (400 MHz, DMSO-d6) δ 7.69 - 7.21 (m, 8H), 7.16 - 7.02 (m, 2H), 5.27 - 5.12 (m, 2H). 4 50 - 4.18 (m, 1H), 4.07 - 3.96 (m, 1H), 3.70 - 3.36 (m, 2H), 3.34 - 3.19 (m, 3H), 2.39 (s, 3H), 2.08 - 1.85 (m, 2H). |
| 34 | 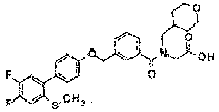 | - | 2-[[3-[[4-(4,5-difluoro-2-methylsulfanyl-phenyl)phenoxy]methyl]benzoyl]-(tetrahydropyran-4-ylmethyl)amino]acetic acid | MS (ESI) m/z 542 (M+H)+ |
| 35 | 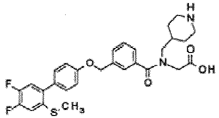 | TFA | 2-[[3-[[4-(4,5-difluoro-2-methylsulfanyl-phenyl)phenoxy]methyl]benzoyl]-(4-piperidylmethyl)amino]acetic acid trifluoroacetate | MS (ESI) m/z 541 (M+H)+ |
| 36 | 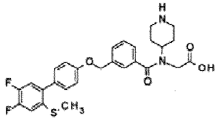 | TFA | 2-[[3-[[4-(4,5-difluoro-2-methylsulfanyl-phenyl)phenoxy]methyl]benzoyl]-(4-piperidyl)amino]acetic acid trifluoroacetate | MS (ESI) m/z 527 (M+H)+ |
| 37 | 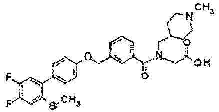 | TFA | 2-[[3-[[4-(4,-difluoro-2-methylsulfanyl-phenyl)phenoxy]methyl]benzoyl]-[(1-methyl-4-piperidyl)methyl]amino]acetic acid trifluoroacetate | MS (ESI) m/z 555 (M+H)+ |
| 38 | 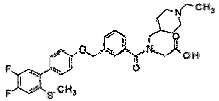 | TFA | 2-[[3-[[4-(4,5-difluoro-2-methylsulfanyl-phenyl)phenoxy]methyl]benzoyl]-[(1-ethyl-4-piperidyl)methyl]amino]acetic acid trifluoroacetate | MS (ESI) m/z 569 (M+H)+ |
| 39 | 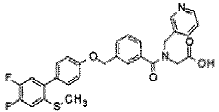 | TFA | 2-[[3-[[4-(4,5-difluoro-2-methylsulfanyl-phenyl)phenoxy]methyl]benzoyl]-(3-pyridylmethyl)amino]acetic acid trifluoroacetate | MS (ESI) m/z 535 (M+H)+ 1H NMR (400 MHz, DMSO-d6) δ 8.84 - 8.42 (m, 3H), 815 - 7.15 (m, 10H), 715 - 6.93 (m, 2H), 5.18 (s, 2H), 4.82 - 4.52 (m, 2H), 4.13 - 3.97 (m, 2H), 2.39 (s, 3H). |

**Table 2-6**

| Example No | moistructure | Salt | Name of Compound (IUPAC) | Analysis data |
|---|---|---|---|---|
| 40 | 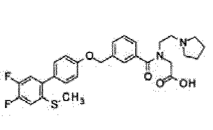 | TFA | 2-[[3-[[4-(4,5-difluoro-2-methylsulfanyl-phenyl)phenoxy]methyl]benzoyl]-(2-pyrrolidin-1-ylethyl)amino]acetic acid trifluoroacetate | MS (ESI) m/z 541 (M+H)+ 1H NMR (400 MHz, DMSO-d6) δ 13.1 (s, 1H), 9.63 (s, 1H), 7.67 - 7.23 (m, 8H), 7.14 - 7.00 (m, 2H), 5.28 - 5.11 (m. 2H), 4.24 - 3.99 (m, 2H), 3.87 - 3.00 (m, 8H), 2.40 (s, 3H), 2.12-1.69 (m, 4H), |
| 41 | 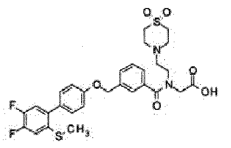 | TFA | 2-[[3-[[4-(4,5-difluoro-2-methylsulfanyl-phenyl)phenoxy]methyl]benzoyl]-[2-(1,1-dioxo-1,4-thiazinan-4-yl)ethyl]amino]acetic acid trifluoroacetate | MS (ESI) m/z 605 (M+H)+ 1H NMR (400 MHz, DMSO-d6) δ 7.69 - 7.04 (m, 10H), 5.31 - 5.11 (m, 2H) 4.21 - 4.00 (m, 2H), 3.73 - 2 69 (m, 12H), 2.40 (s, 3H) |
| 42 | 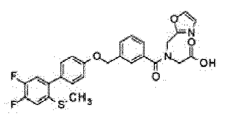 | - | 2-[[3-[[4-(4,5-difluoro-2-methylsulfanyl-phenyl)phenoxy]methyl]benzoyl]-(oxazol-2-ylmethyl)amino]acetic acid | MS (ESI) m/z 525 (M+H)+ 1H NMR (400 MHz, DMSO-d6) δ 8.10 (s, 1H), 7.64 7.01 (m, 11H). 5.26 - 5.12 (m, 2H), 4.87 - 4 56 (m, 2H), 4.18 - 4.02 (m, 2H), 2.39 (s, 3H), |
| 43 | 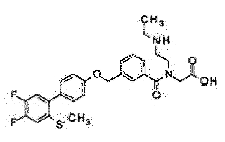 | TFA | 2-[[3-[[4-(4,5-difluoro-2-methylsulfanyl-phenyl)phenoxy]methyl]benzoyl]-[2-(ethylamino)ethyl]amino]acetic acid trifluoroacetate | MS (ESI) m/z 515 (M+H)+ 1H NMR (400 MHz, DMSO-d6) δ 13.31 - 12.80 (m, 1H), 8.58-8.12 (m, 2H), 7.80 - 6.91 (m, 10H), 5.35 - 5.02 (m, 2H), 4.24 - 3 97 (m, 2H), 3.80 - 3.52 (m, 2H), 3.27 - 2.81 (m. 4H), 2.40 (s, 3H), 1 32 - 0 97 (m, 3H), |
| 44 | 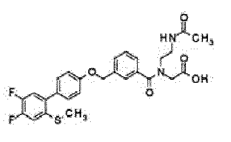 | | 2-[2-acetamidoethyl-[3-[[4-(4,5-difluoro-2-methylsulfanyl-phenyl)phenoxy]methyl]benzoyl] amino]acetic acid | MS (ESI) m/z 529 (M+H)+ 1H NMR (400 MHz, DMSO-d6) δ 8.00 - 7.80 (m, 1H), 7.59 - 7.22 (m, 8H), 7.12 - 7 03 (m, 2H), 5.30 - 5.08 (m, 2H), 4.24-3.84 (m, 2H), 3.58 - 3 39 (m, 2H), 3.33 - 3.12 (m, 2H), 2 39 (s, 3H), 1.90 - 1.84 (m, 3H) |
| 45 | 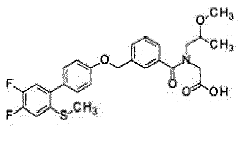 | - | 2-[[3-[[4-(4,5-difluoro-2-methylsulfanyl-phenyl)phenoxy]methyl]benzoyl]-(2-methoxypropyl)amino]acetic acid | MS (ESI) m/z 516 (M+H)+ 1H NMR (400 MHz, DMSO-d6) δ 7.63- 7.19 (m, 8H), 7.08 (d, J = 8.6 Hz, 2H), 5.30 - 5.08 (m, 2H), 4.36 - 3.89 (m, 3H). 3.40 - 3.11 (m 5H), 2.40 (s, 3H), 119-0.79 (m, 3H). |
| 46 | 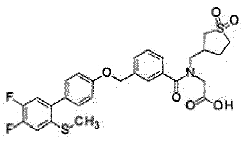 | - | 2-[[3-[[4-(4,5-difluoro-2-methylsulfanyl-phenyl)phenoxy]methyl]benzoyl]-[(1,1-dioxothiolan-3-yl)methyl]amino]acetic acid | MS (ESI) m/z 576 (M+H)+ 1H NMR (400 MHz, DMSO-d6) δ 13.35 - 12.55 (m, 1H), 7.67-7.17 (m, 8H), 7.17 - 7.02 (m, 2H), 5.31 - 5.09 (m, 2H). 4.23 - 1.41 (m. 11H). |
| 47 | 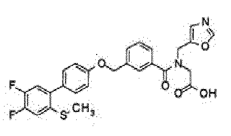 | - | 2-[[3-[[4-(4,5-difluro-2-methylsulfanyl-phenyl)phenoxy]methyl]benzoyl]-(oxazol-5-ylmethyl)amino]acetic acid | MS (ESI) m/z 525 (M+H)+ 1H NMR (400 MHz, DMSO-d6) δ 11.46 - 17.05 (m, 1H), 8.35 (s, 1H), 7.66 - 7.12 (m, 9H), 7.12 - 7.04 (m, 2H), 5.33 - 5.09 (m, 2H), 4.86 - 4.46 (m, 2H), 3.98 - 3 71 (m, 1H), 2.39 (s, 3H), |

**Table 2-7**

| Example No | moistructure | Salt | Name of Compound(IUPAC) | Analysis data |
|---|---|---|---|---|
| 48 | 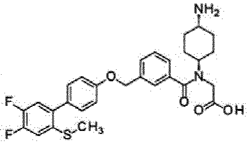 | TFA | 2-[(4-ammocyclohexyl)-[3-[[4-(4,5-difluoro-2-methylsulfanyl-phenyl)phenoxy]methyl]benzoyl] anino]acetic acid trifluoroacetate | MS (ESI) m/z 541 (M+H)+ |
| 49 | 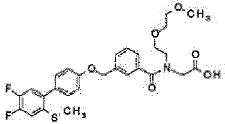 | - | 2-[[3-[[4-(4,5-difluoro-2-methylsulfanyl-phenyl)phenoxy]methyl]benzoyl]. [2-(2-methoxyethoxy)ethyl]amino]aceti c acid | MS (ESI) m/z 546 (M+H)+ |
| 50 | 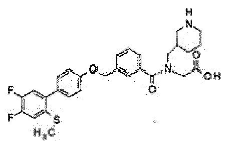 | TFA | 2-[[3-[[4-(4,5-difluoro-2-methylsulfanyl-phenyl)phenoxy]methyl]benzoyl]-(3-piperidylmethyl)amino]aceitic acid trifluoroacetate | MS (ESI) m/z 541 (M+H)+ |
| 51 | 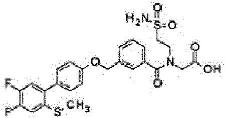 | - | 2-[[3-[[4-(4,5-difluoro-2-methylsulfanyl-phenyl)phenoxy]methyl]benzoyl]-(2-sufamoylethyl)amino]acetic acid | MS (ESI) m/z 551 (M+H)+ 1H NMR (400 MHz, DMSO-d6) δ 7.74 - 7.19 (m, 8H), 7.14 - 6.80 (m. 4H), 5.30 - 5.06 (m, 2H), 4.23 - 3.97 (m, 2H), 3.88 - 3.61 (m, 4H), 2.40 (s, 3H). |
| 52 | 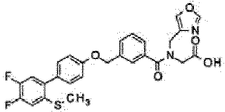 | - | 2-[[3-[[4-(4,5-difluoro-2-methylsulfanyl-phenyl)phenoxy]methyl]benzoyl]-(oxazol-4-ylmethyl)amino]acetic acid | MS (ESI) m/z 525 (MtH)+ 1H NMR (400 MHz, DMSO-d6) δ 8.45 - 8.35 (m, 1H), 8.12(s, 1H), 7.71 - 7.23 (m, 9H), 7.08 (d, J = 8.8 Hz, 2H). 5.30 - 5.09 (m. 2H), 4.65 - 4 31 (m. 2H), 4.07 - 3.94 (m, 2H), 2.40 (s, 3H). |
| 53 | 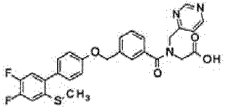 | - | 2-[[3[[4-(4,5-difluoro-2-methylsulfanyl-phenyl)phenoxy]methyl]benzoyl]-(pyrirridin-4-ylmethyl)amino]acetic acid | MS (ESI) m/z 536 (M+H)+ 1H NMR (400 MHz, DMSO-d6) δ 9.14 (d, J = 3.7, 1.4 Hz, 1H), 8.76 (t, J = 5.7 Hz, 1H), 7.66 - 7.17 (m. 9H). 7.09 (d. J = 8.7 Hz, 1H), 6.99 (d, J = 8.7 Hz, 1H), 5.26 - 5.07 (m, 2H), 4.80 - 4.57 (m, 2H), 4.20 - 4.09 (m, 2H), 2.39 (s, 3H). |
| 54 | 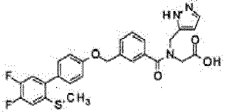 | - | 2-[[3-[[4-(4,5-difluoro-2-methylsulfanyl pnenyl)phenoxy]methyl]benzoyl]-(1H-pyrazol-5-ylmethyl)amino]acetic acid | MS (ESI) m/z 524 (M+H)+ 1H NNR (400 MHz, DMSO-d6) δ 7.75 - 7 16 (m, 10H), 7.07 (d, J = 8.6, 1.7 Hz, 2H), 6.30 - 6.16 (m, 1H), 5.25 - 5.13 (m, 2H). 4.70 - 4.38 (m. 2H), 4.07 - 3.82 (m, 2H). 2.39 (s, 3H). |
| 55 | 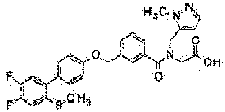 | - | 2-[[3-[[4-(4,5-difluoro-2-methylsulfanyl-phenyl)phenoxy]methyl]benzoyl]-[(2-methylpyrazol-3-yl)methyl]amino]acetic acid | MS (ESI) m/z 538 (M+H)+ 1H NMR (400 MHz, DMSO-d6) δ 7.75- 6.97 (m, 11H), 6.36 - 6.27 (m, 1H), 5.18 (s, 2H), 4.85 - 4.49 (m, 2H), 4.12 - 3.76 (m, 2H), 3.51 (s, 3H), 2.39 (s, 3H). |

**Table 2-8**

| Example No | moistructure | Salt | Name of Compound (IUPAC) | Analysis data |
|---|---|---|---|---|
| 56 | 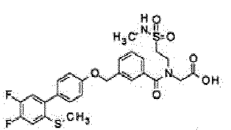 | - | 2-[[3-[[4-(4,5-difluoro-2-methylsulfanyl-phenyl)phenoxy]methyl]benzoyl]-[2-(methylsulfanoyl)ethyl]amino]ac etic acid | MS (ESI) m/z 565 (M+H)+ 1H NMR (400 MHz, DMSO-d6) δ 7.74-6.80 (m, 11H), 5.30 - 5.01 (m, 2H), 4.24 - 3.95 (m, 2H), 3.83 - 3.28 (m, 4H), 2.70 - 2.57 (m, 2H), 2.44 - 2.33 (m. 4H). |
| 57 | 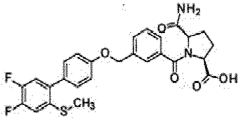 | - | (2S,55) or (2S, 5R)-5-carbamoyl-1-[3-[[4-(4,5-difluoro-2-methylsulfanyl-phenyl)phenoxy]methyl]benzoyl] pyrrolidine-2-carboxylic acid | MS (ESI) m/z 527 (M+H)+ 1H NMR (400 MHz, DMSO-d6) δ 8.11 - 7.24 (m, 10H), 7.09 (d. J = 8.1 Hz, 2H), 5.17 (s, 2H), 4 63 - 4.14 (m, 2H), 2.39 (s, 3H), 2.38 - 2.20 (m, 2H), 2.06 - 1.76 (m, 2H), |
| 58 | 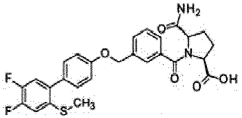 | - | (2S,5S) or (2S, 5R)-5-carbamoyl-1-[3-[[4-(4,5-difluoro-2-methylsulfanyl-phenyl)phenoxy]methyl]benzoyl] pyrrolidine-2-carboxylic acid | MS (ESI) m/z 527 (M+H)+ |
| 59 | 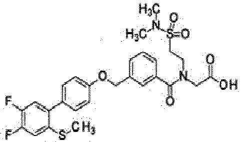 | - | 2-[[3-[[4-(4,5-difluoro-2-methylsulfanyl-phenyl)phenoxy]methyl]benzoyl]-[2-(dimethylsulfamoyl)ethyl]amino]a cetic acid | MS (ESI) m/z 579 (M+H)+ 1H NMR (400 MHz, DMSO-d6) δ 7.65 - 7.20 (m, 8H), 7.14 - 7.01 (m, 2H), 5.32 - 5.11 (m, 2H), 4.25 - 4.02 (m, 2H), 3.83 - 3.38 (m, 4H), 2.82 (s, 3H), 2.61 (s, 3H), 2 39 (s, 3H) |
| 60 | 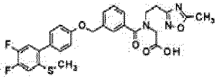 | - | 2[[3-[[4-(4,5-difluoro-2-methylsulfanyl-phenyl)phenoxy]methyl]benzoyl]-[2-(5-methyl-1,2,4-oxadiazol-3-yl)ethyl]amino]acetic acid | MS (ESI) m/z 554 (M+H)+ 1H NMR (400 MHz, DMSO-d6) δ 13.1 - 12.6 (m, 1H), 7.58 - 7.52 (m, 1H), 7.52- 7.44 (m, 1H), 7,44 - 7.40 (m, 1H), 7.40 - 7.22 (m, 5H), 7.12 - 7.04 (m, 2H), 5.25 5.12 (m, 2H), 4 22 - 3.95 (m, 2H), 3.82 - 3.58 (m, 2H), 3.10 - 2.91 (m, 2H), 2.60 - 2.45 (m, 3H), 2.39 (s, 3H). |
| 61 | 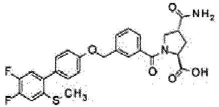 | - | (2S,4R)-4-carbamoyl-1-[3-[[4-(4,5-difluoro-2-methylsulfanyl-phenyl)phenoxy]methyl]benzoyl] pyrrolidine-2-carboxylic acid | MS (ESI) m/z 527 (M+H)+ 1H NMR (400 MHz, DMSO-d6) δ 7.80 - 7.17 (m, 9H), 7.17- 6.89 (m, 3H), 5.30 - 5.10 (m, 2H), 4.58 - 4.35 (m, 1H). 3.84 - 3.64 (m, 1H), 3.19 - 2.87 (m, 2H), 2.39 (s, 3H), 2.21 1.88 (m, 2H). |
| 62 | 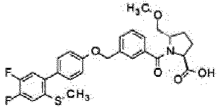 | - | (2S,5S)-1-[3-[[4-(4,5-difluoro-2-methylsulfanyl-phenyl)phenoxy]methyl]benzoyl]-5-(methoxymethyl)pyrTQIldine-2-carboxylic acid | MS (ESI) m/z 528 (M+H)+ 1H NMR (400 MHz, DMSO-d6) δ 7.61- 7.23 (m, 8H), 7.11 - 7.04 (m. 2H), 5.30 - 5.12 (m. 2H), 4.54 - 4.04 (m, 2H), 3.57 - 3.36 (m, 1H), 3.34 - 2.83 (m, 3H), 3.00 - 2.86 (m, 1H), 2.46 - 2.35 (m, 3H), 2 34 - 1.99 (m, 1H), 1.98 - 1 75 (m, 3H) |
| 63 | 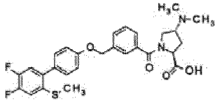 | TFA | (2S,4R)-1-[3-[[4-(4,5-difluoro-2-methylsulfanyl-phenyl)phenoxy]methyl]benzoyl]-4-(dimethylamino)pyrrolidine-2-carboxylic acid trifluoroacetate | MS (ESI) m/z 627 (M+H)+ 1H NMR (400 MHz, DMSO-d6) δ 10.27 - 9.81 (m, 1H), 7.83 - 6.86 (m, 10H), 5.36 5.00 (m, 2H), 4.76 - 4.47 (m, 1H), 4.08 - 3.64 (m, 3H), 2.96 - 2.63 (m, 6H), 2.63 - 2.36 (m, 5H). |

**Table 2-9**

| Example No | moistructure | Salt | Name of Compound (IUPAC) | Analysis data |
|---|---|---|---|---|
| 64 | 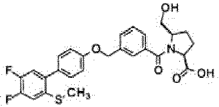 | - | (2S,5R)-1-[3-[[4-(4,5-difluoro-2-methylsulfanyl-phenyl)phenoxy]methyl]benzoyl]-5-(hydroxymethyl)pyrrolidine-2-carboxylic acid | MS (ESI) m/z 514 (M+H)+ |
| 65 | 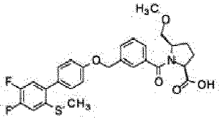 | - | (2S,5R)-1-[3-[[4-(4,5-difluoro-2-methylsulfanyl-phenyl)phenoxy]methyl]benzoyl]-5-(methoxymethyl)pyrrolidine-2-carboxylic acid | MS (ESI) m/z 528 (M+H)+ 1H NMR (400 MHz, DMSO-d6) δ 7 80 - 6.93 (m, 10H), 5.33 - 5.04 (m, 2H), 4.54 - 4.21 (m, 2H), 3.39 - 2.89 (m, 5H), 2 39 (s, 3H), 2.36 - 1.69 (m, 4H). |
| 66 | 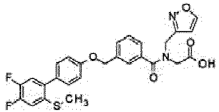 | - | 2-[[3-[[4-(4,5-difluoro-2-methylsulfanyl-phenyl)phenoxy]methyl]benzoyl]-(isoxazol-3-ylmethyl)amino]acetic acid | MS (ESI) m/z 525 (M+H)+ 1H NMR (400 MHz, DMSO-d6) δ 8.91 (d, J = 7.7 Hz, 1H), 7.72 - 6.87 (m, 10H), 6.71 - 6.50 (m, 1H), 5.19 (s, 2H), 4.81 4.53 (m, 2H), 4.12 - 3.97 (m, 2H), 2.39 (s, 3H) |
| 67 | 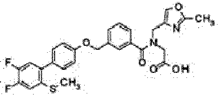 | - | 2-[[3-[[4-(4,5-difluoro-2-methylsulfanyl-phenyl)phenoy]methyl]benzoyl]-[(2-methyloxazol-4-yl)methyl]amino]acetic acid | MS (ESI) m/z 539 (M+H)+ 1H NMR (400 MHz, DMSOd6) δ 12.8 (brs, 1H), 7.98 - 7.91 (m, 1H), 7.71 - 7.42 (m, 4H), 7.40 - 7.23 (m, 4H), 7.12 - 7.03 (m, 2H), 5.29 - 5.12 (m, 2H), 4.55- 4.24 (m, 2H), 4.09- 3.91 (m, 2H), 2.42 - 2.37 (m, 6H). |
| 68 | 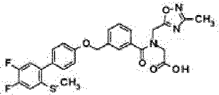 | - | 2-[[3-[[4-(4,5-difluoro-2-methylsulfanyl-phenyl)phenoxy]methyl]benzoyl]-[(3-methyl-1,2,4-oxadiazol-5-y))methyl]amino]acetic acid | MS (ESI) m/z 540 (M+H)+ 1H NMR (400 MHz, DMSO-d6) δ 13.1 (brs, 1H), 7.64 - 7.56 (m, 1H), 7.55 - 7.46 (m, 2H), 7.41 - 7.25 (m, 5H), 7.13 - 7.02 (m, 2H), 5.19 (s, 2H), 4.97 - 4.74 (m, 2H), 4.24 - 4.13 (m, 2H), 2.39 (s, 3H), 2.37 - 2.32 (m, 3H). |
| 69 | 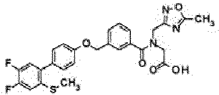 | - | 2-[[3-[[4-(4,5-difluoro-2-methylsulfanyl-pheny)phenozy]methyl]benzoyl]-[(5-methyl-1,2,4-oxadiazol-3-yl)methyl]amino]acetic acid | MS (ESI) m/z 540 (M+H)+ 1H NMR (400 MHz, DMSO-d6) δ 13.3 - 12.7 (m, 1H), 7.62 - 7.42 (m, 3H), 7.40 - 7 24 (m, 5H), 7.11 - 7.03 (m, 2H), 5.24 - 5.18 (m, 2H), 4.84 - 4.51 (m, 2H), 4.19 - 4.00 (m, 2H), 2.62 - 2 57 (m, 3H), 2.39 (s, 3H). |
| 70 | 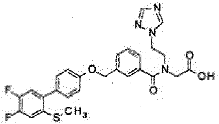 | - | 2-[[3-[[4-(4,5-difluoro-2-methylsulfanyl-phenyl)phenoxy]methyl]benzoyl]-[2-(1,2,4triazol-1-yl)ethyl]amino]acetic acid | MS (ESI) m/z 539 (M+H)+ 1H NMR (400 MHz, DMSO-d6) δ 8.54 (d, J = 59.2 Hz, 1H), 7.96 (d, J = 39.5 Hz, 1H), 7.58 - 6.92 (m, 10H). S.16 (s, 2H), 4.54 - 4.27 (m, 2H). 4.17 - 3.60 (m, 4H), 2.39 (s, 3H). |
| 71 | 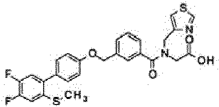 | - | 2-[[3-[[4-(4,5-difluoro-2-methylsulfanyl-phenyl)phenoxy]methyl]benzoyl-(thiazol-4-ylmethyl)amino]acetic acid | MS (ESI) m/z 541 (M+H)+ 1H NMR (400 MHz, DMSO-d6) δ 9.18 - 9.07 (m, 1H), 7.71 - 7.20 (m, 9H). 7.14 - 7.00 (m, 2H), 5 28 5.12 (m, 2H), 4.86 - 4.55 (m, 2H), 4.11 - 3.97 (m, 2H), 2.39 (s, 3H). |

**Table 2-10**

| Example No. | moistructure | Salt | Name of Compound (IUPAC) | Analysis data |
|---|---|---|---|---|
| 72 | 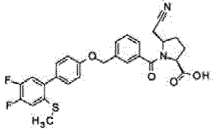 | - | (2S)-5-(cyanomethyl)-1-[3-[[4-(4,5-difluoro-2-methylsulfanyl-phenyl)phenoxy]methyl]benzol] pyrrolidine-2-carboxylic acid | MS (ESI) m/z 523 (M+H)+ |
| 73 | 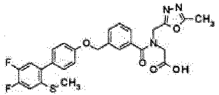 | - | 2-[[3-[[4-(4,5-difluoro-2-methylsulfanyl-phenyl)phenoxy]methyl]benzoyl]-[(5-methyl-1,3,4-oadiazol-2-yl)methyl]amino]acetic acid | MS (ESI) m/z 540 (M+H)+ 1H NMR (400 MHz, DMSO-d6) δ 7.64 - 7.24 (m, 8H), 7.12 - 7,05 (m, 2H), 5.26 - 5.15 (m, 2H). 4.97 - 4.64 (m, 2H), 4.20 - 4.05 (m, 2H), 2.53 - 2 44 (m, 3H), 2.42 - 2.37 (m, 3H). |
| 74 | 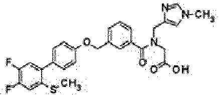 | - | 2-[[3-[[4-(4,5-difluoro-2-methylsulfanyl-phenyl)phenoxy]methyl]benzoyl]-[(1-methylimidazol-4-yl)methyl]amino]acetic acid | MS (ESI) m/z 538 (M+H)+ 1H NMR (400 MHz, DMSO-d6) δ 9.26 - 9.62 (m, 1H), 7.77 - 7.30 (m, 9H), 7.14 (d, J = 7.9 Hz, 2H), 5.30- 5.20 (m, 2H), 4.80 - 4.53 (m, 2H), 4.21 - 4.02 (m, 2H), 3.96 - 3.80 (m, 3H), 2.46 (s, 3H). |
| 75 | 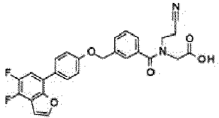 | - | 2-[2-cyanoethyl-[3-[[4-(4,5 4,5-difluorobenzofuran-7-yl)phenoxy]methyl]benzoyl] amino]acetic acid | MS (ESI) *m*/*z* 491 (M+H)+ |
| 76 | 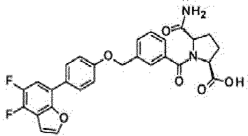 | - | (2S,55) or (2S, 5R)-5-carbamoyl-1-[3-[[4-(4,5-difluorobenzofuran-7-yl)phenoxy]methyl]benzoyl] pyrrolidine-2-carboxylic acid | MS (ESI) m/z 521 (M+H)+ 1H NMR (400 MHz, DMSO-d6) δ 13.5 (s, 1H), 8.19 (d, J = 2.2 Hz, 1H). 8.11 - 7.87 (m, 1H), 7.83 (d, J = 8.4 Hz, 2H). 7.78 - 7.67 (m, 1H), 7.67 - 7.54 (m, 3H), 7.54 - 7 44 (m, 1H), 7.42 - 7.36 (m, 1H), 7.24 (d, J = 2.2 Hz, 1H), 7.19 (d, J = 8.4 Hz, 2H), 5.21 (s, 2H), 4.62 - 4.20 (m, 2H), 2.45 - 2.19 (m, 2H), 2.05 - 1.79 (m, 2H), |
| 77 | 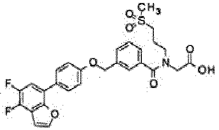 | - | 2-[[3-[[4-(4,5-difluorobenzofuran-7-yl)phenoxy]methyl]benzoyl]-(3-methylsulfonylpropyl)amino]acet c acid | MS (ESI) m/z 558 (M+H)+ 1H NMR (400 MHz, DMSO-d6) δ 8.19 (d, J = 2.3 Hz, 1H), 7.83 (d, J = 8.8 Hz, 2H), 7.70 - 7.3B (m. 4H), 7.40 - 7.07 (m, 4H), 5.35 - 6.12 (m, 2H), 4.16 - 3.94 (m, 2H), 3.62 - 2.79 (m, 7H), 2.11 - 1.81 (m 2H), |
| 78 | 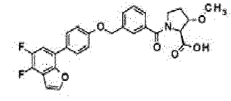 | - | (2S,3S)-1-[3-[[4-(4,5-difluorobenzofuran-7-yl)phenoxy]methyl]benzoyl]-3-methoxy-pyrrolidine-2-carboxylic acid | MS (ESI) m/z 508 (M+H)+ 1H NMR (400 MHz, DMSO-d6) δ 8.19 (d, J = 2.2 Hz, 1H), 7.82 (d, J = 8.8 Hz, 2H), 7.65 - 7.43 (m, 5H), 7.32 - 7.14 (m, 3H), 5.32 - 5.20 (m, 2H), 4.48 - 4.21 (m, 1H), 4.04 - 3.98 (m, 1H), 3.69 - 3.46 (m 2H), 3.38- 3.20 (m, 3H), 2.09 - 1.80 (m. 2H). |
| 79 | 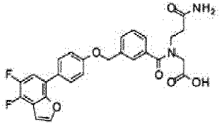 | - | 2-[(3-amino-3-oxo-propyl)-[3-[[4-(4,5-difluorobenzofuran-7-y))phenoxy]methyl]benzoyl] amino]acetic acid | MS (ESI) m/z 509 (M+H)+ 1H NMR (400 MHz, DMSO-d6) δ 12.7 (s, 1H), 8.19 (d, J = 2.3 Hz, 1H), 7.87 - 7.78 (m, 2H), 7.67 - 7.12 (m, 9H), 6.93 - 6.81 (m, 1H), 5.31 - 5.16 (m, 2H), 4 19 - 3.93 (m, 2H), 3.64 - 3.40 (m, 2H), 2.49 - 233 (m, 2H) |

**Table 2-11**

| Example No | molstructure | Salt | Name of Compound (IUPAC) | Analysis data |
|---|---|---|---|---|
| 80 | 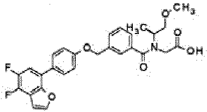 | - | 2-[[3-[[4-(4,5-difluorobenzofuran-7-yl)phenoxy]methy]benzoyl]-[(1S)-2-methoxy-1-methylethyl]amino]acetic acid | MS (ESI) m/z 510 (M+H)+ |
| 81 | 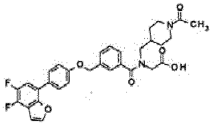 | - | 2-[(1-acetyl+piperidyl)methyl-[3-[[4-(4,5-difluorobenzofuran-7-yl)phenoxy]methyl]benzoyl] amino]acetic acid | MS (ESI) m/z 577 (M+H)+ |
| 82 | 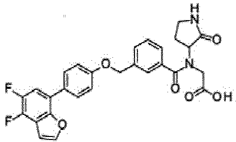 | - | 2-[[3-[[4-(4,5-difluorobenzofuran-7-yl)phenoxy]methyl]benzoyl]-(2-oxopyrrolidin-3-yl)amino]acetic acid | MS (ESI) m/z 521 (M+H)+ 1H NMR (400 MHz, DMSO-d6) δ 8 19 (d, J = 2.3 Hz, 1H), 8.04 - 7.86 (m, 1H), 7.86 - 7.79 (m, 2H), 7.67 - 7.38 (m, 5H), 7.23 (d, J = 2 3 Hz, 1H), 7.21 - 7.15 (m, 2H), 5.32 - 5.21 (m, 2H), 4.45 - 4,33 (m, 1H), 4.30 - 4.18 (m 1H), 4.06 - 3.86 (m, 1H), 3.18 - 2.94 (m, 2H), 2.33 - 2.08 (m, 2H) |

### Test Example 1

### Measurement of Glycogen Synthase Activity

A human GYS1 expression plasmid (pCDNA3. 1 (+) -hGYS1) was constructed by the following method. By using a human skeletal muscle cDNA of Human MTC Panel I (Takara Bio Inc., 636742) as a template, a human GYS1 gene was amplified by a PCR method using cloning primers (Forward Primer: ATGCCTTTAAACCGCAC, Reverse Primer: TTAGTTACGCTCCTCGC). By using the amplified human GYS1 sequence as a template, restriction enzyme sequences were added by a PCR method using sub-cloning primers (Forward Primer: CCCTCGAGACCATGCCTTTAAACCGCACTT, and Reverse Primer: GGTCTAGATTAGTTACGCTCCTCGCCCAG), and then the human GYS1 gene was introduced between Xho I and Xba I sites of pCDNA3.1(+) (Invitrogen, V790-20).

Glycogen synthase was prepared by the following method. Human kidney-derived HEK293T cells were seeded in a dish (Thermo Fisher Scientific K.K., 168381) by using a 10% FBS-containing DMEM (Nacalai Tesque, Inc., 0845874) medium, and cultured overnight. Then, by using Lipofectamine LTX (Invitrogen, 15338-100), the cells were transfected with the human GYS1 expression vector according to the attached manual. After culturing under conditions of 37°C and 5% CO₂ for 2 days, the resultant was lysed in a lysis buffer (50 mM Tris-HCl (pH 8.0), 10 mM EDTA, 2 mM EGTA, 100 mM NaF, 1 mM PMSF, 1 mM DTT, and 1x Complete (Roche, 1873580)), homogenized, and then centrifuged at 16000×g at 4°C for 15 minutes. The precipitated fraction re-dissolved by adding the lysis buffer was used as a glycogen synthase for evaluation.

The glycogen synthase activity was measured by the following method. To a polystyrene 96-well plate, solutions each containing 30 mM glycylglycine (pH 7.3), 40 mM KCl, 20 mM MgCl₂, 9.2% DMSO containing one of the test compounds at one of various concentrations, and 10 mM Glucose-6-phosphate (Sigma-Aldrich, G7879) were added at 12 µL/well.

Next, a substrate solution containing 30 mM glycylglycine (pH 7.3), 4.3 mg/mL glycogen (Sigma-Aldrich, G8876), 21.6 mM UDP-glucose (Sigma-Aldrich, U4625), 21.6 mM phosphoenolpyruvic acid (Sigma-Aldrich, P0564), and 4.05 mM NADH (Sigma-Aldrich, N8129) was added at 18 µL/well.

Moreover, an enzyme solution containing 50 mM Tris-HCl (pH 8.0), 27 mM DTT (NACALAI TESQUE, INC., 14128-04), 0.2 mg/mL bovine serum albumin, 0.17 mg/mL glycogen synthase, and 1.5 µL pyruvate kinase/lactate dehydrogenase solution (Sigma-Aldrich, P0294) was added at 18 µL/well to prepare reaction solutions. After the reaction solutions were incubated (at 37°C for 25 minutes for Examples 1 to 61 and 67 to 82 or at 37°C for 20 minutes for Examples 62 to 66), the absorbance at 340 nm was measured by using Benchmark Plus (Bio-Rad Laboratories, Inc.).

The activity of each test compound was calculated by the following method. The absorbance at 340 nm of the reaction solution containing the compound and DMSO was subtracted from the absorbance at 340 nm of a reaction solution not containing the compound but containing only DMSO to calculate the change (ΔA340) in absorbance. The relative activity (%) of the test compound at each of the various concentrations was calculated, where the ΔA340 of a reaction solution containing the compound of Example 1 of WO/2011/058154 at a final concentration of 10 µM was taken as 100%. EC₅₀ representing the concentration of the compound at which an increase in the relative activity by 50% was caused was calculated using XLfit (idbs). Tables 3-1, 3-2, and 3-3 show the results.

**Table 3-1**

| Example No. | EC50 (µM) |
|---|---|
| 1 | 0.013 |
| 2 | 0.0043 |
| 3 | 0.016 |
| 4 | <0.003 |
| 5 | <0.01 |
| 6 | 0.017 |
| 7 | 0.011 |
| 8 | 0.015 |
| 9 | 0.016 |
| 10 | 0.0072 |
| 11 | 0.010 |
| 12 | 0.016 |
| 13 | 0.015 |
| 14 | 0.015 |
| 15 | 0.013 |
| 16 | 0.016 |
| 17 | 0.010 |
| 18 | 0.0073 |
| 19 | 0.0034 |
| 20 | 0.0065 |

**Table 3-2**

| Example No. | EC60 (µM) |
|---|---|
| 21 | 0.015 |
| 22 | 0.013 |
| 23 | 0.017 |
| 24 | 0.011 |
| 25 | 0.015 |
| 26 | 0.014 |
| 27 | 0.010 |
| 28 | 0.010 |
| 29 | 0.019 |
| 30 | 0.015 |
| 31 | 0.018 |
| 32 | 0.020 |
| 33 | 0.021 |
| 34 | 0.024 |
| 35 | 0.019 |
| 36 | 0.021 |
| 37 | 0.023 |
| 38 | 0.019 |
| 39 | 0.021 |
| 40 | 0.024 |
| 41 | 0.021 |
| 42 | 0.019 |
| 43 | 0.019 |
| 44 | 0.025 |
| 45 | 0.0077 |
| 46 | 0.025 |
| 47 | 0.018 |
| 48 | 0.013 |
| 49 | 0.0095 |
| 50 | 0.0063 |
| 51 | 0.0052 |
| 52 | 0.0044 |
| 53 | 0.0079 |
| 54 | 0.015 |
| 55 | 0.022 |
| 56 | <0.003 |
| 57 | 0.010 |

**Table 3-3**

| Example No. | EC50 (µM) |
|---|---|
| 58 | 0.0073 |
| 59 | 0.016 |
| 60 | 0.011 |
| 61 | 0.019 |
| 62 | 0.017 |
| 63 | 0.017 |
| 64 | 0.022 |
| 65 | 0.018 |
| 66 | 0.015 |
| 67 | 0.013 |
| 68 | <0.003 |
| 69 | 0.020 |
| 70 | 0.021 |
| 71 | 0.017 |
| 72 | 0.011 |
| 73 | 0.015 |
| 74 | 0.008 |
| 75 | 0.022 |
| 76 | 0.022 |
| 77 | 0.020 |
| 78 | 0.020 |
| 79 | 0.022 |
| 80 | 0.024 |
| 81 | 0.020 |
| 82 | 0.019 |
| Compound of Example 1 of WO/2011/058154 | 0.051 ~ 0.17 |

### Test Example 2

### Measurement of PPAR-α Activity

The PPAR-α activity was measured according to the published article (THE JOURNAL OF BIOLOGICAL CHEMISTRY Vol. 270, No .22,: 12953-12956, 1995).

Plasmids used for the PPAR-α activity measurement were constructed as follows. A luciferase expression plasmid (UASx5-TK-Luc) used was obtained by introducing a sequence in which five yeast GAL4-binding sequences are linked in tandem into a portion upstream of a thymidine kinase promoter of pTAL-Luc (Takara Bio Inc., 6252-1). A PPAR-α receptor expression plasmid (hGR-GAL4-hPPARα) used was obtained by introducing a human GR N-terminal region (1-76aa), a yeast GAL4 DNA-binding region (1-147aa), and a PPARα ligand-binding region (167-468aa) between Not I and Sal I sites of pExchange-1 Core Vector (Invitrogen, 211176).

A reporter assay was conducted by the following method using African green monkey kidney-derived CV-1 cells. The CV-1 cells were seeded in a 96-well plate (Thermo Fisher Scientific K.K., 4938) at 2×10⁴ cells/well by using a 10% FBS-containing DMEM (NACALAI TESQUE, INC., 0845874) medium. After culturing under conditions of 37°C and 5% CO₂ for 2 hours, the cells were transfected with the plasmids. The transfection was conducted by using Lipofectamine LTX (Invitrogen, 15338-100) according to the attached manual. The plasmid solution was prepared by adding a mixture solution of the luciferase expression plasmid and the PPAR-α receptor expression plasmid to OPTI-MEM I (Invitrogen, 11058-021). After the transfection, the test compound was added, and the cells were cultured at 37°C in the presence of 5% CO₂ for 18 to 20 hours. After the culturing, the luciferase activity was measured with Luminescensor JNR (ATTO) by using Bright-Glo (Promega, E2620).

The fold induction of PPAR-α by each test compound was calculated by the following method. A relative fold induction (%) of PPAR-α by the test compound was defined as 100 (A/B), where A represents the maximum value of the PPAR-α activities of the test compound at 3 µM, 10 µM, 30 µM, and 100 µM, and B represents the PPAR-α activity of the compound of WO/2011/058154 at 100 µM. Tables 4-1, 4-2, and 4-3 show the results.

**Table 4-1**

| Example No. | Relative fold induction (%) of PPAR α |
|---|---|
| 1 | 12 |
| 2 | 5.0 |
| 3 | 11 |
| 4 | 10 |
| 5 | 10 |
| 6 | 86 |
| 7 | 16 |
| 8 | 11 |
| 9 | 24 |
| 10 | 6.7 |
| 11 | 7.4 |
| 12 | 4.7 |
| 13 | 5.3 |
| 14 | 7.5 |
| 15 | 12 |
| 16 | 12 |
| 17 | 13 |
| 18 | 23 |
| 19 | 21 |

**Table 4-2**

| Example No. | Relative fold induction (%) of PPAR α |
|---|---|
| 20 | 13 |
| 21 | 15 |
| 22 | 14 |
| 23 | 22 |
| 24 | 13 |
| 25 | 13 |
| 26 | 11 |
| 27 | 10 |
| 28 | 10 |
| 29 | 22 |
| 30 | 83 |
| 31 | 8.4 |
| 32 | 7.1 |
| 33 | 13 |
| 34 | 7.3 |
| 35 | 8.5 |
| 36 | 8.0 |
| 37 | 10 |
| 38 | 8.9 |
| 39 | 22 |
| 40 | 9.4 |
| 41 | 12 |
| 42 | 13 |
| 43 | 9.0 |
| 44 | 25 |
| 45 | 25 |
| 46 | 4.6 |
| 47 | 7.3 |
| 48 | 4.0 |
| 49 | 29 |
| 50 | 4.8 |
| 51 | 5.8 |
| 52 | 8.1 |
| 53 | 8.2 |
| 54 | 8.7 |
| 55 | 11 |
| 56 | 8.0 |

**Table 4-3**

| Example No. | Relative fold induction (%) of PPAR α |
|---|---|
| 57 | 6.7 |
| 58 | 7.8 |
| 59 | 10 |
| 60 | 14 |
| 61 | 17 |
| 62 | 14 |
| 63 | 13 |
| 64 | 20 |
| 65 | 7.0 |
| 66 | 22 |
| 67 | 19 |
| 68 | 35 |
| 69 | 23 |
| 70 | 14 |
| 71 | 20 |
| 72 | 12 |
| 73 | 16 |
| 74 | 16 |
| 75 | 38 |
| 76 | 42 |
| 77 | 40 |
| 78 | 65 |
| 79 | 7.1 |
| 80 | 91 |
| 81 | 6.4 |
| 82 | 11 |
| Compound of Example 1 of WO/2011/058154 | 100 |

### Test Example 3

### Evaluation of Glycogen Accumulation in L6

The glycogen accumulation activity was evaluated in skeletal muscle cells according to the published article (ANALYTICAL BIOCHEMISTRY, Vol. 261,: 159-163, 1998) by the following method. Rat skeletal muscle-derived L6 myoblasts (ATCC) were seeded in a 96-well collagen-coated plate (IWAKI, 4860-010) by using a growth medium (10% FBS-containing α-MEM medium (NACALAI TESQUE, INC., 21444-05)) under a condition of 4×10⁴ cells/100 µL/well. After culturing under conditions of 37°C and 5% CO₂ overnight, the medium was replaced with a differentiation medium (2% FBS-containing α-MEM medium), followed by culturing for 3 days to differentiate the myoblasts into myotube cells. On the day of evaluation, the medium was replaced with a glucose-deprived medium (0.1% BSA-containing DMEM medium (Gibco, 11966)). After culturing for 4 hours, the medium was replaced with assay media (0.1% BSA-containing DMEM medium (NACALAI TESQUE, INC., 08456-65)) containing 0.6% DMSO and one of the test compounds at a concentration which was twice the final evaluation concentration at 15 µL/well. Further, an assay medium containing D-[U-C14] glucose (PerkinElmer Inc., NEC042V) at 1.9 µL/well was added at 15 µL/well to adjust the final volume to 30 µL/well. Then, the cells were incubated under conditions of 37°C and 5% CO₂ for 3 hours. After the incubation, each medium was removed by suction with an aspirator, and the wells were rinsed with PBS at 200 µL/well twice. Then, 1 N NaOH was added at 50 µL/well, and the cells were lysed by incubation at 60°C for 10 minutes. After the temperature of the lysed cells was returned to room temperature, each whole cell lysate and 10 mg/mg glycogen (Sigma-Aldrich, G8876) at 5 µL/well were added to a Multiscreen HTS (Millipore, MSFCN6B) to which 100% ethanol was added in advance at 100 µL/well, followed by incubation at 4°C for 20 minutes. After the incubation, aspiration was conducted by using Multiscreen HTS Vacuum Manifold (Millipore), followed by rinse with 66% ethanol at 200 µL/well twice. After ethanol remaining on the filter was completely removed by drying, MicroScint40 (PerkinElmer Inc.) was added at 50 µL/well, the amount of [14C] glycogen was measured with TopCountNXT (PerkinElmer Inc.). The activity of each test compound was calculated by the following method. Specifically, the relative activity (%) was calculated as 100 × (B-A) / (C-A), where A represents a value (cpm) measured for a well not containing the compound but containing only DMSO, B represents a value measured for a well containing the compound and DMSO, and C represents a value measured for a well containing the compound of Example 1 of WO/2011/058154 at a final concentration of 30 µM. EC₅₀ representing the concentration of the compound at which an increase in activity by 50% was caused was calculated using XLfit (idbs).

**Table 5-1**

| Example No. | EC50 (µM) |
|---|---|
| 6 | 0.57 |
| 20 | 0.71 |
| 28 | <0.3 |
| 35 | 0.63 |
| 40 | 5.4 |
| 42 | 0.88 |
| 62 | <0.3 |
| 65 | 1.8 |
| Compound of Example 1 of WO/2011/058154 | 14~16 |

## Claims

1. A compound represented by the following general formula (I) or a pharmaceutically acceptable salt thereof: 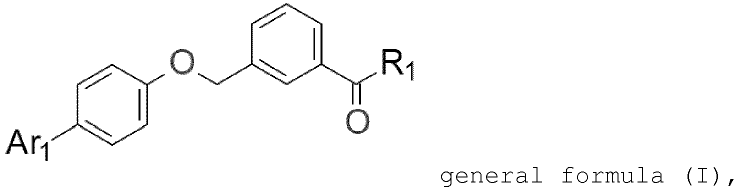 wherein Ar₁ represents any one of the following rings (II) and (III) : 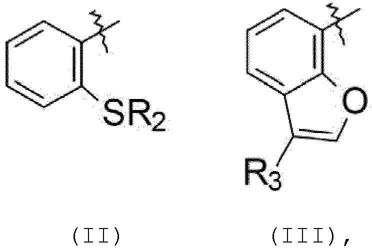 wherein each of the rings may have one or more substituents, and the substituents are selected from the group consisting of acetamido groups, aminocarbonyl groups, halogen atoms, alkyl groups, hydroxyalkyl groups, alkoxyalkyl groups, cyano groups, cyanoalkyl groups, amino groups, aminoalkyl groups, monoalkylaminoalkyl groups, dialkylaminoalkyl groups, alkoxy groups, and halogenoalkoxy groups;
R₂ represents an alkyl group, and R₃ represents a hydrogen atom or an alkyl group, and
R₁ represents any one of the following substituents (IV) and (V): 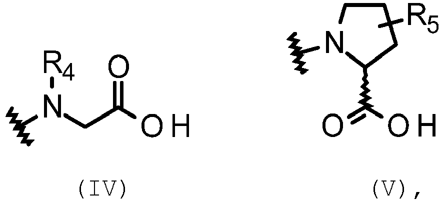
wherein R₄ represents a substituent selected from cyanoalkyl groups, aminocarbonylalkyl groups, dialkylaminocarbonylalkyl groups, monoalkylaminocarbonylalkyl groups, alkylsulfonylalkyl groups, aminoalkyl groups, aminosulfonylalkyl groups, monoalkylaminosulfonylalkyl groups, dialkylaminosulfonylalkyl groups, monoalkylaminoalkyl groups, dialkylaminoalkyl groups, aminocycloalkyl groups, monoalkylaminocycloalkyl groups, dialkylaminocycloalkyl groups, alkoxyalkyl groups, monoalkoxyaminoalkyl groups, alkoxyalkyleneoxyalkyl groups, alkylcarbonylaminoalkyl groups, alkylsulfonylaminoalkyl groups, hydroxyalkyl groups, carboxyalkyl groups, and groups represented by -L₁-B, where L₁ represents a bond or an alkylene group, and B represents an optionally substituted 5-membered or 6-membered heterocyclic group having at least one heteroatom selected from nitrogen atoms, oxygen atoms, and sulfur atoms, and
R₅ represents -L₂-R₆, where L₂ represents a bond or an alkylene group, and R₆ represents a hydroxy group, an alkoxy group, an amide group, an amino group, a monoalkylamino group, a dialkylamino group, a cyano group, an aminocarbonyl group, a monoalkylaminocarbonyl group, or a dialkylaminocarbonyl group.

2. The compound according to claim 1 or a pharmaceutically acceptable salt thereof, wherein B in -L₁-B of R₄ in the general formula (I) is any one of the following groups: 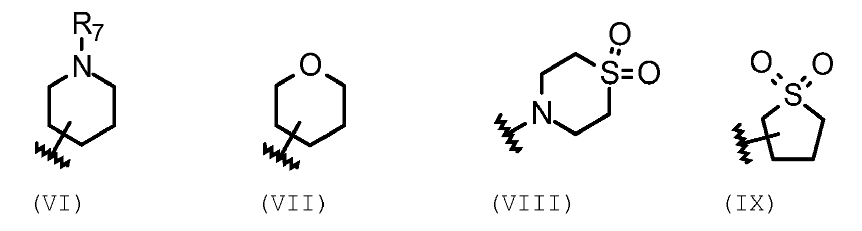 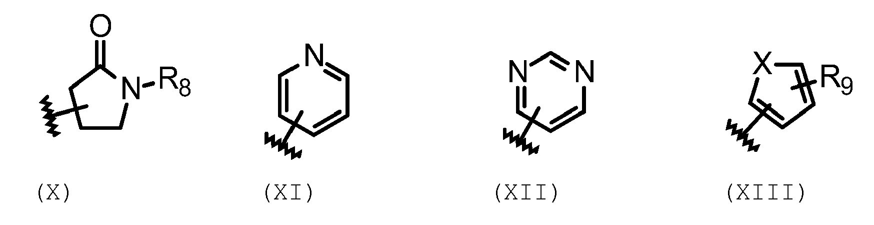 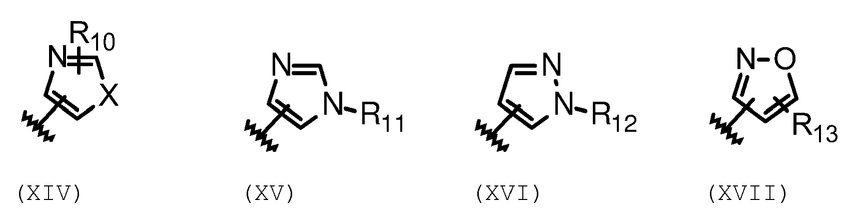 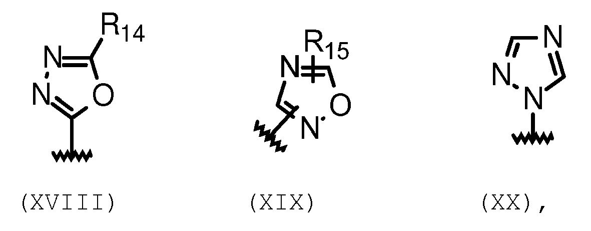 wherein R₇ represents a hydrogen atom, an alkyl group, or an alkylcarbonyl group, R₈ to R₁₅ each represent a hydrogen atom or an alkyl group, and X represents an oxygen atom or a sulfur atom.

3. The compound according to claim 1 or a pharmaceutically acceptable salt thereof, wherein R₁ represents any one of the following substituents (IV), (V-I), and (V-II): 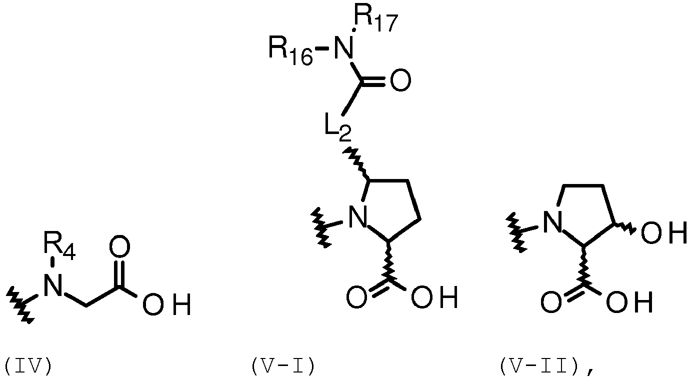 wherein R₄ represents a substituent selected from cyanoalkyl groups, aminocarbonylalkyl groups, dialkylaminocarbonylalkyl groups, monoalkylaminocarbonylalkyl groups, alkylsulfonylalkyl groups, aminoalkyl groups, monoalkylaminoalkyl groups, dialkylaminoalkyl groups, alkoxyalkyl groups, alkylsulfonylaminoalkyl groups, hydroxyalkyl groups, carboxyalkyl groups, and the following (VI-I), (VII-I), and (XI-I) : 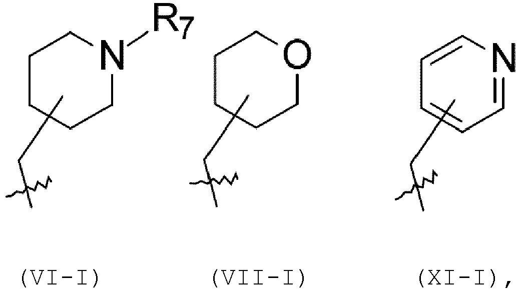
wherein R₇ represents a hydrogen atom or an alkyl group,
R₁₆ and R₁₇, which may be the same or different, each represent a hydrogen atom or an alkyl group, or R₁₆ and R₁₇ taken together may form a ring, and
L₂ represents a bond or an alkylene group.

4. The compound according to any one of claims 1 to 3 or a pharmaceutically acceptable salt thereof, wherein Ar₁ in the general formula (I) has 2 or 3 substituents which are halogen atoms.

5. The compound according to any one of claims 1 to 4 or a pharmaceutically acceptable salt thereof, wherein in the general formula (I), R₂ in the ring (II) represents a methyl group, or R₃ in the ring (III) represents a hydrogen atom or a methyl group.

6. The compound according to any one of claims 3 to 5 or a pharmaceutically acceptable salt thereof, wherein L₂ in the formula (V-I) in the general formula (I) represents a bond or an alkylene group having 1 to 5 carbon atoms.

7. The compound according to any one of claims 3 to 6 or a pharmaceutically acceptable salt thereof, wherein in the general formula (I), each of R₁₆ and R₁₇ in the substituent (V-I) represents a hydrogen atom or a methyl group, and R₇ in the substituent (VI-I) represents a hydrogen atom or a methyl group.

8. A pharmaceutical composition comprising the compound according to any one of claims 1 to 7 or a pharmaceutically acceptable salt thereof.

9. A pharmaceutical composition for treating diabetes mellitus, comprising the compound according to any one of claims 1 to 7 or a pharmaceutically acceptable salt thereof.

10. A glycogen synthase activator comprising the compound according to any one of claims 1 to 7 or a pharmaceutically acceptable salt thereof.
